(19) Europäisches Patentamt
European Patent Office
Office européen des brevets

(11) Veröffentlichungsnummer: **0 022 245**
**A2**

(12) # EUROPÄISCHE PATENTANMELDUNG

(21) Anmeldenummer: 80103772.2

(22) Anmeldetag: 02.07.80

(51) Int. Cl.³: C 07 D 501/20
A 61 K 31/545, C 07 D 285/08

(30) Priorität: 05.07.79 CH 6294/79
20.02.80 CH 1365/80

(43) Veröffentlichungstag der Anmeldung:
14.01.81 Patentblatt 81/2

(84) Benannte Vertragsstaaten:
AT BE CH DE FR GB IT LI LU NL SE

(71) Anmelder: CIBA-GEIGY AG
Patentabteilung Postfach
CH-4002 Basel(CH)

(72) Erfinder: Csendes, Ivan, Dr.
Finkenstrasse 9
CH-4104 Oberwil(CH)

(72) Erfinder: Müller, Beat, Dr.
Vogesenstrasse 31
CH-4106 Therwil(CH)

(72) Erfinder: Scartazzini, Riccardo, Dr.
Conrad Ferdinand Meyer-Strasse 38
CH-4059 Basel(CH)

(74) Vertreter: Zumstein sen., Fritz, Dr. et al,
Bräuhausstrasse 4
D-8000 München 2(DE)

(54) **Amino-thiadiazolyl-Verbindungen, Verfahren zu ihrer Herstellung, sie enthaltende pharmazeutische Präparate; Ausgangsverbindungen und Verfahren zu ihrer Herstellung.**

(57) 7β-Aminothiadiazolylacetamido-3-cephem-4-carbonsaureverbindungen der Formel

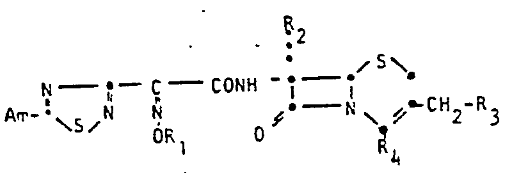

(1).

worin Am eine gegebenenfalls geschützte Aminogruppe darstellt, $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxv bedeutet, $R_3$ für Wasserstoff, Acyloxy oder Heterocyclylthio steht, und $R_4$ einegegebenenfalls geschützte Carboxylgruppe darstellt, und Salze von solchen Verbindungen, die eine salzbildende Gruppierung aufweisen, werden nach verschiedenen an sich bekannten Verfahren hergestellt. Die Verbindungen haben ausgezeichnete antibiotische Eigenschaften. In Form pharmazeutischer Präparate zur parenteralen Verabreichung können sie zur Behandlung von bakteriellen Infektionen verwendet werden

Ferner werden 1,2,4-Thiadiazol Ausgangsverbindungen sowie Verfahren zu ihrer Herstellung beschrieben.



Croydon Printing Company Ltd.

0022245



CIBA-GEIGY AG
Basel (Schweiz)

BEZEICHNUNG GEÄNDERT
siehe Titelseite

Amino-thiadiazolylverbindungen, Verfahren zu ihrer Herstellung, pharmazeutische Präparate enthaltend solche Verbindungen und Verwendung von letzteren

Die Erfindung betrifft neue 7ß-Aminothiadiazolylacetamido-3-cephem-4-carbonsäureverbindungen, Verfahren zu ihrer Herstellung, pharmazeutische Präparate, die solche Verbindungen enthalten und deren Verwendung als Antibiotika.

In der DT-OS 2.745.246 sind gewisse 7ß-Thiadiazolylacetamido-3-cephem-4-carbonsäure-verbindungen mit antibiotischer Wirkung beschrieben worden. Gegenüber diesen Verbindungen stellen die Verbindungen der vorliegenden Erfindung insofern eine Verbesserung dar, als sie sich überraschenderweise durch eine ganz besonders hervorragende antibiotische Wirksamkeit auszeichnen. Diese hervorragende Wirksamkeit ist insbesondere deshalb überraschend, weil die Verbindungen der vorliegenden Erfindung nicht unter die bevorzugten Ausführungsformen der DT-OS 2.745.246 fallen.

Die Erfindung betrifft insbesondere 7ß-Aminothiadiazolylacetamido-3-cephem-4-carbonsäureverbindungen der Formel

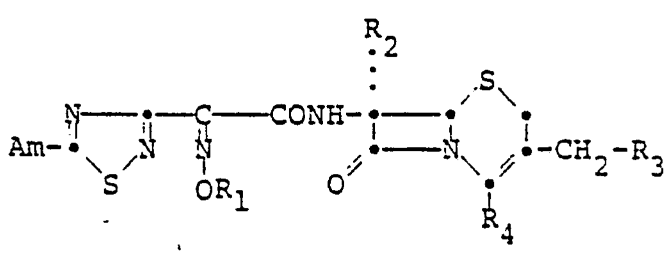

(I),

BAD ORIGINAL

worin Am eine gegebenenfalls geschützte Aminogruppe darstellt, $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, Acyloxy oder Heterocyclylthio steht, und $R_4$ eine gegebenenfalls geschützte Carboxylgruppe darstellt, und Salze von solchen Verbindungen, die eine salzbildende Gruppierung aufweisen, Verfahren zur Herstellung dieser Verbindungen, solche Verbindungen enthaltende pharmazeutische Mittel und deren Verwendung.

In der vorliegenden Beschreibung der Erfindung bedeutet der im Zusammenhang mit Definitionen von Gruppen und Verbindungen verwendete Ausdruck "nieder", z.B. in Gruppen wie Niederalkyl, Niederalkylen, Niederalkoxy, Niederalkanoyl oder Niederalkylamino, oder in Verbindungen wie niederer Alkohol und dergleichen, dass die entsprechenden Gruppen bzw. Verbindungen, sofern nicht ausdrücklich anders definiert, bis zu 6, bevorzugt bis zu 4 C-Atome enthalten.

Eine gegebenenfalls geschützte Aminogruppe Am ist eine primäre, sekundäre oder tertiäre Aminogruppe, wie Amino, Niederalkylamino oder Diniederalkylamino, wobei die primären oder sekundären Aminogruppen wie weiter unten angegeben geschützt sein können. In einer Niederalkyl- oder Diniederal-

BAD ORIGINAL

kylaminogruppe enthält Niederalkyl 1-4 C-Atome und ist beispielsweise Methyl, Aethyl, Propyl oder Butyl. Am ist bevorzugt gegebenenfalls geschütztes Amino oder Methylamino.

Eine Niederalkylgruppe $R_1$ enthält vorzugsweise 1-4 C-Atome und ist beispielsweise Aethyl, Propyl, Butyl oder insbesondere Methyl.

Eine Cycloalkylgruppe $R_1$ enthält vorzugsweise 3-8, in erster Linie 3-6 Ringglieder, und ist z.B. Cyclobutyl, Cyclopentyl oder Cyclohexyl, und insbesondere Cyclopropyl.

Substituenten von substituiertem Niederalkyl oder Cycloalkyl $R_1$ sind u.a. gegebenenfalls veräthertes Hydroxy, z.B. Niederalkoxy, primäres, sekundäres oder tertiäres Amino Am, z.B. Amino oder Diniederalkylamino, gegebenenfalls funktionell abgewandeltes, inkl. verestertes, amidiertes oder geschütztes Carboxyl oder Sulfo, sowie gegebenenfalls durch Niederalkyl N-substituiertes Ureidocarbonyl. Bevorzugt sind die substituierten Niederalkyl- und Cycloalkylgruppen durch eine Carboxyl- oder Sulfogruppe substituiert, wobei diese bevorzugt am Kohlenstoffatom, das mit dem Sauerstoffatom der Oxyiminogruppe verbunden ist, stehen. Solche substituierte Niederalkyl- und Cycloalkylgruppen $R_1$ sind beispielsweise 2-Aminoäthyl, 2-Dimethylaminoäthyl, Carboxymethyl, 1- oder 2-Carboxyäthyl, 1-, 2- oder 3-Carboxyprop-1-yl, 1-, 2- oder 3-Carboxyprop-2-yl, 1-Carboxybut-1-yl, 1-Carboxycycloprop-1-yl und 1-Carboxycyclobut-1-yl, sowie entsprechende durch Sulfo substituierte Niederalkyl- und Cycloalkylgruppen.

Die Carboxy- und Sulfogruppen im Rest $R_1$ können

BAD ORIGINAL

beispielsweise durch Niederalkyl, wie Methyl oder Aethyl, oder eine der physiologisch abspaltbaren Gruppen, z.B. Pivaloÿloxymethyl, verestert oder durch ein Amin, wie $NH_3$ oder ein primäres oder sekundäres Amin, wie ein Mono- oder Diniederalkylamin, wie Methyl- oder Aethylamin, oder Dimethyl- oder Diäthylamin amidiert sein, oder wie etwa für $R_4$ angegeben, geschützt sein. In einer Ureidocarbonylgruppe im Rest $R_1$ können die beiden Stickstoffatome unabhängig voneinander durch Niederalkyl, wie Methyl oder Aethyl, substituiert sein. Solche Gruppen sind beispielsweise Ureidocarbonyl oder 1,3-Dimethylureidocarbonyl.

Gegebenenfalls N-substituiertes Carbamoyl als Rest $R_1$ ist eine Gruppe -C(=O)-NHR, worin R Wasserstoff, Niederalkyl, z.B. Methyl, Aethyl oder 1- oder 2-Propyl, gegebenenfalls geschütztes Carboxyniederalkyl, z.B. Carboxymethyl, 1- oder 2-Carboxyäthyl oder 1-, 2- oder 3-Carboxypropyl, worin Carboxy durch eine der üblichen Carboxylschutzgruppen geschützt, beispielsweise durch Niederalkyl, z.B. Methyl, Aethyl, n- oder iso-Propyl, oder n- oder tert.Butyl verestert sein kann, gegebenenfalls geschütztes Sulfoniederalkyl, z.B. Sulfomethyl, 1- oder 2-Sulfoäthyl oder 1-, 2- oder 3-Sulfopropyl, worin Sulfo durch eine der üblichen Sulfoschutzgruppen geschützt, beispielsweise durch Niederalkyl, z.B. Methyl oder Aethyl, verestert sein kann, gegebenenfalls geschütztes Hydroxyniederalkyl, z.B. Hydroxymethyl, 2-Hydroxyäthyl oder 2- oder 3-Hydroxypropyl, worin Hydroxy durch eine der üblichen Hydroxyschutzgruppen geschützt ist, z.B. acyliert, wie acetyliert, sein kann, gegebenenfalls geschütztes Aminoniederalkyl, z.B. 2-Aminoäthyl, 2- oder 3-Aminopropyl oder 2-, 3- oder 4-Aminobutyl, worin Amino durch eine der üblichen Aminoschutzgruppen geschützt, z.B. acyliert, wie acetyliert, sein kann, Arylniederalkyl, beispielsweise Phenylniederalkyl, z.B. Benzyl oder 1- oder 2-Phenyläthyl, Halogenniederalkyl, beispielsweise Fluor-, Chlor- oder Brom-nieder-

BAD ORIGINAL

alkyl, z.B. 2-Chloräthyl, 3-Chlorpropyl- oder 4-Chlorbutyl, oder worin R Aryl, z.B. Phenyl oder durch Niederalkyl, z.B. Methyl, Niederalkoxy, z.B. Methoxy, Halogen, z.B. Chlor oder Nitro, ein- bis dreifach substituiertes Phenyl, bedeutet.

Eine Niederalkoxygruppe $R_2$ enthält bevorzugt 1-4 C-Atome und ist beispielsweise Aethoxy, Propoxy, Butoxy, oder insbesondere Methoxy.

In einer Acyloxygruppe $R_3$ ist Acyl beispielsweise der Acylrest einer gegebenenfalls substituierten niederaliphatischen Carbonsäure, einer Niederalkoxycarbonsäure, einer Niederalkansulfonsäure, einer aromatischen Sulfonsäure, einer aromatischen Carbonsäure, einer Durch Aryl-substituierten Niederalkancarbonsäure oder einer gegebenenfalls N-substituierten Carbaminsäure.

Solche Acyloxygruppen $R_3$ sind beispielsweise Niederalkanoyloxy, insbesondere Acetyloxy, ferner Formyloxy, Propionyloxy, Butyryloxy, Isobutyryloxy, Valeryloxy, Isovaleryloxy, Hexanoyloxy, Heptanoyloxy, Pivaloyloxy, Acetoacetoxy, Oxalyloxy, Succinyloxy und dergleichen, Niederalkoxycarbonyloxy, wie Methoxycarbonyloxy, Aethoxycarbonyloxy, Propoxycarbonyloxy, 1-Cyclopropyläthoxycarbonyloxy, Isopropoxycarbonyloxy, Butoxycarbonyloxy, tert.-Butoxycarbonyloxy, Pentyloxycarbonyloxy, tert.-Pentyloxycarbonyloxy, Hexyloxycarbonyloxy und dergleichen, Niederalkansulfonyloxy, wie Mesyloxy, Aethansulfonyloxy, Propansulfonyloxy, Isopropansulfonyloxy, Butansulfonyloxy und dergleichen, Arensulfonyloxy, wie Benzolsulfonyloxy, Tosyloxy und dergleichen, Aroyl, wie Benzoyl, Toluoyl, Napthoyl, Phthaloyl, Indancarbonyl und dergleichen, Arylniederalkanoyl, wie Phenylacetyl und dergleichen, wobei der oben angegebene Acylrest einen oder zwei geeignete Substituen-

BAD ORIGINAL

ten, wie Halogen, z.B. Chlor, Brom, Jod oder Fluor, Hydroxy, Cyano, Nitro, niederes Alkoxy, z.B. Methoxy, Aethoxy, Propoxy, Isopropoxy und dergleichen, niederes Alkyl, z.B. Methyl, Aethyl, Propyl, Isopropyl, Butyl und dergleichen, niederes Alkenyl, z.B. Vinyl, Allyl und dergleichen oder z.B. Phenyl, Tolyl und dergleichen aufweisen kann. Geeignete Beispiele für das Acyloxy mit einem oder mehreren dieser Substituenten sind vorzugsweise Halogenniederalkanoyloxy, z.B. Chloracetyloxy, Dichloracetyloxy, Trichloracetyloxy oder Trifluoracetyloxy.

In dem Acylrest einer gegebenenfalls N-substituierten Carbaminsäure sind N-Substituenten gegebenenfalls durch Halogen, z.B. Chlor, oder durch Niederalkanoyl, z.B. Acetyl oder Propionyl, substituierte Niederalkylreste, z.B. Methyl, Aethyl, 2-Chloräthyl, oder 2-Acetoxyäthyl. Solche Acyloxygruppen $R_3$ sind z.B. Carbamoyloxy, N-Methylcarbamoyloxy, N-Aethylcarbamoyloxy, N-(2-Chloräthyl)-carbamoyloxy oder N-(2-Acetoxyäthyl)-carbamoyloxy.

In einer Heterocyclylthiogruppe $R_3$ ist Heterocyclyl insbesondere ein gegebenenfalls substituierter, über ein Ringkohlenstoffatom an die Thiogruppe gebundener, heterocyclischer Rest mit 1 bis 4 Ringstickstoffatomen und gegebenenfalls einem weiteren Ringheteroatom der Gruppe Sauerstoff und Schwefel.

Solche Heterocyclylreste sind in erster Linie gegebenenfalls substituierte, z.B. die unten genannten Substituenten enthaltende, monocyclische, fünf- oder sechsgliedrige diaza-, triaza-, tetraza-, thiaza-, thiadiaza-, thiatriaza-, oxaza- oder oxadiazacyclische Reste aromatischen oder partiell gesättigten Charakters.

BAD ORIGINAL

Substituenten solcher Heterocyclylreste sind u.a. Niederalkyl, insbesondere Methyl, sowie Aethyl, n-Propyl, Isopropyl oder geradkettiges oder verzweigtes Butyl, oder durch Hydroxy, verestertes Hydroxy, wie Niederalkanoyloxy, Halogen, wie Chlor, Carboxy, verestertes Carboxy, wie Niederalkoxycarbonyl, Sulfo, amidiertes Sulfo, Amino, Mono- oder Diniederalkylamino, Acylamino, wie Niederalkanoylamino, oder durch substituiertes, wie durch Carboxy oder Halogen substituiertes Niederalkanoylamino substituiertes Niederalkyl, beispielsweise 2-Hydroxyäthyl, 2-Acetoxyäthyl, 2-Chloräthyl, Carboxymethyl, 2-Carboxyäthyl, Aethoxycarbonylmethyl, 2-Aethoxycarbonyläthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder Heterocyclyl, wie Furyl, z.B. 2-Furyl, Thienyl, z.B. 2-Thienyl, oder Oxazolyl, z.B. 2- oder 5-Oxazolyl, oder funktionelle Gruppen, wie Halogen, z.B. Fluor, Chlor oder Brom, gegebenenfalls substituiertes Amino, wie gegebenenfalls durch Niederalkyl mono- oder di-substituiertes Amino, z.B. Amino, Methylamino oder Dimethylamino, Acylamino, wie Niederalkanoylamino oder durch Halogen oder Carboxy substituiertes Niederalkanoylamino, wie Acetylamino, 3-Chlorpropionylamino oder 3-Carboxypropionylamino, Nitro, Hydroxy, Niederalkoxy, z.B. Methoxy, Aethoxy, n-Butyloxy oder 2-Aethylhexyloxy, oder gegebenenfalls funktionell abgewandeltes Carboxy, wie Carboxy, verestertes Carboxy, wie Niederalkoxycarbonyl, z.B. Methoxycarbonyl oder Aethoxycarbonyl, gegebenenfalls substituiertes, wie N-mono- oder N,N-diniederalkyliertes Carbamoyl, z.B. N-Methylcarbamoyl oder N,N-Dimethylcarbamoyl, oder Cyan,

BAD ORIGINAL

sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte Heterocyclylthiogruppen $R_3$, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio, z.B. 1H-1,2,3-Triazol-4-ylthio, 1-Methyl-1H-1,2,3-triazol-4-ylthio, 1H-1,2,4-Triazol-3-ylthio, 5-Methyl-1H-1,2,4-triazol-3-ylthio, 3-Methyl-1-phenyl-1H-1,2,4-triazol-5-ylthio, 4,5-Dimethyl-4H-1,2,4-triazol-3-ylthio oder 4-Carboxymethyl- oder 4-Phenyl-4H-1,2,4-triazol-3-ylthio, insbesondere gegebenenfalls wie angegeben substituiertes Tetrazolylthio, z.B. 1H-Tetrazol-5-ylthio, 1-Methyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Phenyl-1H-tetrazol-5-ylthio oder 1-(4-Chlorphenyl)-1H-tetrazol-5-ylthio, gegebenenfalls durch Niederalkyl oder Thienyl substituiertes Thiazolylthio oder Isothiazolylthio, z.B. 2-Thiazolylthio, 4-(2-Thienyl)-2-thiazolylthio, 4,5-Dimethyl-2-thiazolylthio, 3-Isothiazolylthio, 4-Isothiazolylthio oder 5-Isothiazolylthio, insbesondere auch gegebenenfalls wie angegeben substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 2-(3-Carboxypropionylamino)-1,3,4-thiadiazol-5-ylthio, 1,2,4-Thiadiazol-5-ylthio, 3-Methyl-1,2,4-thiadiazol-5-ylthio oder 1,2,5-Thiadiazol-3-ylthio, Thiatriazolylthio, z.B. 1,2,3,4-Thiatriazolyl-5-ylthio, gegebenenfalls wie angegeben substituiertes Oxazolylthio

BAD ORIGINAL

oder Isoxazolylthio, z.B. 5-Oxazolylthio, 4-Methyl-5-oxazolylthio, 2-Oxazolylthio, 4,5-Diphenyl-2-oxazolylthio oder 3-Methyl-5-isoxazolylthio, oder gegebenenfalls wie angegeben substituiertes Oxadiazolylthio, z.B. 1,2,4-Oxadiazol-5-ylthio, 2-Methyl-1,3,4-oxadiazol-5-ylthio, 2-Phenyl-1,3,4-oxadiazol-5-ylthio, 5-(4-Nitrophenyl)-1,3,4-oxadiazol-2-ylthio oder 2-(2-Thienyl)-1,3,4,-oxadiazol-5-ylthio.

Bevorzugte Heterocyclylthiogruppen $R_3$, worin der heterocyclische Rest einen entsprechenden monocyclischen, sechliedrigen Rest oder einen entsprechenden partiell gesättigten Rest darstellt, sind u.a. gegebenenfalls durch Halogen substituiertes 1-Oxidopyridylthio, z.B. 1-Oxido-2-pyridylthio oder 4-Chlor-1-oxido-2-pyridylthio, gegebenenfalls durch Hydroxy substituiertes Pyridazinylthio, z.B. 3-Hydroxy-6-pyridazinylthio, gegebenenfalls durch Niederalkyl, Niederalkoxy oder Halogen substituiertes N-Oxido-pyridazinylthio, z.B. 2-Oxido-6-pyridazinylthio, 3-Chlor-1-oxido-6-pyridazinylthio, 3-Methyl-2-oxido-6-pyridazinylthio, 3-Methoxy-1-oxido-6-pyridazinylthio, 3-Aethoxy-1-oxido-6-pyridazinylthio, 3-n-Butyloxy-1-oxido-6-pyridazinylthio oder 3-(2-Aethylhexyloxy)-1-oxido-6-pyridazinylthio, oder gegebenenfalls durch Niederalkyl, Amino, Diniederalkylamino oder Carboxy substituiertes 2-Oxo-1,2-dihydro-pyrimidinylthio, z.B. 2-Oxo-1,2-dihydro-4-pyrimidinylthio, 6-Methyl-2-oxo-1,2-dihydro-4-pyrimidinylthio, 5-Methyl-2-oxo-1,2-dihydro-4-pyrimidinylthio, 6-Amino-2-oxo-1,2-dihydro-4-pyrimidinylthio, 6-Dimethylamino-2-oxo-1,2-dihydro-4-pyrimidinylthio, 5-Carboxy-2-oxo-1,2-dihydro-4-pyrimidinylthio oder 6-Carboxy-2-oxo-1,2-dihydro-4-pyrimidinylthio oder gegebenenfalls auf die gleiche Weise substituiertes 4-Oxo-3,4-dihydro-2-pyrimidinylthio, wie insbesondere das unsubstituierte 4-Oxo-3,4-dihydro-2-pyrimidinylthio, oder gegebenfalls durch Niederalkyl, wie Methyl oder Aethyl, und bis zu zwei Oxo substituiertes Triazinyl-

BAD ORIGINAL

thio, insbesondere N-Niederalkyl-5,6-dioxo-1,2,4-triazin-3-ylthio, z.B. 1-, 2- oder 4-Methyl-5,6-dioxo-1,2,4-triazin-3-ylthio, oder die den Oxoverbindungen entsprechenden tautomeren aromatischen Hydroxyverbindungen.

Die in Verbindungen der Formel (I) vorhandenen funktionellen Gruppen, insbesondere Carboxyl- und Amino-, ferner Hydroxy- und Sulfogruppen, sind gegebenenfalls durch Schutzgruppen geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in "Methoden der organischen Chemie", Houben-Weyl, 4. Auflage, Bd. 15/1, Georg Thieme Verlag, Stuttgart, 1974.

So sind Carboxylgruppen, wie die Carboxylgruppe $R_4$, z.B. in üblicherweise in veresterter Form geschützt, wobei solche Estergruppierungen unter schonenden Bedingungen leicht spaltbar sind.

Geeignete geschützte Carboxylgruppen sind beispielsweise Niederalkoxycarbonyl, z.B. Methoxy- oder Aethoxycarbonyl, oder insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste dar-

BAD ORIGINAL

stellt, wie gegebenenfalls, z.B. wie oben erwähnt substituiertes Benzyloxycarbonyl, z.B. 4-Nitro-benzyloxycarbonyl oder 4-Methoxybenzyloxycarbonyl, oder gegebenenfalls, z.B. wie oben erwähnt substituiertes Diphenylmethoxycarbonyl, z.B. Diphenylmethoxycarbonyl oder Di-(4-methoxyphenyl)-methoxycarbonyl, oder 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, 2-($S_1$)($S_2$)($S_3$)-Silyläthoxycarbonyl, worin die Substituenten $S_1$, $S_2$ und $S_3$ unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit z.B. bis zu 15 C-Atomen, wie einen entsprechenden gegebenenfalls substituierten Niederalkyl-, Arylniederalkyl-, Cycloalkyl- oder Arylrest, bedeuten, z.B. 2-Triniederalkylsilyläthoxycarbonyl, wie 2-Trimethylsilyläthoxycarbonyl oder 2-(Dibutyl-methylsilyl)-äthoxycarbonyl, oder 2-Triarylsilyläthoxycarbonyl, wie 2-Triphenylsilyläthoxycarbonyl, oder 1-Niederalkoxyniederalkoxycarbonyl, wie Methoxymethoxycarbonyl, 1-Methoxyäthoxycarbonyl oder 1-Aethoxymethoxycarbonyl, oder 1-Niederalkylthioniederalkoxycarbonyl, wie 1-Methylthiomethoxycarbonyl oder 1-Aethylthioäthoxycarbonyl oder Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, oder Polyhalogenaryloxycarbonyl, wie Pentachlorphenyloxycarbonyl. Veresterte Carboxylgruppen sind ebenfalls entsprechende Silyloxycarbonyl-, insbesondere organische Silyloxycarbonylgruppen oder entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B.

BAD ORIGINAL

Chlor, als Substituenten. Geeignete Silyl- bzw. Stannylschutzgruppen sind in erster Linie Triniederalkylsilyl, insbesondere Trimethylsilyl, ferner Dimethyl-tert.butylsilyl, Niederalkoxy-niederalkyl-halogen-silyl, z.B. Methoxy-methyl-chlor-silyl, oder Diniederalkyl-halogensilyl, z.B. Dimethylchlor-silyl, oder entsprechend substituierte Stannylverbindungen, z.B. Tri-n-butyl-stannyl.

Bevorzugte geschützte Carboxylgruppen sind tert.-Butyloxycarbonyl, gegebenenfalls, z.B. wie erwähnt substituiertes Benzyloxycarbonyl, z.B. 4-Nitrobenzyloxycarbonyl und Diphenylmethoxycarbonyl.

Eine unter physiologischen Bedingungen spaltbare, veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet. Solche Gruppen sind Niederalkanoyloxymethoxycarbonyl, z.B. Acetyloxymethyloxycarbonyl oder Pivaloyloxymethoxycarbonyl, Aminoniederalkanoyloxymethoxycarbonyl, insbesondere α-Amino-niederalkanoyloxymethoxycarbonyl, z.B. Glycyloxymethoxycarbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Eine geschützte Aminogruppe, wie die Gruppe Am, kann z.B. in Form einer leicht spaltbaten Acylamino, Mono-, Di- oder Triarylmethylamino-, verätherten Mercaptoamino-, 1-Acyl-2-niederalkylidenamino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 C-Atomen, insbesondere einer

BAD ORIGINAL

gegebenenfalls, z.B. durch Halogen oder Aryl, substituierten aliphatischen Carbonsäure oder gegebenenfalls, z.B. durch Halogen, Niederalkoxy oder Nitro, substituierten aromatischen Carbonsäure, oder eines Kohlensäurehalbesters. Solche Acylgruppen sind beispielsweise Niederalkanoyl, wie Formyl, Acetyl, Propionyl, Halogenniederalkanoyl, wie 2-Halogenacetyl, insbesondere 2-Chlor-, 2-Brom-, 2-Jod-, 2,2-Dichlor- oder 2,2,2-Trichloracetyl, Phenylacetyl, Phenoxyacetyl, Thienylacetyl, Benzoyl, 4-Chlor-, 4-Methoxy- oder 4-Nitrobenzoyl, Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, oder 2-Halogen-niederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, 2-$(S_1)(S_2)(S_3)$-Silyläthoxycarbonyl, worin die Substituenten $S_1$, $S_2$ und $S_3$ unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie einen entsprechenden gegebenenfalls substituierten Niederalkyl-, Arylniederalkyl-, Cycloalkyl- oder Arylrest, bedeuten, z.B. 2-Triniederalkylsilyläthoxycarbonyl, wie 2-Trimethylsilyläthoxycar-

BAD ORIGINAL

bonyl oder 2-(Dibutyl-methyl-silyl)-äthoxycarbonyl, oder 2-Triarylsilyläthoxycarbonyl, wie 2-Triphenylsilyläthoxycarbonyl, oder Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl.

Weitere Acylgruppen in Acylaminogruppen sind Reste organischer Phosphor-, Phosphon- oder Phosphinsäuren der Formel $(R^1)(R^2)P(=O)-$, worin $R^1$ und $R^2$ unabhängig voneinander eine durch einen Kohlenwasserstoffrest verätherte Hydroxygruppe oder einen Kohlenwasserstoffrest bedeuten, wobei die Kohlenwasserstoffreste bevorzugt bis zu 15 C-Atome enthalten, beispielsweise aliphatischer, araliphatischer, cycloaliphatischer oder aromatischer Natur sind, gegebenenfalls z.B. durch Niederalkyl, Niederalkoxy, Aryl, Halogen oder Nitro substituiert sind, und beispielsweise Alkyl, insbesondere Niederalkyl, wie Methyl, Aethyl, Propyl, Isopropyl oder Butyl, Phenylniederalkyl, wie Benzyl, p-Nitro- oder p-Chlorbenzyl, Cycloalkyl, wie Cyclopropyl, Cyclopentyl, Cyclohexyl oder Cycloheptyl, oder Homoaryl, wie Phenyl, o-, m- oder p-Tolyl, p-Methoxyphenyl, p-Biphenylyl, p-Chlorphenyl oder p-Nitrophenyl, bedeuten. Solche Reste sind beispielsweise Dimethylphosphoryl, Diäthylphosphoryl, Dipropylphosphoryl, Diisopropylphosphoryl, Dibenzylphosphoryl, Di-p-nitrobenzylphosphoryl, Dicyclohexylphosphoryl, Diphenylphosphoryl, Phenoxy-phenyl-phosphonyl, Diäthylphosphinyl und Diphenylphosphinyl.

In einer Mono-, Di- oder Triarylmethylaminogruppe sind die Arylreste insbesondere gegebenenfalls substituierte Phenylreste. Solche Gruppen sind beispielsweise Benzyl-, Diphenylmethyl- oder Tritylamino.

Eine verätherte Mercaptogruppe in einer mit einem solchen Rest geschützten Aminogruppe is in erster Linie Arylthio oder Arylniederalkylthio, worin Aryl ins-

BAD ORIGINAL

besondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 1-Acyl-2-niederalkylidenrest ist Acyl vorzugsweise der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure oder eines Kohlensäurehalbesters, wie eines Kohlensäure-niederalkylhalbesters. Entsprechende Schutzgruppen sind in erster Linie 1-Niederalkanoyl-2-propyliden, z.B. 1-Acetyl-2-propyliden, oder 1-Niederalkoxycarbonyl-2-propyliden, z.B. 1-Aethoxycarbonyl-2-propyliden.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine Aminogruppe kann auch in protonierter Form geschützt werden; als Anionen kommen in erster Linie diejenigen von starken anorganischen Säuren, wie von Halogenwasserstoffsäuren, z.B. das Chlor- oder Bromanion, oder von Sulfonsäure, wie p-Toluolsulfonsäure, in Frage.

BAD ORIGINAL

Bevorzugte Aminoschutzgruppen sind die Acylreste von Kohlensäurehalbestern, insbesondere tert.-Butyloxycarbonyl, gegebenenfalls, z.B. wie angegeben substituiertes Benzyloxycarbonyl oder Diphenylmethoxycarbonyl, oder 2-Halogen-niederalkoxycarbonyl, wie 2,2,2-Trichloräthoxycarbonyl.

Hydroxyschutzgruppen sind z.B. Acylreste, wie 2,2-Dichloracetyl oder insbesondere die im Zusammenhang mit einer geschützten Aminogruppe genannten Acylreste von Kohlensäurehalbestern, insbesondere 2,2,2-Trichloräthoxycarbonyl, oder organischen Silyl- oder Stannylreste, ferner leicht abspaltbare 2-oxa- oder 2-thiaaliphatische oder -cycloaliphatische Kohlenwasserstoffreste, in erster Linie 1-Niederalkoxy-niederalkyl oder 1-Niederalkylthio-niederalkyl, z.B. Methoxymethyl, 1-Methoxyäthyl, 1-Aethoxy-äthyl, 1-Methylthiomethyl, 1-Methylthio-äthyl oder 1-Aethylthioäthyl, oder 2-Oxa- oder 2-Thiacycloniederalkyl mit 5-7 Ringatomen, z.B. 2-Tetrahydrofuryl oder 2-Tetrahydropyranyl oder entsprechende Thiaanaloge, sowie leicht abspaltbare, gegebenenfalls substituierte α-Phenylniederalkylreste, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Sulfogruppe ist in erster Linie eine mit einem aliphatischen, cycloaliphatischen, cycloaliphatisch-aliphatischen, aromatischen oder araliphatischen Alkohol, wie einem Niederalkanol, oder mit einem Silyl- oder Stannylrest, wie Triniederalkylsilyl, veresterte Sulfogruppe. In einer Sulfogruppe kann die Hydroxygruppe beispielsweise wie die Hydroxygruppe in einer veresterten Carboxygruppe veräthert sein.

BAD ORIGINAL

Salze sind insbesondere diejenigen von Verbindungen der Formel I mit sauren Gruppen, z.B. mit freien Carboxyl- und Sulfogruppen. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-,Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aetyhl-piperidin, Cycloalkylamine, z.B. Dicyclohexylamin, oder Benzylamine, z.B. N,N'Debenzyl-äthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure, oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer freien Carboxylgruppe und freien Aminogruppe können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen nur pharmazeutisch unbedenkliche Salze, die deshalb bevorzugt werden.

BAD ORIGINAL

Die Gruppe $=N-O-R_1$ kann in syn- (Z-) oder anti- (E-) Form vorliegen, wobei die syn-Form bevorzugt ist.

Die Aminothiadiazolylgruppe der Teilformel

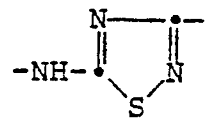

kann auch in ihrer tautomeren Form als Iminothiadiazolinyl-gruppe der Teilformel

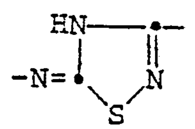

oder als Mischung beider Formen vorliegen. Das Gleichgewicht zwischen den beiden Tautomeren hängt von der Art der Substituenten und äusseren Faktoren, wie Temperatur, Lösungsmittel oder pH-Wert, ab. Im Rahmen der vorliegenden Erfindung wird diese Gruppe in der Beschreibung und in den Ansprüchen nur als "Aminothiadiazolyl" bezeichnet, obgleich die Iminothiadiazolinylform ebenfalls umfasst werden soll.

Die Verbindungen der Formel I, worin Schutzgruppen entfernt sind, worin aber Carboxylgruppen gegebenenfalls in physiologisch spaltbarer Form verestert sind, und ihre pharmazeutisch verwendbaren, nichttoxischen Salze sind wertvolle, antibiotisch wirksame Substanzen, die insbesondere als antibakterielle Antibiotika verwendet werden können. Beispielsweise sind sie in vitro gegen gram-positive und gram-negative Mikroorganismen, wie gegen Kokken, z.B. Staphylokokken, wie Staphylococcus aureus, Streptokokken, wie Streptococcus pyogenes und Streptococcus pneumoniae, Enterokokken und Mikrokokken, inklusive Neisseria Arten, z.B. Neisseria gonorrhoeae, in Minimalkonzentrationen von

BAD ORIGINAL

< 0,005 mcg/ml bis etwa 32 mcg/ml, und gegen Enterobacteriaceae, z.B. gegen *Escherichia coli*, *Klebsiella*, *Serratia*, *Enterobacter* oder *Proteus spp*, sowie gegen *Pseudomonas aeruginosa*, *Haemophilus influenzae* und andere gram-negative und gram-positive Stäbchenbakterien, in Minimalkonzentration von < 0,005 bis etwa 16 mcg/ml, wirksam. *In vivo*, bei subcutaner Application an der Maus, sind sie ebenfalls gegen gram-positive und gram-negative Bakterien, z.B. *Staphylokokken*, *Escherichia coli*, *Proteus* und *Pseudomonas aeruginosa*, in Minimaldosen von <0,15 bis etwa 100 mg/kg wirksam.

Gewisse bevorzugte Verbindungen zeichnen sich durch eine besonders lange Verweildauer im Körper aus. In der Maus beispielsweise beträgt die Halbwertszeit bis zu 60 Minuten.

Die neuen Verbindungen können deshalb entsprechend, z.B. in Form von antibiotisch wirksamen Präparaten zur Behandlung von durch gram-positive oder gram-negative Bakterien und Kokken, insbesondere durch von Enterobakterien, wie *Escherichia coli*, verursachten Infektionen Verwendung finden.

Hervorzuhebende Gruppen sind Verbindungen der Formel I, worin Am eine gegebenenfalls geschützte sekundäre oder eine tertiäre Aminogruppe darstellt und $R_1$, $R_2$, $R_3$ und $R_4$ die unter Formel I angegebenen Bedeutungen haben, oder worin $R_1$ für Wasserstoff, substituiertes Niederalkyl oder Cycloalkyl, oder gegebenenfalls N-substituiertes Carbamoyl steht und Am, $R_2$, $R_3$ und $R_4$ die unter Formel I gegebenen Bedeutungen haben, oder worin $R_2$ Niederalkoxy bedeutet und Am, $R_1$, $R_3$ und $R_4$ die unter Formel I genannten Bedeutungen haben, und die Salze von solchen Verbindungen mit salzbildenden Gruppen.

BAD ORIGINAL

Verbindungen der Formel I, worin die funktionellen Gruppen geschützt sind, werden als Ausgangsmaterialien zur Herstellung von Verbindungen der Formel I verwendet.

Die vorliegende Erfindung betrifft in erster Linie diejenigen Verbindungen der Formel I, worin Am Amino oder Methylamino, $R_1$ Wasserstoff, Niederalkyl, insbesondere Methyl, durch Carboxy substituiertes Niederalkyl oder Cycloalkyl, insbesondere 2-Carboxy-2-propyl oder 1-Carboxy-1-cyclopropyl, oder eine Gruppe -C(=O)-NHR, worin R Wasserstoff oder Niederalkyl insbesondere Methyl, bedeutet, $R_2$ Wasserstoff oder Methoxy, $R_3$ Niederalkanoyloxy, wie Acetoxy, Carbamoyloxy, N-Niederalkylcarbamoyloxy, oder gegebenenfalls substituiertes, mit einem Ringkohlenstoffatom an die Thiogruppe gebundenes Heterocyclylthio, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell hydriert und gegebenenfalls beispielsweise durch Niederalkyl, wie Methyl, N,N-Diniederalkylaminoniederalkyl, wie 2-N,N-Dimethylaminoäthyl, Carboxyniederalkyl, wie Carboxymethyl oder 2-Carboxyäthyl, Sulfoniederalkyl, wie Sulfomethyl oder 2-Sulfoäthyl, Amino, Carboxyniederalkanoylamino, Carbamoyl, Oxo oder Hydroxy substituiert sein können, und $R_4$ Carboxyl oder in physiologisch spaltbarer Form verestertes Carboxyl, z.B. Acyloxymethoxycarbonyl, darstellt, pharmazeutisch verwendbare Salze von solchen Verbindungen, sowie die zu ihrer Herstellung verwendbaren Ausgangsstoffe und Zwischenprodukte.

Besonders hervorzuheben sind Verbindungen der Formel I, worin Am Amino oder Methylamino, $R_1$ Wasserstoff, Methyl, 2-Carboxy-2-propyl, 1-Carboxy-1-cyclopropyl, Carbamoyl oder N-Methylcarbamoyl, $R_2$ Wasserstoff oder Methoxy, $R_3$ Acetoxy, Carbamoyloxy, gegebenenfalls durch Niederalkyl,

BAD ORIGINAL

Carboxyniederalkyl, Sulfoniederalkyl oder Diniederalkylamino-niederalkyl substituiertes Tetrazolylthio, z.B. 1-Methyl-1H-tetrazol-5-ylthio, 1-Sulfomethyl-1H-tetrazol-5-ylthio, 1(2-Sulfoäthyl)-1H-tetrazol-5-ylthio, 1-Carboxymethyl-1H-tetrazol-5-ylthio, 1-(2-Dimethylaminoäthyl)-1H-tetrazol-5-ylthio, gegebenenfalls durch Niederalkyl substituiertes Thiadiazolylthio, wie 2-Methyl-1,3,4-thiadiazol-5-ylthio oder 3-Methyl-1,2,4-thiadiazol-5-ylthio, gegebenenfalls durch Hydroxy substituiertes Pyridazinylthio, z.B. 3-Hydroxy-pyridazin-6-ylthio, gegebenenfalls durch Hydroxy substituiertes Pyrimidinylthio, z.B. 4-Hydroxy-2-pyrimidinyl- bzw. das dazu tautomere 4-Oxo-3,4-dihydro-2-pyrimidinylthio, oder durch Niederalkyl und zwei Hydroxy substituiertes Triazinylthio, wie 2-Niederalkyl-5,6-dihydroxy-1,2,4-triazin-3-ylthio, bzw. dazu tautomeres 1,2,5,6-Tetrahydro-2-niederalkyl-5,6-dioxo-1,2,4-triazin-3-ylthio, z.B. 1,2,5,6-Tetrahydro-2-methyl-5,6-dioxo-1,2,4-triazinyl-3-ylthio, und $R_4$ Carboxyl oder in physiologisch spaltbarer Form verestertes Carboxyl, z.B. Pivaloylmethoxycarbonyl, oder Phthalidyloxycarbonyl, bedeutet, pharmazeutisch verwendbare Salze von solchen Verbindungen, sowie die zu ihrer Herstellung verwendbaren Ausgangsstoffe und Zwischenprodukte.

Die Erfindung betrifft insbesondere die in den Beispielen beschriebenen Verbindungen der Formel I, deren pharmazeutisch unbedenklichen Salze, sowie die dort beschriebenen neuen Ausgangstoffe und Zwischenprodukte.

Die Verbindungen der vorliegenden Erfindung werden nach an sich bekannten Verfahren erhalten.

Verbindungen der Formel I und Salze von solchen Verbindungen, die eine salzbildende Gruppe aufweisen, werden beispielsweise hergestellt, indem man

BAD ORIGINAL

a) in einer Verbindung der Formel

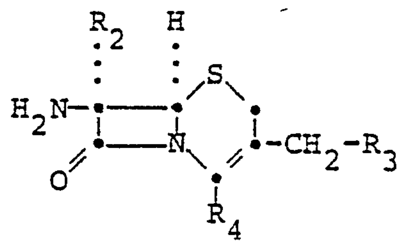

(II),

worin $R_2$, $R_3$ und $R_4$ die oben angegebenen Bedeutungen haben, und worin die 7β-Aminogruppe gegebenenfalls durch eine die Acylierung erlaubende Gruppe substituiert ist, die 7β-Aminogruppe durch Behandeln mit einem den Acylrest einer Carbonsäure der Formel

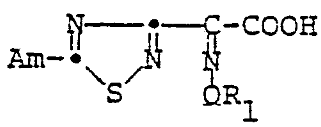

(III)

einführenden Acylierungsmittel, worin Am und $R_1$ die oben angegebenen Bedeutungen haben, acyliert oder

b) eine Verbindung der Formel

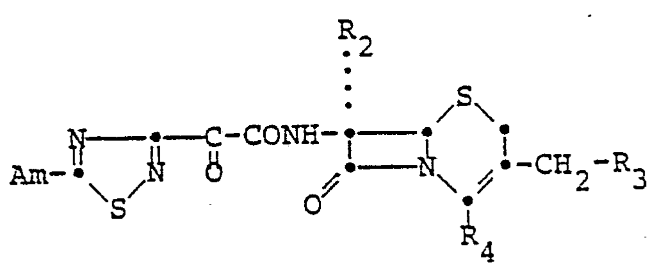

(IV)

worin Am, $R_2$, $R_3$ und $R_4$ die oben angegebenen Bedeutungen haben, mit einem Hydroxylamin der Formel $H_2N-O-R_1$ (V), worin $R_1$ die oben angegebene Bedeutung hat, umsetzt, oder

c) eine 2-Cephemverbindung der Formel

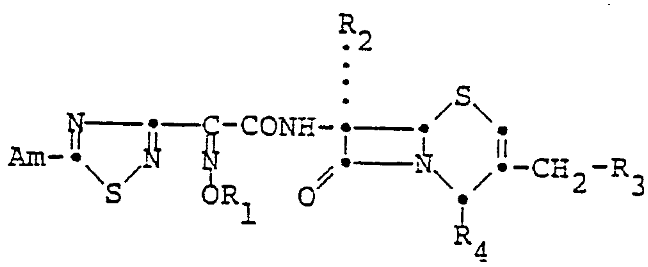

(VI),

worin Am, $R_1$, $R_2$, $R_3$ und $R_4$ die oben angegebenen Bedeutungen haben, isomerisiert, oder

d) zur Herstellung einer Verbindung der Formel (I), worin Am eine primäre oder sekundäre Aminogruppe bedeutet, eine Verbindung der Formel

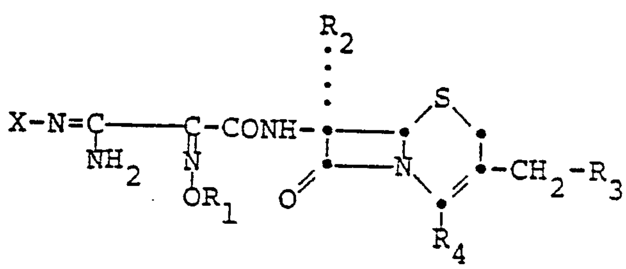

(VII),

worin X Halogen oder Hydroxy bedeutet und $R_1$, $R_2$, $R_3$ und $R_4$ die oben angegebenen Bedeutungen haben, mit einem Salz der Rhodanwasserstoffsäure, bzw. mit einem Isorhodanwasserstoffsäure-ester, oder eine Verbindung der Formel (VII), worin X Wasserstoff ist, mit Rhodan behandelt, oder

BAD ORIGINAL

e) zur Herstellung einer Verbindung der Formel (I), worin $R_1$ Wasserstoff bedeutet und worin Am, $R_2$, $R_3$ und $R_4$ die oben angegebenen Bedeutungen haben, eine Verbindung der Formel

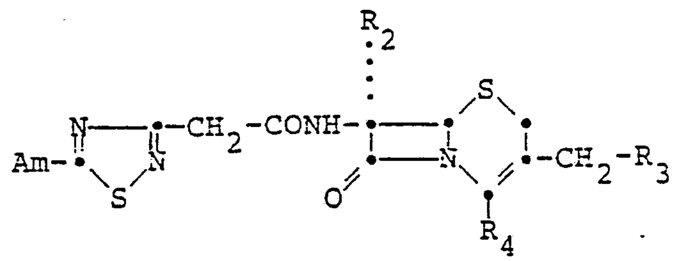

(VIII),

worin Am, $R_2$, $R_3$ und $R_4$ die oben angegebenen Bedeutungen haben, mit einem Nitrosierungsmittel behandlelt, oder

f) Zur Herstellung einer Verbindung der Formel (I), worin $R_1$ gegebenenfalls substituiertes Niederalkyl oder Cycloalkyl, oder gegebenenfalls N-substituiertes Carbamoyl bedeutet und worin Am, $R_2$, $R_3$ und $R_4$ die oben angegebene Bedeutung haben, in einer Verbindung der Formel

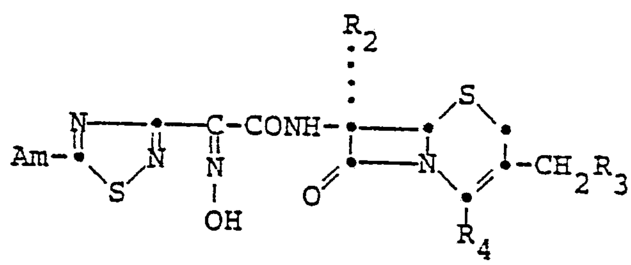

(If)

worin Am, $R_2$, $R_3$ und $R_4$ die oben gegebenen Bedeutungen haben, die Hydroxyiminogruppe mit einem den Rest $R_1$ einführenden Mittel behandelt, oder

g) zur Herstellung einer Verbindung der Formel (I), worin $R_2$ Niederalkoxy bedeutet, und worin Am, $R_1$, $R_3$ und $R_4$ die oben gegebenen Bedeutungen haben, in eine Verbindung der Formel

BAD ORIGINAL

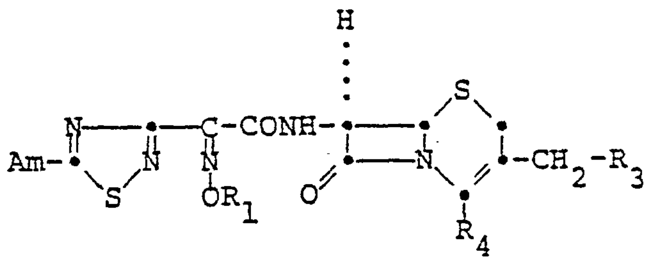

(Ig),

worin Am, $R_1$, $R_3$ und $R_4$ die oben gegebenen Bedeutungen haben, wobei funktionelle Gruppen in geschützter Form vorliegen, in 7α-Stellung eine Niederalkoxygruppe einführt, oder

h) zur Herstellung einer Verbindung der Formel (I), worin $R_3$ für Heterocyclthio steht und worin Am, $R_1$, $R_2$ und $R_4$ die oben gegebenen Bedeutungen haben, eine Verbindung der Formel

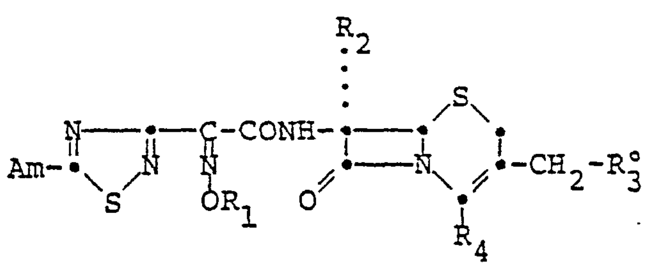

(Ih),

worin Am, $R_1$, $R_2$ und $R_4$ die oben gegebenen Bedeutungen haben, und $R_3^\circ$ einen durch einen Heterocyclylthiorest $R_3$ ersetzbaren Rest bedeutet, mit einem Mercaptan H-$R_3$, behandelt, oder

i) zur Herstellung einer Verbindung der Formel (I), worin $R_3$ für Acyloxy steht und worin Am, $R_1$, $R_2$ und $R_4$ die oben gegebenen Bedeutungen haben, eine Verbindung der Formel

BAD ORIGINAL

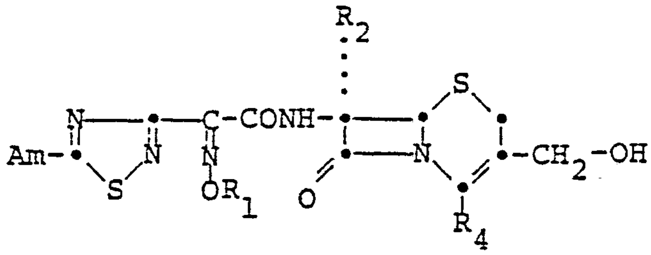

(Ii),

worin Am, $R_1$, $R_2$ und $R_4$ die oben gegebenen Bedeutungen haben, mit einem Acylierungsmittel behandelt, oder

j) zur Herstellung einer Verbindung der Formel (I), worin $R_4$ freies Carboxyl darstellt und worin Am, $R_1$, $R_2$ und $R_3$ die oben gegebenen Bedeutungen haben, in einer Verbindung der Formel

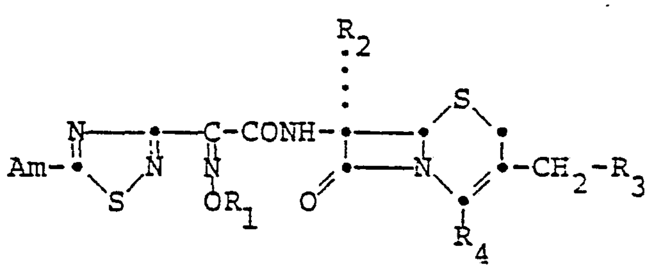

(Ij),

worin Am, $R_1$, $R_2$ und $R_3$ die oben gegebenen Bedeutungen haben und $R_4$ eine geschützte Carboxylgruppe darstellt, die Carboxylschutzgruppe entfernt und durch Wasserstoff ersetzt, oder

k) zur Herstellung einer Verbindung der Formel (I), worin $R_4$ eine veresterte Carboxylgruppe darstellt und worin Am, $R_1$, $R_2$ und $R_3$ die oben gegebenen Bedeutungen haben, in einer Verbindung der Formel

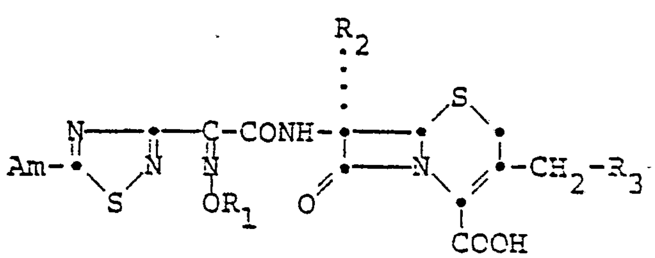

(Ik),

BAD ORIGINAL

worin Am, $R_1$, $R_2$ und $R_3$ die oben gegebenen Bedeutungen haben, die 4-Carboxylgruppe verester, oder

l) zur Herstellung von Verbindungen der Formel (I), worin Am eine primäre, sekundäre oder tertiäre Aminogruppe ist, eine Verbindung der Formel

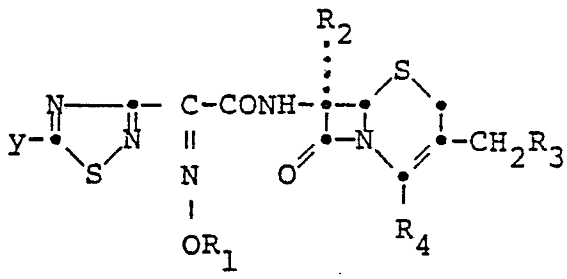

(XVIII)

worin y Halogen ist und $R_1$, $R_2$, $R_3$ und $R_4$ die oben angegebenen Bedeutungen haben, mit Ammoniak oder einem primären oder sekundären Amin Am-H, oder einem Metallamid davon, umsetzt, und, wenn notwendig, in einer erhaltenen Verbindung nicht erwünschte Schutzgruppen abspaltet, und/oder, wenn erwünscht, eine erhaltene Verbindung mit salzbildender Gruppe in ein Salz, oder ein erhaltenes Salz in eine freie Verbindung oder in ein anderes Salz überführt.

<u>Verfahren a) (Acylierung)</u>: Gegebenenfalls vorhandene, die Aminogruppe substituierende und deren Acylierung erlaubende Reste in einem Ausgangsmaterial der Formel II sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen. Bei der Silylierung oder Stannylierung einer Carboxylgruppe in einem Ausgangsmaterial

BAD ORIGINAL

der Formel II, kann, bei Verwendung eines Ueberschusses des Silylierungs- oder Stannylierungsmittels, die Aminogruppe ebenfalls silyliert oder stannyliert werden.

Die genannten Ylidengruppen sind in erster Linie Arylmethylengruppen, worin Aryl insbesondere für einen carbocyclischen, in erster Linie monocyclischen Arylrest, z.B. für gegebenenfalls, wie durch Nitro oder Hydroxy, substituiertes Phenyl steht.

Die übrigen in den Ausgangsstoffen der Formel II vorhandenen funktionellen Gruppen können durch die bereits unter den Verbindungen der Formel I genannten Schutzgruppen geschützt sein. Bevorzugt sind alle nicht an der Acylierungsreaktion teilnehmenden reaktionsfähigen funktionellen Gruppen, insbesondere aber gegebenenfalls vorhandene, acylierbare Amino-, Hydroxy- und Mercaptogruppen, geschützt.

Den Acylrest einer Carbonsäure der Formel III einführende Acylierungsmittel sind beispielsweise die Carbonsäure selbst oder reatkionsfähige funktionelle Derivate davon.

Falls eine freie Säure der Formel III, worin alle funktionellen Gruppen ausser der reagierenden Carboxylgruppe geschützt sein können, zur Acylierung eingesetzt wird, verwendet man üblicherweise geeignete Kondensationsmittel, wie Carbodiimide, beispielsweise N,N'-Diäthyl-, N,N'-Dipropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazolinium-3'-sulfonat und N-tert.-Butyl-5-methyl-isoxazolinium-perchlorat, oder eine Acylaminoverbindung, z.B. 2-Aethoxy-1-äthoxycarbonyl-1,2-dihydro-chinolin.

BAD ORIGINAL

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. Amid-bildendes funktionelles Derivat einer Säure der Formel III, worin alle funktionellen Gruppen ausser der reagierenden Säuregruppe geschützt sein können, ist in erster Linie ein Anhydrid einer solchen Säure, inklusive und vorzugsweise ein gemischtes Anhydrid. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phophorhaltigen Säure, z.B. Phosphorsäure, Diäthylphosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

BAD ORIGINAL

Weitere zur Reaktion mit der Aminogruppe geeignete Säurederivate in einer Säure der Formel III, worin alle funktionellen Gruppen ausser der reagierenden Carboxylgruppe geschützt sein können, sind aktivierte Ester, wie Ester mit vinylogen Alkoholen (d.H. Enolen, wie vinylogen Niederalkenolen, oder Iminomethylesterhalogeniden, wie Dimethyliminomethylesterchlorid (hergestellt aus der Carbonsäure und Dimethyl-chlormethyliden-iminium-chlorid der Formel $[(CH_3)_2N{=}CHCl]^{\oplus}Cl^{\ominus}$), oder Arylester, wie 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem Säurederivat, wie einem Anhydrid, insbesondere mit einem Säurehalogenid, wird bevorzugt in Anwesenheit eines säurebindenden Mittels,beispielsweise einer organischen Base,wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

BAD ORIGINAL

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig, bei erniedrigter oder erhöhter Temperatur, etwa bei $-40^{o}$ bis etwa $100^{o}$, bevorzugt bei $-10^{o}$ bis $+40^{o}$, und/oder in einer Inertgas-, z.B. Stickstoffatmosphäre.

Die Acylierung kann auch in Gegenwart einer die gewünschte Amidbindung herstellenden Acylase erfolgen. Solche Acylasen sind bekannt und werden durch eine Reihe von Mikroorganismen gebildet, z.B. durch Acetobacter, wie Acetobacter aurantium, Achromobacter, wie Achromobacter aeris, Aeromonas, wie Aeromonas hydrophila, oder Bacillus megaterium, z.B. 400. In die enzymatische Acylierung werden insbesondere Amide, Ester oder Thioester, wie entsprechende Niederalkyl-, z.B. Methyl- oder Aethylester, der Carbonsäure der Formel III eingesetzt. Die enzymatische Acylierung wird in einem den Mikroorganismus enthaltenden Nährmedium, in einem Filtrat der Kulturbrühe oder gegebenenfalls nach Isolierung der Acylase, inklusive nach Adsorption an einen Träger, in einem wässrigen, gegebenenfalls einen Puffer enthaltenden Medium bei etwa 30-40°, bevorzugt bei etwa 37°, durchgeführt.

BAD ORIGINAL

In einer acylierenden Säure der Formel III oder in einem Säurederivat davon kann eine geschützte Aminogruppe auch in ionischer Form vorliegen, d.h. das Ausgangsmaterial der Formel III kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, in situ gebildet werden. So erhält man z.B. ein gemischtes Anhydrid durch Behandeln einer Säure der Formel III, mit entsprechend geschützten funktionellen Gruppen, oder eines geeigneten Salzes davon, wie eines Ammoniumsalzes, z.B. mit einem organischem Amin, wie Pyridin oder 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, mit einem Halbester eines Kohlensäurehalbhalogenids, z.B. Chlorameisensäureäthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Verfahren b) (Umsatz der Oxomethylengruppe mit Hydroxylaminen): In einer Verbindung der Formel IV, worin funktionelle Gruppen gegebenenfalls geschützt sind, wird die Oxogruppe in der 7ß-Acylamidogruppe durch Behandeln mit Hydroxylamin oder einem substituierten Hydroxylamin der Formel $H_2N-O-R_1$ (V) in eine Hydroxyiminomethylen- bzw. $R_1$-substituierte Hydroxyiminomethylengruppe übergeführt.

BAD ORIGINAL

Die Reaktion der Oxogruppe mit der Hydroxylaminverbindung wird auf übliche Weise ausgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, wie zwischen etwa -20° bis etwa +100°, bevorzugt bei etwa 0° bis etwa 30°, gegebenenfalls in einer Inertgas-, wie Stickstoffatmosphäre, reagieren lässt. Die Hydroxylaminverbindung kann, auch in situ, aus einem ihrer Salze, beispielsweise einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triniederalkylamin, wie Triäthylamin oder Aethyl-diisopropylamin, oder einer heterocyclischen tertiären Base, wie Pyridin, in Freiheit gesetzt werden.

Verfahren c) (Isomerisierung): In einer 2-Cephem-Verbindung der Formel VI weist die gegebenenfalls geschützte Carboxylgruppe in 4-Stellung vorzugsweise die α-Konfiguration auf.

2-Cephem-Verbindungen der Formel VI werden isomerisiert, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters,

BAD ORIGINAL

in erster Linie Basen des Pyridin-Typs, wie Pyridin selber, sowie Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphatische oder araliphatische Basen, wie N,N,N-Triniederalkylamine, z.B. N,N,N-Trimethylamin, N,N-Diisopropyl-N-äthylamin, N-Niederalkyl-azacycloalkane, z.B. N-Methyl-piperidin, oder N-Phenyl-niederalkyl-N,N-diniederalkyl-amine, z.B. N-Benzyl-N,N-dimethylamin, sowie Gemische davon, wie das Gemisch einer Base vom Pyridintyp und eines N,N,N-Tri-niederalkylamins, z.B. Pyridin und Triäthylamin. Ferner können auch anorganische oder organische Salze von Basen, insbesondere von mittelstarken bis starken Basen mit schwachen Säuren, wie Alkalimetall-, oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei Isomerisierung mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösungsmittelgemisches, wobei als Reaktionsmittel verwendete, unter den Reaktionsbedingungen flüssige Basen gleichzeitig auch als Lösungsmittel dienen können, unter Kühlen, bei Zimmertemperatur oder unter Erhitzen, vorzugsweise in einem Temperaturbereich von etwa -30°C bis etwa +100°C, in einer Inertgas-, z.B. Stickstoffatmosphäre, und/oder in einem geschlossenen Gefäss.

BAD ORIGINAL

Die so erhältlichen 3-Cephem-verbindungen lassen sich in an sich bekannter Weise, z.B. durch Adsorption und/oder Kristallisation, von gegebenenfalls noch vorhandenen 2-Cephem-verbindungen abtrennen.

Die Isomerisierung von 2-Cephem-verbindungen der Formel VI wird bevorzugt durchgeführt, indem man diese in 1-Stellung oxidiert, wenn erwünscht, ein erhältliches Isomerengemisch der 1-Oxide trennt, und die so erhältliches 1-Oxide der entsprechenden 3-Cephem-verbindungen reduziert.

Als geeignete Oxidationsmittel für die Oxidation in 1-Stellung von 2-Cephem-verbindungen kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens $10^{-5}$ in Frage. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxyd und einer Carbonsäure in situ gebildet werden können. Dabei ist es zweckmässig, einen grossen Ueberschuss der Carbonsäure zu verwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

BAD ORIGINAL

Die Oxidation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20%, wobei man auch grössere Ueberschüsse, d.h. bis zur 10-fachen Menge des Oxidationsmittels oder darüber, verwenden kann. Die Oxidation wird unter milden Bedingungen, z.B. bei Temperaturen von etwa -50°C bis etwa +100°C, vorzugsweise von etwa -10°C bis etwa +40°C durchgeführt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten, und die man gegebenenfalls zusam-

BAD ORIGINAL

men mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; reduzierende trivalente anorganische oder organische Phosphorverbindungen, wie Phosphine, ferner Ester, Amide und Halogenide der phosphinigen, phosphonigen oder phosphorigen Säure, sowie diesen Phosphorsauerstoffverbindungen entsprechenden Phosphor-Schwefelverbindungen, worin organische Reste in erster Linie aliphatische, aromatische oder araliphatische Reste, z.B. gegebenenfalls substituierte Niederalkyl-, Phenyl oder Phenylniederalkylgruppen darstellen, wie z.B. Triphenylphosphin, Tri-n-butylphosphin, Diphenylphosphinigsäuremethylester, Diphenylchlorphosphin, Phenyldichlorphosphin, Benzolphosphonigsäuredimethylester, Butanphosphonigsäuremethylester, Phosphorigsäuretriphenylester, Phosphorigsäuretrimethylester, Phosphortrichlorid, Phosphortribromid, etc.; reduzierende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenen-

BAD ORIGINAL

falls substituierte Niederalkyl- oder Phenylgruppen aufweisen können, wie Chlorsilan, Bromsilan, Di- oder Trichlorsilan, Di- oder Tribromsilan, Diphenylchlorsilan, Dimethylchlorsilan, Trimethyljodsilan oder auch Halogensilanverbindungen, worin alle Wasserstoffe durch organische Reste ersetzt sind, wie Triniederalkylhalogensilan, z.B. Trimethyljodsilan, oder cyclische, schwefelhaltige Silane, wie 1,3-Dithia-2,4-disilacyclobutane oder 1,3,5-Trithia-2,4,6-trisila-cyclohexane, deren Siliciumatome durch Kohlenwasserstoffreste, wie insbesondere Niederalkylreste substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituierte Niederalkylen- oder Niederalkylgruppen substituiert ist, wie N-Chlormethylen-N,N-diäthyliminiumchlorid oder N-Chlormethylen-pyrrolidiniumchlorid;und komplexe Metallyhdride, wie Natriumborhydrid, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. die in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind inbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäure-

BAD ORIGINAL

chlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, 1,3-Propansulton, 1,4-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B.Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20° C bis etwa 100° C, wobei bei Verwendung von sehr reaktionsfähigen Aktivierungsmitteln die Reaktion bei tieferen Temperaturen durchgeführt werden kann.

BAD ORIGINAL

Verfahren d) (Thiadiazolringbildung): In einer Verbindung der Formel VII ist X als Halogen beispielsweise Chlor, Jod oder bevorzugt Brom. Geeignete Salze der Rhodanwasserstoffsäure (Thiocyansäure) sind beispielsweise Alkali-, Erdalkali- oder auch Schwermetallsalze, z.B. das Natrium-, Kalium-, Magnesium- oder Bleisalz, oder auch Ammoniumsalze, die von Ammoniak oder organischen Stickstoffbasen abgeleitet sind, z.B. das Ammoniumsalz, oder Mono-, Di-, Tri- oder Tetraniederalkylammoniumsalze, worin Niederalkyl Methyl, Aethyl, Isopropyl oder dergleichen bedeutet.

Geeignete Isorhodanwasserstoffsäureester sind beispielsweise Isorhodanwasserstoffsäureniederalkylester, die auch als Niederalkylisothiocyanate bezeichnet werden, wie Methyl-, Aethyl-, Propyl- oder Butylisothiocyanat.

Die Kondensation von Verbindungen der Formel VII mit einem Salz der Rhodanwasserstoffsäure oder mit Rhodan ergibt Verbindungen der Formel (I), worin Am eine freie, primäre Aminogruppe ist, während die Kondensation mit einem Isorhodanwasserstoffsäureester eine Verbindung der Formel (I) ergibt, worin Am eine freie sekundäre Aminogruppe, beispielsweise eine Niederalkylaminogruppe ist.

Die Reaktion wird bevorzugt in einem inerten Lösungsmittel, wie einem niederen Alkohol, z.B. Methanol, Aethanol, Propanol oder Isopropanol, einem Aether, wie Diäthyläther, Dioxan oder Tetrahydrofuran einem Amid wie Dimethylformamid, einem aliphatischen oder aromatischen Kohlenwasserstoff, wie Hexan, Benzol oder Toluol und dergleichen, oder auch in Mischungen davon, gegebenenfalls unter Kühlen oder Erwärmen, d.h. bei Temperaturen zwischen etwa -80° bis etwa +100°, bevorzugt zwischen etwa -20° bis etwa +20°, gegebenenfalls in einer Inertgasatmosphäre, durchgeführt.

BAD ORIGINAL

Eine Verbindung der Formel VII, worin X Halogen ist, kann auch in situ hergestellt werden, gegebenenfalls in Gegenwart des Rhodanwasserstoffsalzes, beispielsweise indem man eine Verbindung der Formel VII, worin X Wasserstoff ist, in einem der genannten Lösungsmittel mit einem Hypohalogenit, z.B. Natrium- oder Kaliumhypobromit, oder mit einem Halogen und einer Alkalimetallbase, wie einem Hydroxid, Karbonat oder Alkoholat, z.B. mit Brom und Natriummethylat oder Kaliumcarbonat, behandelt. Diese Halogenierung findet bevorzugt unter Kühlen, z.B. bei etwa -15° bis etwa 0° statt.

Eine Verbindung der Formel VII, worin X Hydroxy ist, kann ebenfalls in situ hergestellt werden, beispielsweise indem man eine Verbindung der Formel VII, worin X Wasserstoff ist, mit einem Oxidationsmittel, wie Wasserstoffperoxid, behandelt.

Die Reaktion einer Verbindung der Formel VII, worin X Wasserstoff ist, mit dem Rhodan, findet ebenfalls in einem der genannten inerten Lösungsmittel und unter ähnlichen Reaktionsbedingungen statt. Gegebenenfalls kann auch ein Säureadditionssalz einer Verbindung der Formel VII eingesetzt werden, das in situ in die freie Verbindung übergeführt wird, oder das Rhodan kann in situ gebildet werden, z.B. aus einem Rhodanid, z.B. Bleirhodanid, und einem Halogen, z.B. Brom.

Verfahren e) (Nitrosierung der Methylengruppe): In einer Verbindung der Formel VIII sind die funktionellen Gruppen bevorzugt geschützt.

BAD ORIGINAL

Geeignete Nitrosierungsmittel sind salpetrige Säure und davon abgeleitete Derivate, wie Nitrosylhalogenide, z.B. Nitrosylchlorid oder Nitrosylbromid, Salze der salpetrigen Säure, wie Alkalimetallsalze, z.B. Natrium- oder Kaliumnitrit, oder insbesondere Ester der salpetrigen Säure, wie Niederalkylester, z.B. Butyl-, Pentyl- oder insbesondere iso-Amylnitrit.

Wenn als Nitrosierungsmittel ein Salz der salpetrigen Säure eingesetzt wird, wird die Raktion vorzugsweise in Gegenwart einer starken anorganischen oder auch organischen Säure, wie Chlorwasserstoffsäure, Schwefelsäure, Ameisensäure oder Essigsäure, durchgeführt. Bei Verwendung eines Esters der salpetrigen Säure wird die Reaktion vorzugsweise in Gegenwart einer starken Base, wie eines Alkalimetallalkylates durchgeführt.

Die Nitrosierung wird in einem geeigneten Lösungsmittel, wie Wasser, einer Carbonsäure, z.B. Essigsäure, einem niederen Alkohol, z.B. Methanol oder Aethanol, einem Aether, z.B. Dioxan oder Tetrahydrofuran, oder einem Kohlenwasserstoff, z.B. Hexan oder Benzol, oder in Mischungen davon, unter Kühlung oder Erwärmung, insbesondere bei etwa -15° bis Raumtemperatur, durchgeführt.

Verfahren f) (Alkylierung und Carbamoylierung der Hydroxyiminogruppe): In dem Ausgangsmaterial der Formel If sind alkylierbare bzw. carbamoylierbare Gruppen, ausser der reagierenden Hydroxyiminogruppe, bevorzugt geschützt.

BAD ORIGINAL

Die Verätherung der freien Hydroxygruppe wird mit einem den gewünschten, gegebenenfalls substituierten Niederalkyl- oder Cycloalkylrest $R_1$ einführenden konventionellen Verätherungsmittel durchgeführt.

Geeignete Verätherungsmittel sind beispielsweise Diazoverbindungen, wie Diazoniederalkane oder -cycloalkane, z.B. Diazomethan, Diazoäthan oder Diazo-n-butan, die gegebenenfalls im Alkanteil substituiert sein können. Diese Reagentien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und je nach Diazoreagens unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls, z.B. durch Niederalkyl, wie Methyl, Halogen, wie Brom oder Nitro substituierten Benzolsulfonsäuren, z.B.Methansulfon-, Trifluormethansulfon- oder p-Toluolsulfonsäure, z.B.

BAD ORIGINAL

Diniederalkylsulfate, wie Dimethylsulfat, ferner Fluorsulfonsäureniederalkylester z.B. Fluorsulfonsäuremethylester oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organischen Basen, wie, üblicherweise sterische gehinderte, Triniederalkylamine, z.B. N,N-Diisopropyl-N-äthyl-amin (vorzugsweise zusammen mit Niederalkyl-halogensulfaten oder gegebenenfalls Halogensubstituierten Methansulfonsäure-niederalkylestern) an, wobei unter Kühlen, bei Raumtemperatur oder unter Erwärmen, z.B. bei Temperaturen von etwa -20°C bis etwa 50° C und, wenn notwendig, in einem geschlossenen Gefäss und/oder in einer Inertgas-, z.B. Stickstoffatmosphäre gearbeitet wird.

Weitere Verätherungsmittel sind entsprechende Acetale z.B. gem-Niederalkoxyniederalkane, wie 2,2-Dimethoxy-propan, die in Gegenwart einer starken organischen Sulfonsäure, wie p-Toluolsulfonsäure, und eines geeigneten

BAD ORIGINAL

Lösungsmittels, wie eines Diniederalkyl- oder Niederalkylensulfoxyds, z.B. Dimethylsulfoxyd, zur Anwendung gelangen, oder Orthoester, z.B. Orthoameisensäure-triniederalkylester, z.B. Orthoameisensäure-triäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden.

Weitere Verätherungsmittel sind entsprechende Tri-$R_1$-oxoniumsalze (sogenannte Meerweinsalze), Di-$R_1$-O-Carbeniumsalze oder Di-$R_1$-Haloniumsalze, worin $R_1$ den einzuführenden Rest darstellt, beispielsweise Triniederalkyloxoniumsalze, sowie Diniederalkoxycarbenium- oder Diniederalkylhaloniumsalze, insbesondere die entsprechenden Salze mit komplexen, fluorhaligen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxoniumhexafluorantimonat, -hexachlorantimonat, -hexafluorphosphat, oder -tetrafluorborat, Dimethoxycarbeniumhexafluorphosphat oder Dimethylbromonium-hexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20° C bis etwa 50° C, wenn notwendig, in einem geschlossenen Gefäss und/oder in einer Inertgas-, z.B. Stickstoffatmosphäre.

BAD ORIGINAL

Weitere Verätherungsmittel sind schliesslich entsprechende 1-$R_1$-3-Aryltriazenverbindungen, worin $R_1$ den einzuführenden Rest und Aryl vorzugsweise einen gegebenenfalls substituierten Phenylrest, z.B. Niederalkylphenyl, wie 4-Methylphenyl bedeutet. Solche Triazenverbindungen sind 3-Aryl-1-niederalkyl-triazene, z.B. 3-(4-Methylphenyl)-1-methyl-triazin. Diese Reagentien werden üblicherweise in Gegenwart von inerten Lösungsmitteln, wie gegebenenfalls halogenierten Kohlenwasserstoffen oder Aethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig, in einem geschlossenen Gefäss und/oder in einer Inertgas-, z.B. Stickstoffatmosphäre verwendet.

Die Einführung einer Carbonylgruppe -C(=O)-NHR anstelle des Wasserstoffatoms geschieht auf an sich bekannte Weise durch Carbamoylierung. Zu diesem Zwecke wird eine Verbindung der Formel (If), worin alle reaktionsfähigen Gruppen die nicht carbamoyliert werden sollen oder die die Reagentien verbrauchen und zerstören würden, in geschützter Form vorliegen, gegebenenfalls stufenweise mit einem Carbamoylierungsmittel behandelt. Geeignete Carbamoylierungsmittel sind reaktionsfähige Derivate der Carbamidsäuren R-NH-C(=O)OH, insbesondere die Isocyanate R-N=C=O oder die gemischten Anhydride, z.B. die Carbamidsäurechloride R-NH-C(=O)Cl, die auch in situ aus Phosgen und einem Amin $R-NH_2$ hergestellt werden können.

Der Umsatz mit den Isocyanaten erfolgt in einem der üblichen inerten Lösungsmittel, beispielsweise einem gegebenenfalls halogenierten Kohlenwasserstoff, z.B. Benzol, Xylol oder Methylenchlorid, einem Aether, z.B. Diäthyläther, Dio-

BAD ORIGINAL

xan oder Tetrahydrofuran, oder einem Ester, z.B. Essigsäureäthylester, gegebenenfalls in Gegenwart eines basischen Katalysators, beispielsweise eines tertiären Amins, z.B. Triäthylamin oder Pyridin, bei Raumtemperatur oder etwas erniedrigter oder erhöhter Temperatur, etwa zwischen -20° und +50°.

Zum Umsatz mit einem gemischten Anhydrid der Carbamidsäure wird die Alkoholgruppe mit Vorteil zunächst metallisiert, beispielsweise mit einem Alkalimetallhydrid, z.B. Natrium- oder Lithiumhydrid. Die an der Hydroxygruppe metallierte Verbindung der Formel If) kann auch zunächst mit Phosgen und anschliessend mit dem Amin $R-NH_2$ behandelt werden und auf diese Art stufenweise carbamoyliert werden. Diese Reaktionen finden ebenfalls in einem inerten Lösungsmittel und bei den oben angegebenen Temperaturen statt.

Verfahren g) (Alkoxylierung der 7α-Stellung): In einer Verbindung der Formel If, worin alle funktionellen Gruppen in geschützter Form vorliegen, kann die 7α-Niederalkoxygruppe $R_2$ auf an sich bekannte Weise eingeführt werden, beispielsweise indem man das genannte Zwischenprodukt nacheinander mit einem Anionenbildenden Mittel, einem N-Halogenierungsmittel und einem Niederalkanol behandelt.

Ein geeignetes Anion-bildendes Mittel ist in erster Linie eine metallorganische Base, insbesondere eine Alkalimetall-, in erster Linie eine Lithium-organische Base. Solche Verbindungen sind insbesondere entsprechende Alkoholate, wie geeignete Lithium-niederalkanolate, in erster Linie Lithiummethylat, oder entsprechende Metall-Kohlenwasserstoffbasen, wie Lithium-niederalkane und Lithiumphenyl. Die Umsetzung mit der Anion-bildenden metallorganischen Base wird üblicherweise unter Kühlen, z.B. von etwa 0° C bis etwa -80° C, und in Gegenwart eines geeigneten Lösungs- oder Verdünnungsmittels, z.B. eines

BAD ORIGINAL

Aethers, wie Tetrahydrofuran, bei Verwendung von Lithiummethylat auch in Gegenwart von Methanol, und, wenn erwünscht, in einem geschlossenen Gefäss und/oder in einer Inertgas-, z.B. Stickstoffatmosphäre, vorgenommen.

Als N-halogenierendes Mittel verwendet man üblicherweise ein sterisch gehindertes, organische Hypohalogenit, insbesondere -chlorit, und in erster Linie ein entsprechendes aliphatisches Hypohalogenit, z.B. -chlorit, wie ein tert.-Niederalkyl-hypohalogenit, z.B. -chlorit. In erster Linie wendet man das tert.-Butylhypochlorit an, das man mit dem nichtisolierten Produkt der Anionisierungsreaktion umsetzt.

Die N-halogenierte Zwischenverbindung wird bei Anwesenheit eines Ueberschusses der Anion-bildenden Base, insbesondere von Lithiummethylat, unter den Reaktionsbedingungen und ohne isoliert zu werden in eine 7-Acyliminocephemverbindung umgewandelt, und diese durch Zugabe des Niederalkanols in eine 7α-Niederalkoxy-cephem-Verbindung übergeführt. Falls notwendig, müssen aus dem N-halogenierten Zwischenprodukt die Elemente der Halogenwasserstoff-, insbesondere der Chlorwasserstoffsäure, abgespalten werden; dies geschieht unter Zugabe einer Halogenwasserstoff-abspaltenden Base, wie eines geeigneten Alkalimetall-niederalkanolats, z.B. Lithium-tert.-butylat, wobei diese Reaktion üblicherweise unter den Bedingungen der Anion- und N-Halogenverbindung-bildenden Reaktion stattfindet, wobei man in Gegenwart von Methanol arbeiten und anstelle der Acyliminoverbindung direkt die 7α-Niederalkoxy-cephem-Verbindung erhalten kann. Man geht üblicherweise aus von einer Verbindung der Formel If, worin funktionelle Gruppen in geschützter Form vorliegen, setzt diese mit einem Ueberschuss des Anion-bildenden Mittels, z.B. Lithiummethylat oder Phenyllithium, in Gegenwart von Methanol um, behandelt dann mit dem N-Halogenierungsmittel, z.B. tert.-Butylhypochlorit,

BAD ORIGINAL

und erhält so direkt die gewünschte Verbindung der Formel I, worin funktionelle Gruppen geschützt sind. Man kann auch das Niederalkanol nachträglich zugeben, wobei man die Dehydrohalogenierung und die Zugabe des Niederalkanols bei etwas höheren Temperaturen als die Anion- und N-Halogenverbindung-bildenden Reaktionen, z.B. bei etwa 0°C bis etwa -20°C, wenn notwendig, in einem geschlossenen Gefäss und/oder in einer Inertgas-, z.B. Stickstoffatmosphäre, durchführen kann.

Bei vorstehenden Reaktionen, die unter basischen Bedingungen durchgeführt werden, können 3-Cephemverbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Verfahren h) (Thiolisierung): In einem Ausgangsmaterial der Formel Ih ist ein geeigneter, durch eine Heterocyclylthiogruppe ersetzbarer Rest $R_3^o$ beispielsweise eine der genannten Acyloxygruppen $R_3$, wie eine durch eine gegebenenfalls substituierte niederaliphatische Carbonsäure veresterte Hydroxygruppe, oder eine der genannten Sulfonyloxygruppen $R_3$. Solche veresterten Hydroxygruppen $R_3^o$ sind insbesondere Acetyloxy, ferner Formyloxy und Acetoacetyl; sowie Mesyloxy oder Tosyloxy.

Die Reaktion einer solchen Verbindung mit einer heterocyclischen Mercaptanverbindung $H-R_3$ kann unter neutralen oder schwach basischen Bedingungen in Gegenwart von Wasser und gegebenenfalls einem mit Wasser mischbaren organischen Lösungsmittel durchgeführt werden. Die basischen Bedingungen können beispielsweise durch Zugabe einer anorganischen Base, wie eines Alkalimetall- oder Erdalkali-

BAD ORIGINAL

metallhydroxids, -carbonats oder -hydrogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, oder Nitrile, z.B. Niederalkansäurenitrile, wie Acetonitril, und ähnliche verwendet werden. Die Reaktion wird bei Raumtemperatur oder, je nach Reaktionsfähigkeit, unter Kühlen oder leichtem Erwärmen durchgeführt. Gegebenenfalls kann die Thiolisierung in Gegenwart von in Wasser löslichen Salzen der Jodwasserstoffsäure und/oder der Thiocyansäure, wie entsprechenden Alkali-, Erdalkali-, Ammonium- oder quaternären Ammoniumsalzen einschliesslich phasentransferierenden Salzen, erfolgen. Geeignete Salze sind beispielsweise Lithium-, Kalium-, Magnesium-, Calcium-, und insbesondere Natrium- und Tetra-n.butylammoniumjodid. Die Reaktion wird dann bevorzugt in konzentrierten wässrigen Lösungen dieser Salze durchgeführt.

Verfahren i) (Acylierung der 3-Hydroxymethylgruppe):

In einem Ausgangsmaterial der Formel If liegen bevorzugt alle acylierbaren Gruppen, ausser der zu acylierenden 3-Hydroxymethylgruppe, in geschützter Form vor.

Die Acylierung erfolgt nach an sich bekannten Methoden durch Behandeln mit einem den gewünschten Acylrest einführenden Acylierungsmittel. Geeignete Acylierungsmittel sind die freie Säure selbst oder ein reaktionsfähiges funktionelles Derivat davon, wie ein Anhydrid, inclusive ein gemischtes Anhydrid oder ein inneres Anhydrid, oder ein aktivierter Ester.

Falls eine freie Säure $H-R_3$ zur Acylierung eingesetzt wird, verwendet man geeignete Kondensationsmittel, wie Carbodiimide, beispielsweise N,N'-Diäthyl-, N,N'-Di-

BAD ORIGINAL

propyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazolinium-3'-sulfonat und N-tert.-Butyl-5-methyl-isoxazoliniumperchlorat, oder eine Acylaminoverbindung z.B. 2-Aethoxy-1-äthoxycarbonyl-1,2-dihydrochinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. Ester-bildendes funktionelles Derivat einer Säure ist in erster Linie ein Anhydrid einer solchen Säure, inklusive und vorzugsweise ein gemischtes Anhydrid, aber auch ein inneres Anhydrid, d.h. ein entsprechendes Keten oder ein entsprechendes Isocyanat. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäure, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

BAD ORIGINAL

Zur Reaktion mit der Hydroxygruppe geeignete aktivierte Ester sind z.B. Ester mit vinylogen Alkoholen (d.h. Enolen), wie vinylogen Niederalkenolen, oder Iminomethylesterhalogeniden, wie Dimethyliminomethylesterchlorid (hergestellt aus der Carbonsäure und Dimethyl-chlormethyliden-iminium-chlorid der Formel $[(CH_3)_2N=CHCl]^{\oplus} Cl^{\ominus}$), oder Arylester, wie 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amines, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon,

BAD ORIGINAL

bei Raumtemperatur, wenn notwendig, bei erniedrigter oder erhöhter Temperatur, etwa bei -40° bis etwa +100°, bevorzugt bei -10° bis +40°, und/oder in einer Inertgas-, z.B. Stickstoffatmosphäre.

Ferner kann ein Säurederivat, wenn erwünscht, in situ gebildet werden. So erhält man z.B. ein gemischtes Anhydrid durch Behandeln einer Säure H-$R_3$ mit entsprechend geschützten funktionellen Gruppen, oder eines geeigneten Salzes davon, wie eines Ammoniumsalzes, z.B. mit einem organischen Amin, wie Pyridin oder 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, mit einem Halbester eines Kohlensäurehalbhalogenids, z.B. Chlorameisensäureäthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Zur Einführung einer Carbamoylgruppe wird die Verbindung der Formel If mit einem geeigneten Kohlensäurederivat, insbesondere mit einer Isocyanat- oder Carbaminsäureverbindung, wie einem Silylisocyanat, z.B. Silyltetraisocyanat, einem Sulfonylisocyanat, z.B. Chlorsulfonylisocyanat, mit einem Carbaminsäurehalogenid, z.B. -chlorid (die zu N-unsubstituierten 3-Aminocarbonyloxymethyl-Verbindungen führen), oder dann mit einer N-substituierten Isocyanat- oder mit einer N-mono- oder N,N-disubstituierten Carbaminsäure-Verbindung, wie einem entsprechenden Carbaminsäurehalogenid, z.B. -chlorid, umgesetzt, wobei man üblicherweise in Gegenwart eines Lösungs- oder Verdünnungsmittels und, wenn notwendig, unter Kühlen oder Erwärmen, in einem geschlossenen Gefäss und/oder in einer Inertgas-, z.B. Stickstoffatmosphäre, arbeitet.

BAD ORIGINAL

Verfahren j) (Ueberführung der geschützten in die freie Carboxylgruppe $R_4$): In einer Ausgangsverbindung der Formel Ij wird die geschützte Carboxylgruppe $R_4$ in an sich bekannter Weise, wie mittels Solvolyse, insbesonder Hydrolyse, Alkoholye oder Acidolyse, oder mittels Reduktion, insbesondere Hydrogenolyse oder chemische Reduktion, gegebenenfalls stufenweise und gegebenenfalls gleichzeitig mit anderen geschützen funktionellen Gruppen, freigesetzt.

So kann man z.B. eine gegebenenfalls in 2-Stellung durch eine Silylgruppe oder in 1-Stellung durch Niederalkoxy oder Niederalkylthio substituierte Niederalkoxycarbonylgruppe, eine Polycycloalkoxycarbonyl- oder eine Diphenylmethoxycarbonylgruppe durch Behandeln mit einem geeigneten sauren Mittel, wie Ameisensäure oder Trifluoressigsäure, gegebenenfalls unter Zugabe einer nucleophilen Verbindung, wie Phenol oder Anisol, in die freie Carboxylgruppe überführen. Eine gegebenenfalls substituierte Benzyloxycarbonylgruppe kann z.B. mittels Hydrogenolyse durch Behandeln mit Wasserstoff in Gegenwart eines Hydrierkatalysators, wie eines Palladiumkatalysators freigesetzt werden. Ferner kann man bestimmt substituierte Benzylcarbonylgruppen, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoffabgebenden Mittels, das zusammen mit dem Metall nascierenden Wasserstoff zu erzeugen vermag, wie einer Säure, in erster Linie einer Carbonsäure oder α-Hydroxycarbonsäure, wie insbesondere Essigsäure, Ameisensäure, Glycolsäure, Diphenylglycolsäure, Milchsäure, Mandelsäure, p-Chlormandelsäure, Weinsäure, und dergleichen, oder eines Alkohols oder Thiols, wobei man vor-

BAD ORIGINAL

zusweise Wasser zugibt, in die freie Carboxylgruppe überführen. Durch Behandeln mit einem reduzierenden Metall oder Metallsalz, wie oben beschrieben, kann man auch eine 2-Halogen-niederalkoxycarbonylgruppe (gegebenenfalls nach Umwandlung einer 2-Brom-niederalkoxycarbonylgruppe in eine 2-Iodniederalkoxycarbonylgruppe) oder eine Aroylmethoxycarbonylgruppe in die freie Carboxylgruppe umwandeln, wobei eine Aroylmethoxycarbonylgruppe ebenfalls durch Behandeln mit einem nucleophilen, vorzugsweise salzbildenden Reagens, wie Natriumthiophenolat oder Natriumjodid gespalten werden kann. Eine substituierte Silyläthoxycarbonylgruppe kann auch durch Behandeln mit einem Salze der Fluorwasserstoffsäure, das Fluoridanion liefert, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetraalkylammoniumfluorid oder Trialkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in die freie Carboxylgruppe übergeführt werden. Eine Polyhalogenaryloxycarbonylgruppe, wie die Pentachlorphenyloxycarbonylgruppe, wird unter milden basischen Bedingungen, wie durch verdünnte Natronlauge oder organische Basen in Gegenwart von Wasser, zur freien Carboxylgruppe verseift.

Eine z.B. durch Silylierung oder Stannylierung geschützte Carboxylgruppe kann in üblicher Weise, z.B. durch Behandeln mit Wasser oder einem Alkohol, freigesetzt werden.

BAD ORIGINAL

Verfahren k) (Veresterung der 4-Carboxylgruppe): Die Ueberführung der freien Carboxylgruppe in einer Verbindung der Formel Ik in eine veresterte Carboxylgruppe, insbesondere in eine solche, die unter physiologischen Bedingungen spaltbar ist, erfolgt nach an sich bekannten Veresterungsmethoden, beispielsweise indem man eine Verbindung der Formel Ik, worin andere funktionelle Gruppen, wie Amino-, Hydroxy- oder Sulfogruppen, gegebenenfalls in geschützter Form vorliegen, oder ein bezüglich der zu veresternden Carboxylgruppe reaktionsfähiges funktionelles Derivat davon, oder ein Salz davon, mit einem entsprechenden Alkohol oder einem reaktionsfähigen funktionellen Derivat davon, verestert.

Geeignete reaktionsfähige funktionelle Derivate der zu veresternden Carboxylgruppe sind Anhydride, insbesondere gemischte Anhydride, und aktivierte Ester.

Die Veresterung der freien Carboxylgruppe mit dem gewünschten Alkohol wird in Gegenwart eines Kondensationsmittels durchgeführt.

Uebliche Kondensationsmittel sind Carbodiimide, beispielsweise N,N'-Diäthyl, N,N'-Dipropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazolinium-3'-sulfonat und N-tert.-Butyl-5-methyl-isoxazoliniumperchlorat, oder eine Acylaminoverbindung, z.B. 2-Aethoxy-1-äthoxycarbonyl-1,2-dihydrochinolin.

Die Kondensationsreaktion wird vorzugsweise in einem wasserfreine Reaktionsmedium, vorzugsweise in Gegenwart eines Lösungs- oder Verdünnungsmittels, z.B. Methy-

BAD ORIGINAL

lenchlorid, Dimethylformaid, Acetonitril oder Tetrahydrofuran, wenn erwünscht oder notwendig, unter Kühlen oder Erwärmen und/oder in einer Inertgasatmosphäre, durchgeführt.

Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure, Diäthylphosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäure, z.B. p-Toluolsulfonsäure.

Zur Reaktion mit dem Alkohol geeignete aktivierte Ester sind z.B. Ester mit vinylogen Alkoholen (d.h. Enolen), wie vinylogen Niederalkenolen, oder Iminiomethylesterhalogeniden, wie Dimethyliminiomethylesterchlorid (hergestellt aus der Carbonsäure und Dimethyl-chlormethyliden-iminium-chlorid der Formel $[(CH_3)_2N=CHCl]^{\oplus} Cl^{\ominus}$), oder Arylester, wie 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem Säurederivat, wie einem Anhydrid, insbesondere mit einem Säurehalogenid wird

BAD ORIGINAL

bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer organischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig, bei erniedrigter oder erhöhter Temperatur, etwa bei -40° bis etwa +100°, bevorzugt bei -10° bis +40°, und/oder in einer Inertgas-, z.B. Stickstoffatmosphäre.

Ferner kann das Säurederivat, wenn erwünscht, in situ gebildet werden. So erhält man z.B. ein gemischtes Anhydrid durch Behandeln der Carbonsäure mit entsprechend geschützten funktionellen Gruppen, oder eines geeigneten Salzes davon, wie eines Ammoniumsalzes, z.B. mit einem organischem Amin, wie Pyriolin oder 4-Methylmorpholin,

BAD ORIGINAL

oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloroacetylchlorid, mit einem Halbester eines Kohlensäurehalbhalogenids, z.B. Chlorameisensäurehalbester oder -isobutylester, oder mit einem Halogenid einer Diniederalkylphosphorsäure, z.B. Diäthylphosphorbromidat, und verwendet das so erhältliche gemischte Anhydrid ohne Isolierung.

Verfahren 1) (Austausch von Y gegen Amine AmH): In einer Verbindung der Formel XVIII ist Y Brom, Jod oder bevorzugt Chlor.

Die Amine Am-H werden in freier Form, d.h. als Ammoniak, Niederalkylamin, wie Methyl-, Aethyl-, Propyl- oder Butylamin, oder Diniederalkylamin, wie Dimethyl-, Diäthyl-, Dipropyl-, Dibutyl-, Methyl-äthyl-, Aethyl-propyl-, Methylbutyl-, Propyl-butylamin, oder dergleichen, oder in Form von Metallamiden davon, beispielsweise als Alkalimetallamide, wie Lithium-, Natrium- oder Kaliumamide, eingesetzt.

Die Austauschreaktion findet bevorzugt in einem inerten organischen Lösungsmittel, wie Benzol, Toluol, Dimethylformamid, Tetrahydrofuran, oder dergleichen, oder, beim Austausch von Y gegen $NH_2$ auch in flüssigem Ammoniak statt, wobei gegebenenfalls die Säure H-Y abfangende Basen, wie tertiäre Amine, z.B. Aethyl-diisopropylamin, Pyridin, oder dergleichen zugefügt werden können.

Die Reaktion erfolgt, je nach Reaktionsfähigkeit, bei Zimmertemperatur oder unter Abkühlen oder Erwärmen, zwischen etwa -70° und etwa +100°, bevorzugt zwischen etwa -70° und etwa +50°.

BAD ORIGINAL

Abspaltung von Schutzgruppen: In einer erhaltenen Verbindung der Formel I, worin eine oder mehrere funktionelle Gruppen geschützt sind, können diese, z.B. geschützte Carboxyl-, Amino-, Hydroxy-, Mercapto- und/oder Sulfogruppen, in an sich bekannter Weise, wie mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

Die Ueberführung einer geschützten in eine freie Carboxylgruppe kann gemäss dem Verfahren j) erfolgen.

Eine geschützte Aminogruppe, z.B. Am, wird in an sich bekannter und je nach Art der Schutzgruppen in verschiedenartiger Weise, z.B. mittels Solvolyse oder Reduktion, freigesetzt. Eine 2-Halogen-niederalkoxycarbonylaminogruppe (gegebenenfalls nach Umwandlung einer 2-Bromniederalkoxycarbonylgruppe in eine 2-Jod-niederalkoxycarbonylgruppe), eine Acylmethoxycarbonylaminogruppe oder eine 4-Nitrobenzyloxycarbonylaminogruppe kann z.B. durch Behandeln mit einem geeigneten chemischen Reduktionsmittel, wie Zink in Gegenwart einer Carbon- oder α-Hydroxycarbonsäure, wie wässriger Essigsäure, eine Aroylmethoxycarbonylaminogruppe auch durch Behandeln mit einem nucleophilen, vorzugsweise salzbildenden Reagens, wie Natriumthiophenolat, und eine 4-Nitro-benzyloxycarbonylaminogruppe auch durch Behandeln mit einem Alkalimetall-, z.B. Natriumdithionit, eine Diphenylmethoxycarbonylamino-, tert.-Niederalkoxycarbonylamino-, 2-$(S_1)(S_2)(S_3)$-Silyläthoxycarbonylamino- oder Polycycloalkoxycarbonylaminogruppe durch Behandeln z.B. mit Ameisen- oder Trifluoressigsäure, eine gegebenenfalls substituierte Benzyloxycarbonylaminogruppe z.B.

BAD ORIGINAL

mittels Hydrogenolyse durch Behandeln mit Wasserstoff in Gegenwart eines Hydrierkatalysators, wie eines Palladiumkatalysators, eine Triarylmethylgruppe z.B. durch Behandelm mit wässriger Mineralsäure, und eine mit einer organischen Silyl- oder Stannylgruppe geschützte Aminogruppe z.B. mittels Hydrolyse oder Alkoholyse freigesetzt werden. Eine durch 2-Halogenacetyl, wie 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs und anschliessende Solvolyse, wie Alkoholyse oder Hydrolyse, des entstandenen Kondensationsproduktes freigesetzt werden. Eine durch eine substituierte-Silyläthoxycarbonylgruppe geschützte Aminogruppe kann auch durch Behandeln mit einem Salz der Fluorwasserstoffsäure, das Fluoridanionen liefert, wie oben bei der Freisetzung einer entsprechend geschützten Carboxylgruppe angegeben, in die freie Aminogruppe übergeführt werden. Eine Phosphor-, Phosphon- oder Phosphinamidogruppe kann durch Behandeln mit einer Phosphor-haltigen Säure, wie einer Phosphor-Phosphon- oder Phosphinsäure, z.B. Orthophosphorsäure oder Polyphosphorsäure, einem sauren Ester davon, z.B. Monomethyl-, Monoäthyl-, Dimethyl- oder Diäthylphosphat oder Monomethylphosphonsäure, oder einem Anhydrid davon, wie Phosphorpentoxid, und dergleichen, in die freie Aminogruppe übergeführt werden.

Eine in Form einer Azidogruppe geschützte Aminogruppe wird in an sich bekannter Weise durch Reduktion, in die freie Aminogruppe übergeführt, beispielsweise durch katalytische Hydrierung mit Wasserstoff und einem Hydrierkatalysator, wie Platinoxid, Palladium, oder auch Raney-Nickel, oder auch durch Zink und Säure, wie Essigsäure.

BAD ORIGINAL

Die katalytische Hydrierung wird bevorzugt in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20 bis 25°, oder auch bei erniedrigter oder erhöhter Temperatur, durchgeführt.

Eine durch eine Acylgruppe, eine Silyl- oder Stannylgruppe oder durch einen gegebenenfalls substituierten α-Phenylniederalkylrest geschützte Hydroxygruppe wird wie eine entsprechend geschützte Aminogruppe freigesetzt. Eine durch 2,2-Dichloracetyl geschützte Hydroxygruppe wird durch basische Hydrolyse und eine durch einen 2-oxa- oder 2-thia-aliphatischen oder -cycloaliphatischen Kohlenwasserstoffrest geschützte Hydroxygruppe wird durch Acidolyse freigesetzt.

Eine geschützte Sulfogruppe wird analog einer geschützten Carboxylgruppe freigesetzt.

Bevorzugt werden die Schutzgruppen so gewählt, dass sie alle gleichzeitig abgespalten werden können, beispielsweise acidolytisch, wie durch Behandeln mit Trifluoressigsäure oder Ameisensäure, oder reduktiv, wie durch Behandeln mit Zink und Eisessig, oder mit Wasserstoff und einem Hydrierkatalysator, wie einem Palladium/Kohle-Katalysator.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

BAD ORIGINAL

Salzbildung: Salze von Verbindungen der Formel I mit salzbildenden Gruppen können in an sich bekannter Weise hergestellt werden. So kann man Salze von Verbindungen der Formel I mit sauren Gruppen, z.B. durch Behandeln mit Metallverbindungen, wie Alkalimetallsalzen von geeigneten Carbonsäuren, z.B. dem Natriumsalz oder α-Aethylcapronsäure oder Natriumbicarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

BAD ORIGINAL

Herstellung der Ausgangsverbindungen:

Ausgangsverbindungen der Formel II sowie entsprechende Verbindungen mit geschützten funktionellen Gruppen sind bekannt oder können auf an sich bekannte Weise hergestellt werden.

Ausgangsverbindungen der Formel III und Verfahren zur ihrer Herstellung sind bisher nicht bekannt geworden. Diese Verbindungen können gemäss folgendem Reaktionsschema hergestellt werden:

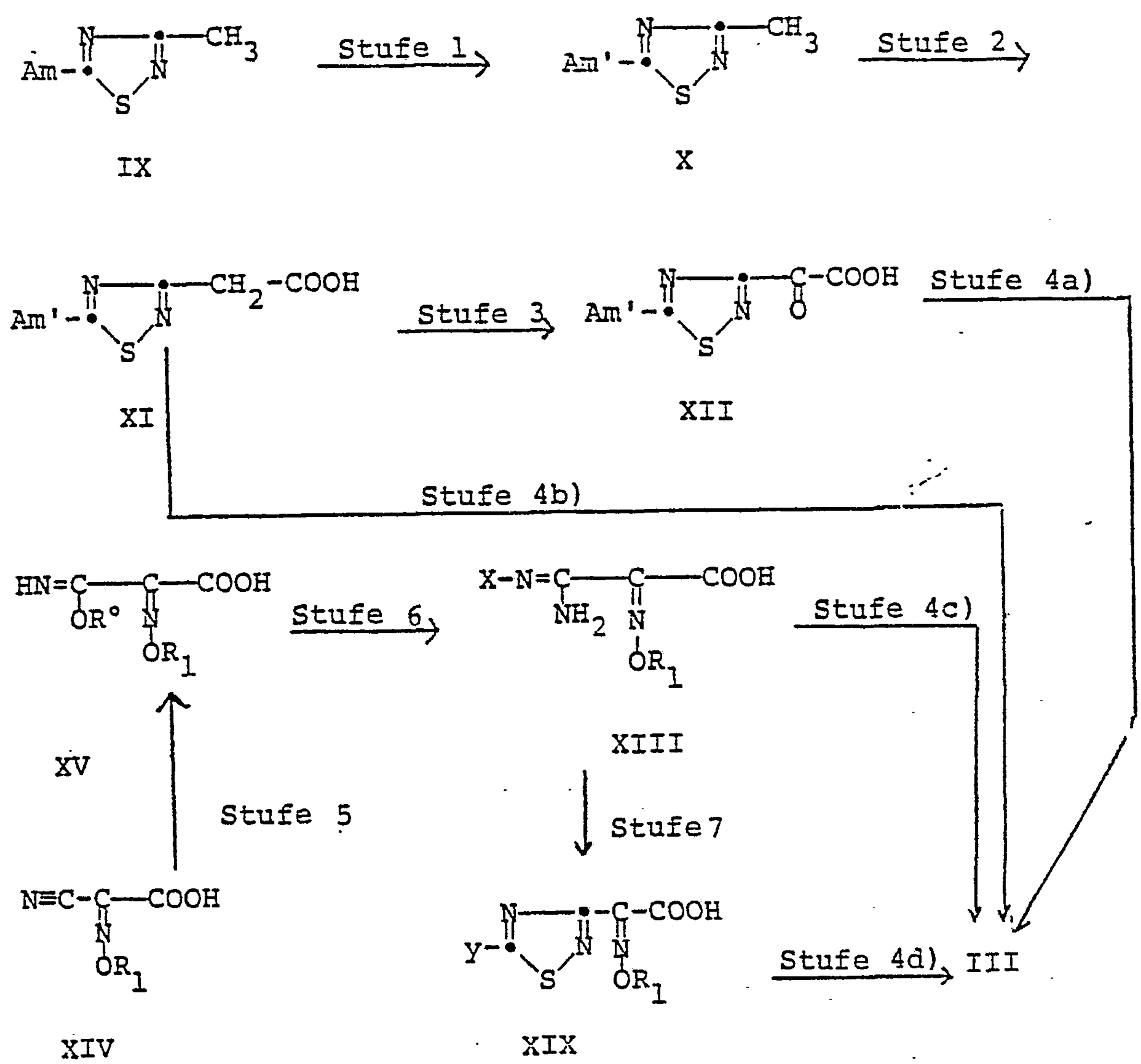


IX X XI XII XV XIII XIV XIX III

BAD ORIGINAL

Stufe 1: Verbindungen der Formel IX sind bekannt oder können auf an sich bekannte Weise hergestellt werden.

In den bekannten Verbindungen der Formel IX wird eine primäre oder sekundäre Aminogruppe nach einem der üblichen Verfahren, z.B. Acylierung oder Silylierung und dergleichen, oder Alkylierung, in eine geschützte oder tertiäre Aminogruppe Am' übergeführt. Eine geschützte Aminogruppe Am' ist eine der unter Am aufgeführten geschützten Aminogruppen, insbesondere eine der genannten Acylaminogruppen, wie insbesondere tert.Butoxycarbonylamino oder N-Methyl-tert.butoxycarbonylamino. Eine tertiäre Aminogruppe Am' ist z.B. eine der genannten Diniederalkylaminogruppen. Bei der Alkylierung kann die Alkylgruppe gegebenenfalls zunächst an das Ringstickstoffatom in 4-Stellung gehen, worauf durch Erhitzen auf etwa 100° eine Umalkylierung in die exo-Aminogruppe auftritt. Eine solche Alkylierung und Umlagerung tritt beispielsweise auch beim Methylieren von 5-Amino-3-methyl-1,2,4-thiadiazol auf.

Stufe 2: Eine Verbindung der Formel XI, gegebenenfalls in veresterter Form, wird erhalten, indem man eine Verbindung der Formel X carboxyliert. Die Carboxylierung erfolgt auf an sich bekannte Weise, indem man eine Verbindung der Formel X metalliert und mit Kohlendioxid behandelt. Als Metallierungsmittel dienen insbesondere Organolithiumverbindungen, wie Niederalkyllithium, z.B. Methyl-, Aethyl- oder Butyllithium, Aryllithium, z.B. Phenyllithium, oder Lithiumamide, wie Lithiumdiniederalkylamide, z.B. Lithiumdiisopropylamid, und dergleichen. Die Metallierung wird in einem inerten Lösungsmittel, wie einem Aether, z.B. Diäthyläther, Dioxan oder Tetrahydrofuran, oder einem Kohlenwasserstoff, z.B. Hexan, Benzol, oder einem tertiären Amin oder Diamin, z.B. Tetramethyläthylendiamin, und dergleichen, bei erniedrigten Tem-

BAD ORIGINAL

peraturen, etwa bei 0° bis -80°, durchgeführt. Anschliessend wird bei der gleichen Temperatur Kohlendioxid eingeleitet oder das Metallierungsgemisch wird auf Trockeneis gegossen. Wenn man statt Kohlendioxid einen Halogenameisensäureester benutzt, erhält man einen Ester einer Säure der Formel XI.

Einen Ester kann man auch erhalten, gegebenenfalls nur als Nebenprodukt, wenn man Verbindungen der Formel X carboxyliert, worin Am' eine durch verestertes Carboxyl geschützte sekundäre Aminogruppe ist. Dabei kann die Acylschutzgruppe von der Aminogruppe auf das Methylcarbanion in 3-Stellung wandern, so dass nach Aufarbeitung eine Verbindung der Formel XI resultiert, worin Am' eine freie sekundäre Aminogruppe ist und worin die Carboxylgruppe in veresterter Form vorliegt. Beispielsweise ergibt 5-(N-Methyl-N-tert.butoxycarbonylamino)-3-methyl-1,2,4-thiadiazol bei der Behandlung mit Lithiumdiisopropylamid den 2-(5-Methylamino-1,2,4-thiadiazol-2-yl)essigsäure-tert.butylester. Die Aufarbeitung der Reaktionsmischung erfolgt durch Behandeln mit Wasser und Säure oder Base und Extraktion auf übliche Weise.

Stufe 3: Eine α-Ketoverbindung der Formel XII oder ein Ester davon wird erhalten, indem man eine Verbindung der Formel XI oder einen Ester davon mit einem geeigneten Oxidationsmittel behandelt. Zahlreiche solcher Oxidationsmittel sind bekannt. Bevorzugt wird Selendioxid benutzt. Die Reaktion wird in einem inerten Lösungsmittel, wie einem Aether, z.B. Dioxan oder Tetrahydrofuran, einem Amid, z.B. Dimethylformamid, oder einem tertiären Amin, z.B. Pyridin, oder dergleichen, bei erniedrigter oder erhöhter Temperatur, etwa bei 0° bis etwa 100°, bevorzugt bei Zimmertemperatur bis zu etwa 80° durchgeführt.

Stufe 4a: Eine Verbindung der Formel III oder ein Ester davon, worin Am eine geschützte oder tertiäre Aminogruppe Am' bedeutet, wird erhalten, indem man eine Verbindung der Formel XII

BAD ORIGINAL

oder einen Ester davon mit einem Hydroxylamin der Formel $H_2N-O-R_1$ (V) behandelt. Die Reaktion kann wie unter Verfahren b) beschrieben durchgeführt werden.

Stufe 4b): Eine Verbindung der Formel III oder ein Ester davon, worin Am eine geschützte oder tertiäre Aminogruppe Am' bedeutet, kann auch erhalten werden, indem man eine Verbindung der Formel XI oder einen Ester davon nitrosiert und gewünschtenfalls eine erhaltene Verbindung der Formel III, worin $R_1$ Wasserstoff bedeutet, in eine Verbindung der Formel III überführt, worin $R_1$ für gegebenenfalls substituiertes Niederalkyl steht. Die Nitrosierung kann wie unter Verfahren e) und die Alkylierung wie unter Verfahren f) beschrieben durchgeführt werden.

Stufe 4c): Eine Verbindung der Formel III oder ein Ester davon, worin Am eine freie primäre oder sekundäre Aminogruppe bedeutet, kann auch erhalten werden, indem man eine Verbindung der Formel XIII oder einen Ester davon, worin X Halogen oder Hydroxy bedeutet und $R_1$ die oben angegebene Bedeutung hat, mit einem Salz der Rhodanwasserstoffsäure, bzw. einem Isorhodanwasserstoffsäure-ester oder eine Verbindung der Formel XIII, worin X Wasserstoff ist, mit Rhodan behandelt. Die Ringschlussreaktion mit dem Salz der Rhodanwasserstoffsäure oder mit Rhodan wird wie unter Verfahren d) beschrieben durchgeführt.

Stufe 4d): Eine Verbindung der Formel III oder ein Ester davon, worin Am eine primäre, sekundäre oder tertiäre Aminogruppe bedeutet, kann auch erhalten werden, indem man eine Verbindung der Formel XIX oder einen Ester davon, worin Y Halogen ist und $R_1$ die oben angegebene Bedeutung hat, mit Ammoniak oder einem primären oder sekundären Amin Am-H, oder einem Metallamid davon, umsetzt. Der Austausch von Halogen Y, z.B. von Chlor, gegen die Aminogruppe Am erfolgt wie

BAD ORIGINAL

unter Verfahren 1) beschrieben.

In einer Verbindung der Formel III kann eine geschützte Aminogruppe Am' in eine freie Aminogruppe oder in eine andersartig geschützte Aminogruppe übergeführt werden, oder falls $R_1$ Wasserstoff ist, kann die Gruppe =N-OH analog Verfahren f) veräthert oder carbamoyliert werden. Erhaltene Ester von Verbindungen der Formel III werden auf an sich bekannte Weise in die freien Carbonsäuren übergeführt.

Reaktionsfähige Derivate von Verbindungen der Formel III, die zur Acylierung geeignet sind, werden auf an sich bekannte Weise, gegebenenfalls in situ, hergestellt.

Verbindungen der Formel XIII werden ausgehend von Verbindungen der Formel XIV über Verbindungen der Formel XV gemäss den Stufen 6 und 7 erhalten.

Stufe 5: Verbindungen der Formel XV, worin R° Niederalkyl ist, und $R_1$ die oben angegebene Bedeutung hat, und worin die Carboxylgruppe gegebenenfalls geschützt ist, können erhalten werden, indem man an eine Verbindung der Formel XIV, worin $R_1$ die oben angegebene Bedeutung hat und die funktionellen Gruppen gegebenenfalls geschützt sind, den Alkohol HOR° anlagert. Die Anlagerung des Alkohols an das Nitril erfolgt auf an sich bekannte Weise in Gegenwart einer Säure, z.B. einer Halogenwasserstoffsäure, wie Salzsäure, bei erniedrigter oder erhöhter Temperatur, bevorzugt zwischen etwa -30° bis +40°, insbesondere um etwa 0°. Die Reaktion wird in irgendeinem inerten Lösungsmittel durchgeführt, bevorzugt in dem Alkohol HOR° selbst. Man erhält das Säureadditionssalz des Iminoesters der Formel XV oder eines geschützten Derivates davon.

Stufe 6: Eine Verbindung der Formel XV, worin R° Niederalkyl bedeutet und $R_1$ die oben angegebene Bedeutung hat, ein Deri-

BAD ORIGINAL

vat davon, worin funktionelle Gruppen geschützt sind oder ein Säureadditionssalz davon, wird durch Behandeln mit Ammoniak in eine Verbindung der Formel XIII übergeführt, worin X Wasserstoff ist und worin funktionelle Gruppen gegebenenfalls geschützt sind. Statt Ammoniak kann auch Ammoniumhydroxid oder Ammoniumchlorid eingesetzt werden. Die Umsetzung mit Ammoniak erfolgt in einem wässrigen oder nicht wässrigen Lösungsmittel bei Temperaturen zwischen etwa -30° bis etwa +40°. In einer Verbindung der Formel XIII, worin X Wasserstoff ist, kann dieser durch Halogen substituiert werden, wie unter Verfahren d) beschrieben ist.

Stufe 7: Verbindungen der Formel XIX, worin Y Halogen darstellt und $R_1$ die oben gegebene Bedeutung hat, Ester oder Salze davon werden erhalten, indem man eine Verbindung der Formel XIII, worin X Wasserstoff ist, oder einen Ester oder ein Salz davon, mit einem Perhalogenmethylmercaptan der Formel $Y_3C-S-Y$, worin Y die obige Bedeutung hat, umsetzt.

Im Ausgangsmaterial und im Produkt der Formel XIX ist Y bevorzugt Chlor. Die Reaktion wird in Gegenwart einer den entstehenden Halogenwasserstoff H-Y neutralisierenden Base in einem organischen oder organisch-wässrigen Lösungsmittel oder Lösungsmittelgemisch durchgeführt. Als Basen können organische Basen, wie tertiäre Amine, z.B. Triniederalkylamine, wie Trimethylamin, Aethyldiisopropylamin, oder aromatische Amine, wie Pyridin oder anorganische Basen, z.B. Alkalimetallhydroxide, wie Lithium-, Natrium- oder Kaliumhydroxid, oder dergleichen, bevorzugt in stöchiometrischen Mengen, eingesetzt werden. Als Lösungsmittel dienen Wasser, organische Lösungsmittel, wie Alkohol, z.B. Methanol oder Aethanol, Halogenkohlenwasserstoffe, wie Methylenchlorid oder Chloroform, Amide, wie Dimethylformamid, Sulfoxide, wie Dimethylsulfoxid, Aether, wie Dioxan, Tetrahydrofuran, oder dergleichen,

BAD ORIGINAL

oder gegebenenfalls Mischungen oder wässrige Emulsionen davon, denen zur besseren Emulgierung gegebenenfalls ein Emulgator, wie Natriumdodecylsulfat, zugefügt werden kann. Die Reaktion wird bei Zimmertemperatur, unter Abkühlen oder leichtem Erwärmen, je nach Reaktionsfähigkeit, d.h. bei etwa -15° bis etwa + 50°, bevorzugt bei etwa -10° bis etwa +20°, durchgeführt.

Verbindungen der Formel IV werden durch Acylierung von Verbindungen der Formel II mit Verbindungen der Formel XII oder einem reaktionsfähigen funktionellen Derivat davon erhalten.

Verbindungen der Formel VI können als Nebenprodukte bei der Herstellung von Verbindungen der Formel I unter basischen Bedingungen erhalten werden. Sie müssen nicht in reiner Form vorliegen, sondern können im Gemisch mit Verbindungen der Formel I eingesetzt werden.

Verbindungen der Formel VII werden durch Acylierung von Verbindungen der Formel II mit Verbindungen der Formel XIII, oder einem reaktionsfähigen funktionellen Derivat davon, erhalten, oder analog den Stufen 6 und 7, ausgehend von Verbindungen der Formel

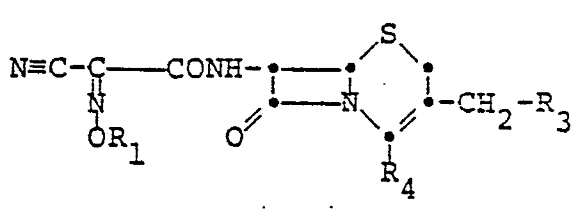

(XVI)

über Verbindungen der Formel

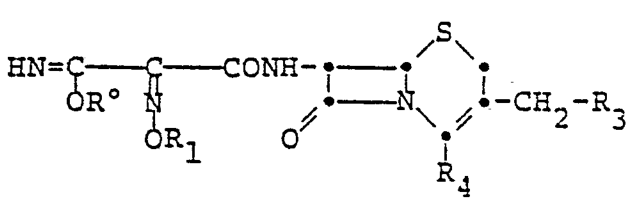

(XVII)

BAD ORIGINAL

die ihrerseits auch durch Acylierung von Verbindungen der Formel II mit Verbindungen der Formel XIV bzw. XV, oder reaktionsfähigen funktionellen Derivaten davon, erhalten werden können.

Verbindungen der Formel VIII können aus Verbindungen der Formel II durch Acylierung mit Verbindungen der Formel XI, oder reaktionsfähigen funktionellen Derivaten davon, gemäss Verfahren a) erhalten werden, gegebenenfalls können die Gruppen Am, $R_2$, $R_3$ und $R_4$ in andere Gruppen Am, $R_2$, $R_3$ oder $R_4$ übergeführt werden, wobei Verfahren angewendet werden können, wie sie für entsprechende Reaktionen zur Herstellung von Verbindungen der Formel I angegeben sind. Verbindungen der Formel VIII, worin $R_4$ Carboxy ist und ihre pharmazeutisch verwendbaren Salze und ihre physiologisch spaltbaren Ester haben ähnliche antibiotische Eigenschaften wie die aktiven Verbindungen der Formel I. Diese Verbindungen und die Verfahren zu ihrer Herstellung sind ebenfalls Gegenstand der vorliegenden Erfindung.

Verbindungen der Formeln (If) bis (Ik) fallen unter die allgemeine Formel I und können gemäss einem der Verfahren a) bis e) erhalten werden. Auch bei diesen Verbindungen können vorhandene Gruppen Am, $R_1$, $R_2$, $R_3$ oder $R_4$ in andere Gruppen Am, $R_1$, $R_2$, $R_3$ oder $R_4$ übergeführt werden, wobei sinngemäss Verfahren angewendet werden können, wie sie für entsprechende Reaktionen zur Herstellung von Verbindungen der Formel I angegeben sind.

Verbindungen der Formel XVIII können auf verschiedene Weise hergestellt werden, beispielsweise analog dem Verfahren a), c), f), i), j) oder k) oder der Stufe 7, insbesondere indem man, analog Verfahren a), in einer Verbindung der Formel II, worin $R_2$, $R_3$ und $R_4$ die angegebenen Bedeutungen haben, und worin die 7β-Aminogruppe gegebenenfalls durch eine die Acylierung erlaubende Gruppe substituiert ist, die 7β-Aminogruppe durch Behandeln mit einem den Acylrest einer Carbon-

BAD ORIGINAL

säure der Formel XIX einführenden Acylierungsmittel acyliert, oder indem man, analog Stufe 9, eine Verbindung der Formel VII, worin X Wasserstoff bedeutet und $R_1$, $R_2$, $R_3$ und $R_4$ die angegebenen Bedeutungen haben, oder ein Salz davon, mit einem Perhalogenmethylmercaptan der Formel $Y_3C-S-Y$ umsetzt, und gewünschtenfalls die bei der Herstellung der Verbindungen der Formel I genannten nachträglichen Operationen durchführt.

Die neuen Zwischenprodukte und Ausgangsverbindungen und Verfahren zur ihrer Herstellung sind ebenfalls Gegenstand der vorliegenden Erfindung.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur äusseren, rektalen, oralen oder vorzugsweise zur parenteralen Verabreichung eignen. Geeignete Formen solcher Präparate sind Salben, Suppositorien, Tabletten Dragees, Kapseln oder Ampullen.

Vorzugsweise verwendet man die pharmakologisch wirksamen Verbindungen der vorliegenden Erfindung in Form von injizierbaren, z.B. intramuskulär oder intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfstoffe, z.B. Konservier-, Stabilisier-, Netz-und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B.

BAD ORIGINAL

mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes. Je nach Art der Infektion und Zustand des infizierten Organismus verwendet man tägliche Dosen von etwa 0,5 g bis etwa 5 g s.c. zur Behandlung von Warmblütern von etwa 70 kg Gewicht.

Die folgenden Beispiele dienen zur Illustration der Erfindung; Temperaturen werden in Celsiusgraden angegeben.

In den Beispielen werden folgende Abkürzungen verwendet:

BOC: tert.-Butyloxycarbonyl
F.: Schmelzpunkt

Dünnschichtchromatographie: Opti-UPC$_{12}$-Platten der Fa. Antec sind mit Silicagel beschichtete Glasplatten, wobei das Silicagel mit Dodecyltrichlorsilan behandelt wurde (zur Chromatographie mit umgekehrter Phase).

Herstellung der Ausgangsverbindungen

Beispiel a): 5-tert.Butoxycarbonylamino-3-methyl-1,2,4-thiadiazol.

Eine Suspension von 20,0 g (0,174 Mol) 5-Amino-3-methyl-1,2,4-thiadiazol (DBP 955.684) in 100 ml BOC-Anhydrid wird bei 100° (Badtemperatur) 12 Stunden gerührt. Das Reaktionsgemisch wird abgekühlt, die flüchtigen Anteile werden im Vakuum entfernt und der teilweise kristalline Rückstand wird durch Umkristallisieren aus Isopropylalkohol gereinigt. Man erhält so 18,2 g der Titelverbindung vom Rf-Wert 0,65 (Silicagel, Essigester). UV-Spektrum (Aethanol): $\lambda$ max ($\varepsilon$) 222 (4000); 245 (8250) nm. IR-Spektrum

BAD ORIGINAL

(Nujol): charakteristische Absorptionsbanden bei 5,90; 6,45; 8,75 µ; F.: 136-7°.

Beispiel b): 2-(5-tert.Butoxycarbonylamino-1,2,4-thiadiazol-3-yl)essigsäure.

Eine frisch zubereitete Lösung von Lithiumdiisopropylamid in 100 ml abs. Tetrahydrofuran [aus 26 ml (0,184 Mol) Diisopropylamin und 104 ml (0,208 Mol) einer ca. 2 N Lösung von Butyllithium in Hexan] wird bei -78° unter Stickstoff mit 10,0 g (0,046 Mol) 5-tert.Butoxycarbonylamino-3-methyl-1,2,4-thiadiazol in 50 ml abs. Tetrahydrofuran versetzt und 30 Min. bei dieser Temperatur gerührt. Nach Erwärmen des Reaktionsgemisches auf -30° wird während 30 Min. $CO_2$ eingeleitet. Die Lösung wird mit 100 ml Wasser versetzt und mit 50 ml Diäthyläther verdünnt. Die organische Phase wird abgetrennt, die wässrige Phase mit 2 N Salzsäure auf pH 2,0 gestellt und mit Aethylacetat extrahiert. Die organische Phase wird abgetrennt, mit wässriger Natriumchloridlösung gewaschen, über Magnesiumsulfat getrocknet und eingeengt. Der Rückstand wird aus Diäthyläther gefällt. Man erhält die Titelverbindung vom Rf-Wert 0,40 (Silicagel; Chloroform:Methanol:Eisessig 85:12:3); F.: 163-5°; UV-Spektrum (Methanol): $\lambda_{max}$ ($\varepsilon$) 215(5250); 245(8250) nm; IR-Spektrum (Nujol): charakteristische Absorptionsbanden bei 2,85; 2,90; 3,15; 6,45 µ.

BAD ORIGINAL

Beispiel c): 2-(5-tert.Butoxycarbonylamino-1,2,4-thiadiazol-3-yl)-2-oxoessigsäure.

Eine Suspension von 5,0 g (19,25 m Mol) 2-(5-tert.-Butoxycarbonylamino-1,2,4-thiadiazol-3-yl)essigsäure und 4,2 g Selendioxid in 100 ml abs. Dioxan wird 3 Std. bei 80° gerührt. Nach Filtrieren des Reaktionsgemisches wird das Filtrat im Vakuum eingeengt. Der Rückstand wird mit 150 ml Aethylacetat verdünnt und mit wässriger Natriumbicarbonatlösung extrahiert. Die Aethylacetat-Phase wird abgetrennt, mit wässriger Natriumchloridlösung gewaschen, über Magnesiumsulfat getrocknet und eingeengt. Man erhält einheitliches 5-tert.Butoxycarbonylamino-3-formyl-1,2,4-thiadiazol vom Rf-Wert 0,65 (Silicagel, Chloroform:Methanol:Eisessig 85:12:3) IR-Spektrum ($CH_2Cl_2$): charakteristische Absorptionsbanden bei 2,90; 5,80: 6,15; 6,50; 8,70 µ. Die wässrige Natriumbicarbonatphase wird mit 2 N Salzsäure auf pH 1,0 gestellt und mit Aethylacetat extrahiert. Die organische Phase wird abgetrennt, mit wässriger Natriumchloridlösung gewaschen, über Magnesiumsulfat getrocknet und eingeengt. Man erhält so die einheitliche Titelverbindung in Form eines Schaumes vom Rf-Wert 0,30 (Silicagel, Chloroform:Methanol:Eisessig 85:12:3); UV-Spektrum (Methanol: $\lambda$ max ($\varepsilon$) 219(7150); 243(7900) nm; IR-Spektrum (Nujol): charakteristische Absorptionsbanden 3,10; 5,80; 6,15; 6,45; 8,60 nm.

BAD ORIGINAL

Beispiel d): 2-(5-tert.Butoxycarbonylamino-1,2,4-thiadiazol-3-yl)-2(Z)-methoxyiminoessigsäure.

Eine Suspension von 3,4 g (12,4 m Mol) 2-(5-tert.-Butoxycarbonylamino-1,2,4-thiadiazol-3-yl)-2-oxoessigsäure, 1,11 g (13,35 m Mol) Methoxyaminhydrochlorid und 1,1 ml Pyridin in 70 ml 95% Aethanol wird 3 Std. bei Raumtemperatur gerührt. Das Reaktionsgemisch wird im Vakuum eingeengt. Der Rückstand wird mit 100 ml Aethylacetat und 100 ml Wasser verdünnt, die wässrige Phase wird mit 2 N HCl auf pH 2 gestellt und anschliessend mit Aethylacetat extrahiert. Die organische Phase wird abgetrennt, mit wässriger Natriumchloridlösung gewaschen, über Magnesiumsulfat getrocknet und eingeengt. Der Rückstand besteht aus einem ca. 9:1 Z/E-Isomerengemisch. Die Titelverbindung wird als einheitliches Produkt durch fraktionierte Kristallisation aus Methylenchlorid erhalten. Rf-Wert: 0,30 (Silicagel, s-Butanol/Eisessig/Wasser 67:10:23); F.: 137-39° (Zersetzung); IR-Spektrum: charakteristische Absorptionsbanden bei 2,75; 2,90; 5,85; 6,18; 6,45; 9,60 μ; UV-Spektrum (Methanol): $\lambda$ max ($\varepsilon$): 234(22250) nm.

Beispiel e): 5-(N-Methyl-N-tert.Butoxycarbonylamino)-3-methyl-1,2,4-thiadiazol

Eine Suspension von 15 g (116,3 m Mol) 5-N-Methylamino-3-methyl-1,2,4-thiadiazol in 55 ml BOC-Anhydrid wird bei 100° (Badtemperatur) 4 Stunden gerührt. Das Reaktionsgemisch wird abgekühlt, die flüchtigen Anteile werden im Vakuum entfernt und der Rückstand wird chromatographiert (Silicagel, Toluol/steigende Anteile Aethylacetat). Man erhält so 22,3 g der Titelverbindung vom Rf-Wert 0,65 (Silicagel, Toluol/Aethylacetat 1:1). F. 47-48°. IR-Spektrum ($CH_2Cl_2$): charakteristische Absorptionsbanden bei 5,90; 6,55; 8,80 μ.

BAD ORIGINAL

Beispiel f): 2-(5-N-Methylamino-1,2,4-thiadiazol-3-yl)-essigsäure-tert.butylester

Eine frisch zubereitete Lösung von Lithiumdiisopropylamid in 30 ml abs. Tetrahydrofuran [aus 7,8 ml(55,2 m Mol) Diisopropylamin und 30 ml einer ca. 2 N Lösung von Butyllithium in Hexan] wird bei -78° unter Stickstoff mit 3,15 g (13,8 m Mol) 5-(N-Methyl-N-tert.butyoxycarbonylamino)-3-methyl-1,2,4-thiadiazol in 15 ml abs. Tetrahydrofuran versetzt und 50 Min. bei dieser Temperatur gerührt. Das Reaktionsgemisch wird unter Rühren auf festes $CO_2$ gegossen und während 30 Min. gerührt. Die Lösung wird mit 60 ml Wasser versetzt und mit Diäthyläther extrahiert. Die wässrige Phase wird mit 2N Salzsäure auf pH 2,0 eingestellt und mit Aethylacetat reextrahiert. Die vereinigten organischen Phasen werden mit wässriger Natriumchloridlösung gewaschen, über Magnesiumsulfat getrocknet und eingeengt.

Nach Chromatographie des Rückstandes an Silicagel mit Toluol/steigende Anteile Aethylacetat erhält man die einheitliche Titelverbindung vom Rf-Wert 0,50 (Silicagel, Aethylacetat) F.: 91-92°. IR-Spektrum ($CH_2Cl_2$): charakteristische Absorptionsbanden bei 2,90, 5,80; 8,65 μ.

Beispiel g): 2-(5-N-Methylamino-1,2,4-thiadiazol-3-yl)-2-oxo-essigsäure-tert.butylester

Eine Suspension von 2,3 g (10,0 m Mol) 2-(5-N-Methylamino-1,2,4-thiadiazol-3-yl)-essigsäure-tert.butylester und 3,0 g Selendioxid in 50 ml abs. Dioxan wird 3 Std. bei 90° gerührt. Nach Filtrieren des Reaktionsgemisches wird das Filtrat im Vakuum eingeengt. Der Rückstand wird mit Diäthyläther verrieben. Man erhält so die Titelverbindung vom Rf-Wert 0,55 (Silicagel, Chloroform/Methanol 9:1). IR-Spektrum ($CH_2Cl_2$): charakteristische Absorptionsbanden bei 2,90; 3,10; 5,80; 5,90; 6,25; 9,35; 9.70 μ.

BAD ORIGINAL

Beispiel h): 2-(5-N-Methylamino-1,2,4-thiadiazol-3-yl)-2-Z-methoxyimino-essigsäure-tert.butylester

Eine Suspension von 2,0 g (8,0 m Mol) 2-(5-Methylamino-1,2,4-thiadiazol-3-yl)-2-oxoessigsäure-tert.butylester, 0,736 g (8,64 m Mol) Methoxyaminhydrochlorid und 0,72 ml Pyridin in 50 ml 95% Aethanol wird 3 Std. bei Raumtemperatur gerührt, dann im Vakuum eingeengt. Der Rückstand, der aus den beiden Z- und E-Isomeren der Titelverbindung besteht, wird aus einem Gemisch von Methylenchlorid/Aethylacetat fraktioniert kristallisiert. Man erhält das einheitliche Z-Isomere vom Rf-Wert 0,75 (Silicagel, Chloroform/Methanol 9:1). F.: 153-155°. IR-Spektrum ($CH_2Cl_2$): charakteristische Absorptionsbanden bei 2,90; 3,10; 5,80; 6,25; 9,50 u. Durch präaparative Schichtchromatographie der Mutterlauge (Silicagel, Chloroform/Methanol 95:5) erhält man das E-Isomere vom Rf-Wert 0,70 (Silicagel, Chloroform/Methanol 9:1).

Beispiel i): 2-(5-N-Methylamino-1,2,4-thiadiazol-3-yl)-2-Z-methoxyiminoessigsäure

Ein Gemisch von 1,5 g (6,24 m Mol) 2-(5-N-Methylamino-1,2,4-thiadiazol-3-yl)-2-Z-methoxyiminoessigsäure-tert.butylester in 15 ml Methylenchlorid wird bei Raumtemperatur mit 15 ml Trifluoressigsäure versetzt und während 40 Min. gerührt, dann in 200 ml Diäthyläther/Hexan 1:2 eingerührt. Der Niederschlag wird durch Filtration isoliert, in Wasser gelöst und die wässrige Phase mit wässriger Natriumbicarbonatlösung auf pH 5,3 gestellt. Die wässrige Lösung wird zum Teil eingeengt und anschliessend mit abs. Aethanol versetzt. Der ausgefallene Niederschlag wird durch Filtration isoliert und mit

BAD ORIGINAL

Diäthyläther gewaschen. Man erhält die Titelverbindung vom Rf-Wert 0,35 (Silicagel, s-Butanol/Eisessig/Wasser 67:10:23). IR-Spektrum (Nujol): charakteristische Absorptionsbanden bei 2,80; 3,05; 5,85; 6,20; 6,40; 9,50 µ.

Die Verbindung vom Beispiel i) kann auch gemäss den folgenden Beispielen j) bis m) hergestellt werden:

Beispielj): 2-(5-N-Methyl-N-tert.butoxycarbonylamino-1,2,4-thiadiazol-3-yl)-essigsäure-tert.butylester und 2-(5-N-Methylamino-1,2,4-thiadiazol-3-yl)-essigsäure-tert.-butylester

Eine frisch zubereitete Lösung von Lithiumdiisopropylamid in 200 ml abs.Tetrahydrofuran [aus 26 ml (0,184 Mol) Diisopropylamin und 110 ml einer ca. 2 M Lösung von Butyllithium in Hexan] wird bei -78° unter Stickstoff mit 21,0 g (92,0 mMol) 5-(N-Methyl-N-tert.butoxycarbonylamino)-3-methyl-1,2,4-thiadiazol in 100 ml abs. Tetrahydrofuran versetzt und 1 Std. bei dieser Temperatur gerührt, dann auf -20° erwärmt und mit 200 ml Wasser versetzt. Das Gemisch wird mit Aethylacetat verdünnt, mit Wasser gewaschen, über Magnesiumsulfat getrocknet und im Vakuum eingeengt. Der Rückstand wird chromatographiert (Silicagel, Toluol/steigende Anteile Aethylacetat).

Man erhält den einheitlichen 2-(5-N-Methyl-N-tert.-butoxycarbonylamino-1,2,4-thiadiazol-3-yl)-essigsäure-tert.-butylester vom Rf-Wert 0,80 (Silicagel, Aethylacetat). IR-Spektrum ($CH_2Cl_2$): charakteristische Absorptionsbanden bei 5,78; 5,85; 6,60 µ, sowie einheitlichen 2-(5-N-Methylamino-1,2,4-thiadiazol-3-yl)-essigsäure tert.butylester vom Rf-Wert 0,50 (Silicagel, Aethylacetat).

BAD ORIGINAL

Beispiel k): 2-(5-N-Methyl-N-tert.butoxycarbonylamino-1,2,4-thiadiazol-3-yl)-2-oxo-essigsäure-tert.butylester

Aus 5,0 g (15,2 mMol) 2-(5-N-Methyl-N-tert.butoxycarbonylamino-1,2,4-thiadiazol-3-yl)-essigsäure-tert.butylester und 4,2 g Selendioxid in 75 ml Dioxan erhält man analog Beispiel g) die Titelverbindung vom Rf-Wert 0,70 Silicagel, Chloroform/Methanol 95:5). F. 96-98°. IR-Spektrum ($CH_2Cl_2$): charakteristische Absorptionsbanden bei 5,80; 5,90; 6,60 µ.

Beispiel l): 2-(5-N-Methyl-N-tert.butoxycarbonylamino-1,2,4-thiadiazol-3-yl)-2-Z-methoxyimino-essigsäure-tert.butylester

Aus 6,3 g (18,3 mMol) 2-(5-N-Methyl-N-tert.butoxycarbonylamino-1,2,4-thiadiazol-3-yl)-2-oxo-essigsäure-tert.butylester, 1,65 g Methoxyaminhydrochlorid und 1,6 ml Pyridin in 40 ml 95% Aethanol erhält man analog Beispiel h) die Titelverbindung vom Rf-Wert 0,80 (Silicagel, Chloroform/Methanol 99:1). F. 125-127°; IR-Spektrum ($CH_2Cl_2$): charakteristische Absorptionsbanden bei 5,78; 5,90; 6,62; 8,90 µ.

Beispiel m): 2-(5-N-Methylamino-1,2,4-thiadiazol-3-yl)-2-Z-methoxyiminoessigsäure

Die Titelverbindung kann auch analog Beispiel i) aus 0,5 g (1,34 mMol) 2-(5-N-Methyl-N-tert.butoxycarbonylamino-1,2,4-thiadiazol-3-yl)-2-Z-methoxyiminoessigsäure-tert.butylester und 5 ml Trifluoressigsäure erhalten werden.

Beispiel n) 2-(5-N-Methyl-N-tert.Butoxycarbonylamino-1,2,4-thiadiazol-3-yl)-2-Z-methoxyimino-essigsäure

Eine Lösung von 500 mg (2,30 mMol) 2-(5-N-Methylamino-1,2,4-thiadiazol-3-yl)-2-Z-methoxyiminoessigsäure in

BAD ORIGINAL

7,5 ml Wasser wird mit 0,48 ml Triäthylamin in 7,5 ml Dioxan versetzt und während 10 Min. bei Raumtemperatur gerührt, dann mit 615,5 mg 2-(tert.Butoxycarbonyloxyimino)-2-phenylacetonitril versetzt und während 4 Stunden bei 40° gerührt. Nach Zugabe von weiteren 300 mg 2-(tert.Butoxycarbonyloxyimino)-2-phenylacetonitril wird für weitere 16 Stunden bei 40° gerührt, anschliessend mit Diäthyläther verdünnt und mit Wasser extrahiert. Die wässrige Phase wird mit 20% Zitronensäure auf pH 3,0 gestellt und mit Aethylacetat extrahiert. Die vereinigten organischen Extrakte werden über Magnesiumsulfat getrocknet und im Vakuum eingeengt. Man erhält die Titelverbindung vom Rf-Wert 0,60 (Silicagel, s-Butanol/Eisessig/Wasser 67:10:23).

Beispiel o): 2-(5-tert.Butoxycarbonylamino-1,2,4-thiadiazol-3-yl)essigsäuremethylester

Eine Mischung von 30,0 g (0,115 Mol) 2-(5-tert.Butoxycarbonylamino-1,2,4-thiadiazol-3-yl)essigsäure in 350 ml Methylenchlorid wird nacheinander mit 26,16 g N,N-Dicyclohexylcarbodiimid, 5,13 ml abs.Methanol und 1,71 g 4-Pyrrolidinopyridin versetzt und während 3 Stunden bei Raumtemperatur gerührt. Nach Zugabe von weiteren 0,5 ml abs.Methanol und 2,6 g N,N-Dicyclohexylcarbodiimid wird für weitere 60 Min. gerührt. Der Niederschlag wird abgenutscht, das Filtrat nacheinander mit wässriger Natriumbicarbonatlösung, Wasser und wässriger Natriumchloridlösung gewaschen, über Magnesiumsulfat getrocknet und im Vakuum eingeengt. Der Rückstand wird mit Diäthyläther verrieben. Man erhält die einheitliche Titelverbindung vom Rf-Wert 0,50 (Silicagel, Toluol:Aethylacetat 1:1). IR-Spektrum ($CH_2Cl_2$): charakteristische Absorptionsbanden bei 2,90; 5,75; 5,85; 6,50; 8,70 µ.

BAD ORIGINAL

Beispiel p): 2-(5-tert.Butoxycarbonylamino-1,2,4-thiadiazol-3-yl)-2-oxoessigsäure-methylester

Eine Suspension von 20,0 g (73.18 mMol) 2-(5-tert.-Butoxycarbonylamino-1,2,4-thiadiazol-3-yl)essigsäure-methylester und 22,0 g Selendioxid in 330 ml abs.Dioxan wird 3 Stunden bei 95° gerührt. Nach Filtrieren des Reaktionsgemisches wird das Filtrat im Vakuum eingeengt. Der Rückstand wird mit Aethylacetat verdünnt, nacheinander mit Wasser, wässriger Natriumbicarbonatlösung und wässriger Natriumchloridlösung gewaschen, über Magnesiumsulfat getrocknet und eingeengt. Man erhält die Titelverbindung in Form eines Schaumes vom Rf-Wert 0,35 (Silicagel, Toluol:Aethylacetat 1:1). IR-Spektrum ($CH_2Cl_2$): charakteristische Absorptionsbanden bei 2,95; 5,75; 5,85; 6,50 µ.

Beispiel q): 2-(5-tert.Butoxycarbonylamino-1,2,4-thiadiazol-3-yl)-2-hydroxyiminoessigsäure-methylester

Eine Suspension von 21,8 g (75,88 mMol) 2-(5-tert.-Butoxycarbonylamino-1,2,4-thiadiazol-3-yl)-2-oxoessigsäure-methylester, 7,48 g Hydroxylaminhydrochlorid und 9,8 ml Pyridin in 300 ml 95% Aethanol wird 3 Stunden bei Raumtemperatur gerührt. Das Reaktionsgemisch wird im Vakuum eingeengt. Der Rückstand wird in Aethylacetat gelöst und die Lösung nacheinander mit verdünnter Salzsäure, Wasser und wässriger Natriumchloridlösung extrahiert, über Magnesiumsulfat getrocknet und im Vakuum eingeengt. Der Rückstand wird aus Diäthyläther kristallisiert. Man erhält die Titelverbindung vom Rf-Wert 0,30 (Silicagel, Chloroform:Methanol 95:5).
F. 131-132°. IR-Spektrum ($CH_2Cl_2$): charakteristische Absorptionsbanden bei 2,85; 5,75; 5,85; 6,50;, 8,70; 8,90 µ.

BAD ORIGINAL

Beispiel r): 2-(5-tert.Butoxycarbonylamino-1,2,4-thiadiazol-3-yl)-2-hydroxyiminoessigsäure

Eine Lösung von 8,0 g (26,46 mMol) 2-(5-tert.Butoxycarbonylamino-1,2,4-thiadiazol-3-yl)-2-hydroxyiminoessigsäuremethylester in 175 ml 95% Aethanol wird mit 8,6 g Kaliumhydroxyd und 70 ml Wasser versetzt und während 5 Stunden bei 40° gerührt. Das Reaktionsgemisch wird im Vakuum eingeengt, der Rückstand in Wasser gelöst, die Lösung mit verdünnter Salzsäure auf pH 8,5 gestellt und mit Aethylacetat extrahiert. Die wässrige Phase wird abgetrennt, mit 2 N HCl auf pH 2,0 gestellt und mit Aethylacetat extrahiert. Die organische Phase wird abgetrennt, mit wässriger Natriumchloridlösung gewaschen, über Magnesiumsulfat getrocknet und eingeengt. Der Rückstand wird aus Diäthyläther kristallisiert. Man erhält die einheitliche Titelverbindung vom Rf-Wert 0,60 (Silicagel, s-Butanol/Eisessig/Wasser 67:10:23). F. 158-160° (Zers.); IR-Spektrum (Nujol): charakteristische Absorptionsbanden bei 3,10; 5,85; 8,65 μ.

Beispiel s): 2-(5-tert.Butoxycarbonylamino-1,2,4-thiadiazol-3-yl)-2-Z-methylcarbamoyloxyiminoessigsäure

Eine Lösung von 2,85 g (8,95 mMol) 2-(5-tert.Butoxycarbonylamino-1,2,4-thiadiazol-3-yl)-2-hydroxyiminoessigsäure in 20 ml Tetrahydrofuran wird bei 0-5° mit 10 ml Methylisocyanat versetzt und bei dieser Temperatur während 2 Stunden gerührt. Das Reaktionsgemisch wird mit n-Hexan versetzt und der entstandene Niederschlag abgenutscht. Nach Umkristallisation des Niederschlages aus Methylenchlorid erhält man die einheitliche Titelverbindung vom Rf-Wert 0,25 ($UPC_{12}$-Platten, Wasser/Acetonitril 6:1). F. 124-127°. IR-Spektrum (Nujol): charakteristische Absorptionsbanden bei 5,75; 5,85; 6,40 μ.

BAD ORIGINAL

Beispiel t): 2-(5-tert.Butoxycarbonylamino-1,2,4-thiadiazol-3-yl)-2-Z-(2-tert.butoxycarbonylprop-2-yloxyimino)essigsäure-methylester

Eine Lösung von 1,4 g (6,27 Mol) 2-Bromisobuttersäure-tert.butylester und 1,94 g (14,03 mMol) fein pulverisiertes Kaliumcarbonat in 12,50 ml Dimethylsulfoxid wird bei Zimmertemperatur unter Stickstoff mit einer Lösung von 1,70 g (5,62 mMol) 2-(5-tert.Butoxycarbonylamino-1,2,4-thiadiazol-3-yl)-2-hydroxyiminoessigsäure-methylester in 7,5 ml Dimethylsulfoxid versetzt und 14 Stunden bei dieser Temperatur gerührt. Das Reaktionsgemisch wird bei 30° im Hochvakuum zur Trockene eingedampft. Der Rückstand wird in 200 ml Essigsäureäthylester aufgenommen und nacheinander mit Wasser, verdünnter Salzsäure (1 N), Wasser und gesättigter Natriumchloridlösung gewaschen, über Magnesiumsulfat getrocknet und im Vakuum zur Trockene eingedampft. Der Rückstand wird aus ca. 5 ml Aether kristallisiert und ergibt die Titelverbindung vom Schmelzpunkt 142-144°.

Beispiel u): 2-(5-tert.Butoxycarbonylamino-1,2,4-thiadiazol-3-yl)-2-Z-(2-tert.butoxycarbonylprop-2-yloxyimino)essigsäure

Eine Lösung von 11 g (22,55 mMol) 2-(5-tert.Butoxycarbonylamino-1,2,4-thiadiazol-3-yl)-2-Z-(2-tert.butoxycarbonylprop-2-yloxyimino)essigsäure-methylester in 290 ml Methanol und 96 ml 2 N Natronlauge wird bei einer Temperatur von 50° 6 1/2 Stunden rühren gelassen. Das Reaktionsgemisch wird bei 35° am Hochvakuum zur Trockne eingedampft. Der Rückstand wird in 200 ml Wasser(destilliert)aufgenommen und einmal mit 200 ml Essigsäureäthylester extrahiert. Die wässrige Phase wird mit 200 ml Essigsäureäthylester überschichtet und mit konz. Salzsäure sauer gestellt. Die Essigsäureäthylesterphase wird nacheinander mit Wasser und gesättigter Natriumchloridlösung gewaschen, über Magnesiumsulfat getrocknet und

BAD ORIGINAL

im Vakuum eingeengt, wobei die Titelverbindung kristallin anfällt. DC-Rf-Wert 0,2 (Silicagel, Chloroform/Methanol 4:1); IR-Spektrum (Dioxan): charakteristische Absorptionsbanden bei 5,81; 5.74; 3,3 µ. F.: 180-183°.

Herstellung von Verbindungen der Formel I

Beispiel 1: 7ß-[2-(5-tert.Butoxycarbonylamino-1,2,4-thiadiazol-3-yl)-2-Z-methoxyiminoacetamido]-3-acetoxymethyl-3-cephem-4-carbonsäurediphenylmethylester.

a) Eine Lösung von 1,5 g (4,96 m Mol) 2-(5-tert.Butoxycarbonylamino-1,2,4-thiadiazol-3-yl)-2-methoxyiminoessigsäure in 20 ml Methylenchlorid und 0,44 ml Pyridin wird bei -15° unter Stickstoff mit 0,88 ml (6,0 m Mol) Diäthylphosphorbromidat versetzt und 30 Min. bei dieser Temperatur gerührt. Nach Zugabe von 2,1 g (4,70 m Mol) 7ß-Amino-3-acetoxymethyl-3-cephem-4-carbonsäure-diphenylmethylester bei -15° wird das Reaktionsgemisch bei Raumtemperatur während 2,5 Std. gerührt, dann mit 250 ml Methylenchlorid verdünnt, nacheinander mit verdünnter Schwefelsäure, Wasser, wässriger Natriumbicarbonatlösung und wässriger Natriumchloridlösung gewaschen, über Magnesiumsulfat getrocknet und im Vakuum eingeengt. Der Rückstand wird durch präparative Schichtchromatographie an Silicagel mit Chloroform gereinigt. Man erhält die einheitliche Titelverbindung vom Rf-Wert 0,40 (Silicagel, Toluol/Aethylacetat 1:1); IR-Spektrum ($CH_2Cl_2$): charakteristische Absorptionsbanden bei 3,10; 5,65; 5,80; 5,95; 6,40; 8,20 µ; UV-Spektrum (Methanol): $\lambda$ max ($\varepsilon$) 232(26280) nm.

BAD ORIGINAL

Die gleiche Verbindung kann auch wie folgt hergestellt werden.

b) Eine Lösung von 200 mg (0,28 m Mol) 7β-[2-(5-tert. Butoxycarbonylamino-1,2,4-thiadiazol-3-yl)-2-hydroxyimino-acetamido]-3-acetoxymethyl-3-cephem-4-carbonsäure-diphenylmethylester in 15 ml Methanol wird tropfenweise mit einer 0°-kalten Lösung von Diazomethan in Diäthyläther versetzt, bis die Umsetzung beendet ist und bei 0-5° während 30 Min. gerührt. Das Reaktionsgemisch wird im Vakuum eingeengt und der Rückstand durch präparative Schichtchromatographie an Silicagel mit Chloroform gereinigt. Man erhält die Titelverbindung vom Rf-Wert 0,40 (Silicagel, Toluol/Aethylacetat 1:1).

Beispiel 2: 7β-[2-(5-Amino-1,2,4-thiadiazol-3-yl)-2-Z-methoxyiminoacetamido]-3-acetoxymethyl-3-cephem-4-carbonsäure (Natriumsalz)

Eine Lösung von 3,8 g (5,23 m Mol) 7β-[2-(5-tert.-Butoxycarbonylamino-1,2,4-thiadiazol-3-yl)-2-methoxyimino-acetamido]-3-acetoxymethyl-3-cephem-4-carbonsäure-diphenylmethylester in 8,5 ml Methylenchlorid und 2,8 ml Anisol wird bei Raumtemperatur mit 39 ml Trifluoressigsäure versetzt und während 25 Min. gerührt, dann in 900 ml Diäthyläther/Hexan 1:1 eingerührt. Der Niederschlag, bestehend aus dem Trifluoracetat der 7β-[2-(5-Amino-1,2,4-thiadiazol-3-yl)-2-Z-methoxyimino-acetamido]-3-acetoxymethyl-3-cephem-4-carbonsäure, wird durch Filtration isoliert, in Methanol-haltigem Wasser (10 ml Methanol/500 ml Wasser) gelöst und die wässrige Phase mit wässriger Natriumbicarbonatlösung auf pH 6,8 gestellt. Die Lösung wird lyophilisiert, und die erhaltenen Natriumsalze werden mittels Chormatographie an Silicagel "Opti $UPC_{12}$" (Fa. Antec) mit Wasser/Acetonitril 6:1 gereinigt. Man erhält die einheitliche Titelverbindung vom Rf-Wert 0,30 ($UPC_{12}$-Platten, Wasser/Acetonitril 6:1); F. ab 150° (Zers.);

BAD ORIGINAL

UV-Spektrum (Methanol): λ max (ε): 234 (12240) nm; IR-Spektrum (Nujol): charakteristische Absorptionsbanden bei 3,00; 5,65; 5,95; 6,20; 6,52; 8,10; 9.60 μ.

Beispiel 3: 7ß-[2-(5-tert.Butoxycarbonylamino-1,2,4-thiadiazol-3-yl)-2-Z-methoxyiminoacetamido]-3-(1-methyltetrazol-5-yl-thiomethyl)-3-cephem-4-carbonsäure-diphenylmethylester.

a) Aus 575 mg (1,90 m Mol) 2-(5-tert.Butoxycarbonylamino-1,2,4-thiadiazol-3-yl)-2-methoxyiminoessigsäure, 880 mg (1,80 m Mol) 7ß-Amino-3-(1-methyltetrazol-5-yl-thiomethyl)-3-cephem-4-carbonsäure-diphenylmethylester und 0,33 ml (2,30 m Mol) Diäthylphosphorbromidat in 10 ml Methylenchlorid und 0,17 ml Pyridin erhält man analog Beispiel 1 die einheitliche Titelverbindung vom Rf-Wert 0,30 (Silicagel, Toluol/Aethylacetat 1:1); IR-Spektrum ($CH_2Cl_2$): charakteristische Absorptionsbanden bei 2,90; 5,65; 5,85; 5,95; 6,10; 6.45; 8,60 μ.

ai) Durch präparative Schichtchromatographie an Silicagel mit Toluol/Aethylacetat 1:1 erhält man ausserdem 7ß-[2-(5-tert.Butoxycarbonylamino-1,2,4-thiadiazol-3-yl)-2-Z-methoxyiminoacetamido]-3-(1-methyltetrazol-5-yl-thiomethyl)-2-cephem-4-carbonsäure-diphenylmethylester vom Rf. 0,35 (Silicagel, Toluol/Aethylacetat 1:1).

Die 3-Cephem-Titelverbindung kann auch wie folgt erhalten werden:

b) Eine Suspension von 0,50 g (1,65 m Mol) 2-(5-tert. Butoxycarbonylamino-1,2,4-thiadiazol-3-yl)-2-methoxyiminoessigsäure und 0,152 g (0,825 m Mol) Cyanurchlorid in 30 ml Aceton wird 30 Min. auf 40° erwärmt, wobei eine Lösung entsteht. Nach Zugabe von 0,166 g (1,65 m Mol) Triäthylamin

BAD ORIGINAL

wird die Lösung während 3 Stunden bei Raumtemperatur gerührt. Nach Zugabe von 0,815 g (1,65 m Mol) 7ß-Amino-3-(1-methyltetrazol-5-yl-thiomethyl)-3-cephem-4-carbonsäure-diphenylmethylester wird für weitere 2 Stunden bei Raumtemperatur gerührt, dann im Vakuum eingeengt, mit 50 ml Wasser verdünnt und mit Methylenchlorid extrahiert. Die organische Phase wird nacheinander mit Phosphatpuffer pH 8,0 und wässriger Natriumchloridlösung gewaschen, über Magnesiumsulfat getrocknet und im Vakuum eingeengt. Der Rückstand enthält die Titelverbindung vom Rf 0,30 (Silicagel, Toluol/Aethylacetat 1:1).

c) 0,50 g (1,65 m Mol) 2-(5-tert.Butoxycarbonylamino-1,2,4-thiadiazol-3-yl)-2-methoxyiminoessigsäure und 7 ml Aethylacetat werden bei -5° zu einer gerührten Suspension eines Vilsmeier-Reagenses (dargestellt aus 0,25 g (1,98 m Mol) Oxalylchlorid und 0,15 ml N,N-Dimethylformamid in 1 ml trockenem Aethylacetat gegeben. Das Gemisch wird während 45 Min. bei 0° gerührt und anschliessend auf -10° abgekühlt. Nach Zugabe von 0,81 g (1,65 m Mol) 7ß-Amino-3-(1-methyltetrazol-5-yl-thiomethyl)-3-cephem-4-carbonsäure-diphenylmethylester wird während 2,5 Stunden bei 0° gerührt, dann mit 50 ml Aethylacetat verdünnt und nacheinander mit Wasser, Phosphatpuffer pH 8,0 und wässriger Natriumchloridlösung gewaschen, über Magnesiumsulfat getrocknet und im Vakuum eingeengt. Das Rohprodukt wird durch Fällen aus Aceton-Hexan weiter gereinigt. Man erhält die Titelverbindung vom Rf. 0,30 (Silicagel, Toluol/Aethylacetat 1:1).

d) Eine Suspension von 629 mg (0,84 m Mol) 7ß-[2-(5-tert.Butoxycarbonylamino-1,2,4-thiadiazol-3-yl)-2-oxo-acetamido-3-(1-methyltetrazol-5-yl-thiomethyl)-3-cephem-4-carbonsäurediphenylmethylester, 0,75 g (0,90 m Mol) Methoxyaminhydrochlorid und 0,7 ml Pyridin in 40 ml 95% Aethanol wird während 3 Std. bei Raumtemperatur gerührt. Das Reaktionsgemisch wird im Vakuum eingeengt. Der Rückstand wird mit

BAD ORIGINAL

25 ml Aethylacetat verdünnt und nacheinander mit Wasser, verdünnter Schwefelsäure und wässriger Natriumchloridlösung gewaschen, über Magnesiumsulfat getrocknet und im Vakuum eingeengt. Der Rückstand wird durch präparative Schichtchromatographie an Silicagel mit Toluol/Aethylacetat 3:2 gereinigt. Man erhält die Titelverbindung vom Rf-Wert 0,30 (Silicagel, Toluol/Aethylacetat 1:1).

e) Aus 185 mg (0,26 m Mol) 7ß-[2-(5-tert.Butoxycarbonylamino-1,2,4-thiadiazol-3-yl)-2-hydroxyiminoacetamido]-3-(1-methyltetrazol-5-yl-thiomethyl)-3-cephem-4-carbonsäurediphenylester in 15 ml Methanol und einer Lösung von Diazomethan in Diäthyläther erhält man analog Beispiel 1b) die Titelverbindung vom Rf-Wert 0,30 (Silicagel, Toluol/Aethylacetat 1:1).

f) Eine Lösung von 187 mg (0,25 m Mol) 7ß-[2-(5-tert.-Butoxycarbonylamino-1,2,4-thiadiazol-3-yl)-2-Z-methoxyaminoacetamido]-3-(1-methyltetrazol-5-yl-thiomethyl)-2-cephem-4-carbonsäure-diphenylmethylester in 4 ml Methylenchlorid wird auf 0° gekühlt, bei dieser Temperatur mit 50 mg m-Chlorperbenzoesäure (ca. 90%ig) versetzt und 30 Min. gerührt. Hierauf wird überschüssige Persäure durch Zugabe von Natriumthiosulfat zerstört. Die Reaktionslösung wird nacheinander mit wässriger Natriumbicarbonat- und wässriger Natriumchloridlösung extrahiert, über Natriumsulfat getrocknet und eingeengt. Der Rückstand wird aus Aceton/Diäthyläther gefällt. Man erhält 7ß-[2-(5-tert.Butoxycarbonylamino-1,2,4-thiadiazol-3-yl)-2-Z-methoxyiminoacetamido-3-(1-methyltetrazol-5-yl-thiomethyl)-3-cephem-4-carbonsäure-diphenylmethylester-1-oxid vom Rf-Wert 0,35 (Silicagel, Essigester).

g) Eine Lösung von 115 mg (0,15 M Mol) des 1-Oxids in 3 ml N,N-Dimethylformamid wird auf -10° gekühlt, mit 0,04 ml (0,30 m Mol) Phosphortrichlorid versetzt und 35 Min. gerührt.

BAD ORIGINAL

Die Reaktionslösung wird auf Eis gegossen und mit 20 ml Aethylacetat verdünnt. Die abgetrennte organische Phase wird nacheinander mit wässriger Natriumbicarbonatlösung und wässriger Natriumchloridlösung extrahiert, über Magnesiumsulfat getrocknet und eingeengt. Nach präparativer Schichtchromatographie an Silicagel mit Toluol/Aethylacetat 1:1 erhält man die einheitliche Titelverbindung vom Rf 0,30 (Silicagel, Toluol/Aethylacetat 1:1).

Beispiel 4: 7β-[2-(5-Amino-1,2,4-thiadiazol-3-yl)-2-Z-methoxyiminoacetamido]-3-(1-methyltetrazol-5-yl-thiomethyl)-3-cephem-4-carbonsäure (Natriumsalz).

a) Aus 650 mg (0,87 m Mol) 7β-[2-(5-tert.Butoxycarbonylamino-1,2,4-thiadiazol-3-yl)-2-Z-methoxyiminoacetamido]-3-(1-methyltetrazol-5-yl-thiomethyl)-3-cephem-4-carbonsäure-diphenylmethylester in 1,4 ml Methylenchlorid, 0,47 ml Anisol und 6,5 ml Trifluoressigsäure erhält man analog Beispiel 2 die Titelverbindung vom Rf-Wert 0,25 ($UPC_{12}$-Platten, Wasser/Acetonitril 6:1); UV-Spektrum (Methanol): $\lambda$ max ($\varepsilon$) 232(25200) nm; IR-Spektrum (Nujol): charakteristische Absorptionsbanden bei 3,00; 5,70; 6,25; 6,55; 9,62 µ.

Die gleiche Verbindung kann auch wie folgt hergestellt werden:

b) Eine Suspension von 0,478 g (1,0 m Mol) 7β-[2-(5-Amino-1,2,4-thiadiazol-3-yl)-2-Z-methoxyiminoacetamido]-3-acetoxymethyl-3-cephem-4-carbonsäure Natriumsalz, 0,207 g 1,5 m Mol) 1-Methyltetrazol-5-yl-thiol, 0,369 g (1,0 m Mol) Tetrabutylammoniumjodid und 2,0 g Kaliumjodid in 2 ml Wasser wird bei 70° während 2,5 Stunden gerührt, dann mit Wasser verdünnt und mit Aethylacetat extrahiert. Die wässrige Phase wird eingeengt und chromatographiert ($UPC_{12}$-Platten, Wasser:

BAD ORIGINAL

Acetonitril 6:1). Man erhält die einheitliche Titelverbindung vom Rf-Wert 0,35 ($UPC_{12}$-Platten, Wasser/Acetonitril 6:1).

Beispiel 5: 7ß-[2-(5-tert.Butoxycarbonylamino-1,2,4-thiadiazol-3-yl)-2-Z-methoxyiminoacetamido]-3-carbamoyloxymethyl-3-cephem-4-carbonsäurediphenylmethylester.

Aus 575 mg (1,9 m Mol) 2-(5-tert.Butoxycarbonylamino-1,2,4-thiadiazol-3-yl)-2-methoxyiminoessigsäure, 1,10 g (1,8 m Mol) 7ß-Ammonium-3-carbamoyloxymethyl-3-cephem-4-carbonsäure-diphenylmethylester-Tosylat und 0,4 ml Diäthylphosphorbromidat in 15 ml Methylenchlorid und 0,3 ml Pyridin erhält man analog Beispiel 1 die Titelverbindung vom Rf-Wert 0,60 (Silicagel, Essigester); UV-Spektrum: $\lambda$ max ($\varepsilon$) 230 (sh) nm; IR-Spektrum (Nujol): charakteristische Absorptionsbanden bei 2,90; 3,10; 5,60; 5,80; 6,25; 6,45 $\mu$.

Beispiel 6: 7ß-[2-(5-Amino-1,2,4-thiadiazol-3-yl)-2-Z-methoxyiminoacetamido]-3-carbamoyloxymethyl-3-cephem-4-carbonsäure (Natriumsalz).

Aus 700 mg (0,95 m Mol) 7ß-[2-(5-tert.Butoxycarbonylamino-1,2,4-thiadiazol-3-yl)-2-methoxyiminoacetamido]-3-carbamoyloxymethyl-3-cephem-4-carbonsäure-diphenylmethylester in 1,5 ml Methylenchlorid, 0,5 ml Anisol und 7,1 ml Trifluoressigsäure erhält man analog Beispiel 2 die Titelverbindung vom Rf-Wert 0,50 ($UPC_{12}$-Platten, Wasser/Acetonitril 6:1); F.:ca.215°(Zersetzung); UV-Spektrum (Methanol): $\lambda$max 230 sh; IR-Spektrum (Nujol): charakteristische Absorptionsbanden bei 2,85; 3,05; 5,60; 5,80; 6,25; 6,45 $\mu$.

BAD ORIGINAL

Beispiel 7: 7ß-[2-(5-tert.Butoxycarbonylamino-1,2,4,thiadiazol-3-yl)-2-Z-methoxyiminoacetamido]-3-(1,2,5,6-tetrahydro-2-methyl-5,6-dioxo-1,2,4-triazin-3-yl-thiomethyl)-ceph-3-em-4-carbonsäure

Zu einem bei -5° vorgelegten Gemisch von 0,2 ml Dimethylformamid und 0,2 ml Oxalylchlorid in 5 ml Aethylacetat gibt man eine Lösung von 0,74 g (2-(5-tert.Butoxycarbonylamino-1,2,4-thiadiazol-3-yl)-2-Z-methoxyiminoessigsäure in 5 ml Aethylacetat und rührt die nunmehr klare Lösung 45 Min. bei 0°. Nach Zugabe einer mit 1 ml 2 N Natronlauge auf pH 7,5 eingestellten Lösung von 0,74 g 7ß-Amino-3-(1,2,5,6-tetrahydro-2-methyl-5,6-dioxo-1,2,4-triazin-3-yl-thiomethyl)-3-cephem-4-carbonsäure in 5 ml Wasser wird das Reaktionsgemisch 3 Std. bei 0°C gerührt und dabei durch Zutropfen von 2N Natronlauge auf pH 7,5 gehalten. Nach Zugabe von 100 ml Aethylacetat wird die wässrige Phase mit 2N Salzsäure auf pH 1,5 gestellt und 3 mal mit je 100 ml Aethylacetat extrahiert. Die vereinigten Extrakte werden mit Natriumchloridlösung gewaschen, mit Magnesiumsulfat getrocknet und nach dem Einengen unter Anspritzen mit Aethylacetat kristallisiert. Man erhält die Titelverbindung, welche sich oberhalb 180° zersetzt. IR-Spektrum (Nujol): charakteristische Absorptionsbanden bei: 3,12; 5,63; 5,78; 6,11; 6.46 µ; UV-Spektrum (Aethanol): $\lambda$ max ($\epsilon$): 238 (35800)nm.

BAD ORIGINAL

Beispiel 8: 7ß-[2-(5-Amino-1,2,4-thiadiazol-3-yl)-2-Z-methoxyiminoacetamido]-3-(1,2,5,6-tetrahydro-2-methyl-5,6-dioxo-1,2,4-triazin-3-yl-thiomethyl)-ceph-3-em-4-carbonsäure-Natriumsalz

0,52 g 7ß-[2-(5-tert.Butoxycarbonylamino-1,2,4-thiadiazol-3-yl)-2-Z-methoxyiminoacetamido]-3-(1,2,5,6-tetrahydro-2-methyl-5,6-dioxo-1,2,4-triazin-3-yl-thiomethyl)-3-cephem-4-carbonsäure werden in 5 ml Trifluoressigsäure aufgelöst und 5 Min. bei Raumtemperatur gerührt. Man isoliert wie im Beispiel 2 und erhält die Titelverbindung vom Rf-Wert 0,2 ($UPC_{12}$-Platten, Wasser/Acetonitril 4:1); F. ca. 210°C (Zers.); IR-Spektrum (Nujol): charakteristische Absorptionsbanden bei 2,292; 5,67; 5,99; 6,29; 6,53 µ.

Beispiel 9: 7ß-[2-(5-Amino-1,2,4-thiadiazol-3-yl)-2-Z-methoxyiminoacetoamido]-3-(3-methyl-1,2,4-thiadiazol-5-yl-thiomethyl)-3-cephem-4-carbonsäure Natriumsalz

Eine Suspension von 1,43 g (30 m Mol) 7ß-[2-(5-Amino-1,2,4-thiadiazol-3-yl)-2-Z-methoxyiminoacetamido]-3-acetoxymethyl-3-cephem-4-carbonsäure Natriumsalz und 0,44 g (3,6 m Mol) 3-Methyl-1,2,4-thiadiazol-5-yl-thiol in 10 ml wässriger Natriumbicarbonatlösung wird bei 70° und einem pH von 6,o-6,5 während 2,5 Stunden gerührt, dann mit festem Natriumbicarbonat auf pH 7,0 gebracht und mit Aethylacetat extrahiert. Die wässrige Phase wird im Vakuum eingeengt. Nach Chromatographie an Silicagel "Opti $UPC_{12}$" mit Acetonitril:Wasser 1:9 erhält man die einheitliche Titelverbindung vom Rf 0,40 ($UPC_{12}$-Platten, Wasser-Acetonitril 4:1). F. ab 185° (Zers.). UV-Spektrum (Methanol): $\lambda$ max ($\varepsilon$): 230 (25050); 272 (17700) nm; IR-Spektrum (Nujol): charakteristische Absorptionsbanden bei 3,0; 5,65; 6,0; 6,55;. 9,60 µ.

BAD ORIGINAL

Beispiel 10: 7ß-[2-(5-Amino-1,2,4-thiadiazol-3-yl)-2-Z-methoxyiminoacetamido]-3-(1-carboxymethyl-tetrazol-5-yl-thiomethyl)-3-cephem-4-carbonsäure Natriumsalz

a) Eine Suspension von 0,478 g (1,0 m Mol) 7ß-[2-(5-Amino-1,2,4-thiadiazol-3-yl)-2-Z-methoxyiminoacetamido]-3-acetoxymethyl-3-cephem-4-carbonsäure Natriumsalz, 0,240 g (1,5 m Mol) 1-Carboxymethyltetrazol-5-yl-thiol, 0,370 g Tetrabutylammoniumjodid und 2,0 g Kaliumjodid in 2 ml Wasser wird bei 70° während 2 Stunden gerührt, dann mit Wasser verdünnt und mit Aethylacetat extrahiert. Die Aethylacetat-Extrakte werden mit wässriger Natriumbicarbonatlösung gewaschen. Die vereinigten wässrigen Phasen werden in der Kälte mit 4N Salzsäurelösung auf pH 1,5 gestellt und mit Aethylacetat extrahiert. Die Aethylacetat-Extrakte werden mit wässriger Natriumchloridlösung gewaschen, über Magnesiumsulfat getrocknet und im Vakuum eingeengt. Der Rückstand wird mit Aethylacetat verrieben, das feste Produkt wird abgenutscht und mit Aethylacetat-Aether 1:1 gewaschen. Der Rückstand wird in methanolhaltigem Wasser gelöst und mit festem Natriumcarbonat auf pH 7 gestellt. Nach Lyophilisation dieser Lösung und Chromatographie des Rückstandes an Silicagel "Opti $UPC_{12}$" mit Acetonitril:Wasser 1:6 erhält man die einheitliche Titelverbindung vom Rf-Wert 0,65 ($UPC_{12}$-Platten, Wasser-Acetonitril 6:1). F.ca.210° (Zers.). IR-Spektrum (Nujol) charakteristische Absorptionsbanden bei 3,00; 5.68; 6,20; 6,55; 9,62 μ; UV-Spektrum (Methanol): $\lambda$ max ($\varepsilon$) 232 (24450) nm.

Die gleiche Verbindung kann auch wie folgt hergestellt werden:

BAD ORIGINAL

b) Eine Suspension von 0,570 g (1 M Mol) 7β-[2-(5-Ammonium-1,2,4-thiadiazol-3-yl)-2-Z-methoxyiminoacetamido]-3-acetoxymethyl-3-cephem-4-carbonsäure-trifluoracetat und 0,160 g (1 m Mol)1-Carboxymethyl-tetrazol-5-yl-thiol in 5 ml wässriger Natriumbicarbonatlösung wird bei einem pH von 6,0-6,5 während 2 Stunden bei 70° gerührt, dann mit festem Natriumbicarbonat auf pH 7,0 gebracht und mit Aethylacetat extrahiert. Die wässrige Phase wird im Vakuum eingeengt. Nach Chromatographie an Silicagel "Opti $UPC_{12}$" (Fa. Antec) mit Acetonitril:Wasser 1:6 erhält man die einheitliche Titelverbindung vom Rf-Wert 0,65 ($UPC_{12}$-Platten, Wasser:Acetonitril 6:1).

Beispiel 11: 7β-[2-(5-tert,Butoxycarbonylamino-1,2,4-thiadiazol-3-yl)-2-methoxyiminoacetamido]-3-{1-[2-N,N-dimethylammonium)äthyl]-1H-tetrazol-5-yl-thiomethyl}-3-cephem-4-carbonsäure-tetrafluorborat

0,298 g (0,99 m Mol) 2-(5-tert.Butoxycarbonylamino-1,2,4-thiadiazol-3-yl)-2-methoxyiminoessigsäure wird bei -5° zu einer gerührten Suspension eines Vilsmeier-Reagenses (dargestellt aus 0,1 ml (1,2 m Mol) Oxalylchlorid und 0,09 ml N,N-Dimethylformamid in 4 ml trockenem Aethylacetat) gegeben und das Gemisch wird bei dieser Temperatur während 30 Min. gerührt (Lösung 1). Andererseits wird eine Mischung von 0,473 g (1,0 m Mol) 7β-Amino-3-{1-[2-(N,N-dimethylamino)äthyl]-1H-tetrazol-5-yl-thiomethyl}-3-cephem-4-carbonsäure-tetrafluoroborat, 1,1 ml N,O-Bis(trimethylsilyl)acetamid und 5 ml trockenes Aethylacetat während 30 Min. bei Raumtemperatur gerührt und anschliessend auf -15° abgekühlt (Lösung 2). Lösung 1 wird zu der -15° kalten Lösung 2 gegeben und die Mischung wird bei dieser Temperatur während 3,5 Stunden gerührt, dann mit 40 ml Aethylacetat verdünnt und mit Wasser extrahiert. Die wässrigen Extrakte werden eingeengt und lyophilisiert. Ein Teil des Lyophilisats wird chromatographiert ($UPC_12$-Platten), Wasser:Acetonitril 1:1). Man erhält die einheitliche Titel-

BAD ORIGINAL

verbindung vom Rf 0,30 ($UPC_{12}$-Platten, Wasser-Acetonitril 1:1). IR-Spektrum (KBr): charakteristische Absorptionsbanden bei 2,95; 5,62;. 5,85; 6,00; 6,45 µ; UV-Spektrum (Methanol): $\lambda$ max ($\varepsilon$) 235 (17400)nm.

Beispiel 12: 7β-[-2-(5-Amino-1,2,4-thiadiazol-3-yl)-2-Z-methoxyiminoacetamido]-3{1-[2-N,N-dimethylaminoäthyl]-1H-tetrazol-5-yl}-thiomethyl-3-cephem-4-carbonsäure Natriumsalz

Eine Lösung von 2,1 g (2,77 m Mol) 7β-[2-(5-tert.-Butoxycarbonylamino-1,2,4-thiadiazol-3-yl)-2-methoxyiminoacetamido]-3-{1-[2-(N,N-dimethylammoniumäthyl]-1H-tetrazol-5-yl}-thiomethyl-3-cephem-4-carbonsäure-tetrafluoroborat in 4,7 ml Methylenchlorid wird bei Raumtemperatur mit 21 ml Trifluoressigsäure versetzt und während 30 Min. gerührt, dann in 1,0 ml Diäthyläther/Hexan 1:2 eingerührt. Der Niederschlag wird durch Filtration isoliert, in 50 ml Methanol gelöst und mit einer methanolischen ca. 3N Natrium-2-äthylhexanoatlösung auf pH 7,0 gestellt. Nach Zugabe von 100 ml Aether wird der gebildete Niederschlag abgenutscht und chromatographiert (Silicagel "Opti $UPC_{12}$", Wasser/Acetonitril 9:1). Man erhält die einheitliche Titelverbindung vom Rf 0,35 ($UPC_{12}$-Platten, Wasser/Acetonitril 4:1). F. 190° (Zers.). IR-Spektrum (Nujol): charakteristische Absorptionsbanden bei 3,0; 5,65; 5,95; 6,20; 6,45 u; UV-Spektrum (Methanol) $\lambda_{max}$ = 231 nm ($\varepsilon$= 27800).

BAD ORIGINAL

Beispiel 13: 7ß-[2-(5-tert.Butoxycarbonylamino-1,2,4-thiadiazol-3-yl)-acetamido]-3-acetoxymethyl-3-cephem-4-carbonsäure-diphenylmethylester

Eine Lösung von 500 mg (1,90 m Mol) 2-(5-tert.Butoxycarbonylamino-1,2,4-thiadiazol-3-yl)essigsäure und 830 mg (1,90 m Mol) 7ß-Amino-3-acetoxymethyl-3-cephem-4-carbonsäure-diphenylmethylester in 10 ml trockenem Tetrahydrofuran wird bei 0° mit 210 mg 1-Hydroxybenzotriazol und 475 mg (2,30 m Mol) N,N'-Dicyclohexylcarbodiimid in 6 ml trockenem Tetrahydrofuran versetzt, während 3,5 Std. bei 0° gerührt und anschliessend auf Raumtemperatur erwärmt. Nach Filtrieren des Reaktionsgemisches wird das Filtrat mit 50 ml Aethylacetat verdünnt und nacheinander mit wässriger Natriumbicarbonat- und wässriger Natriumchloridlösung gewaschen, über Magnesiumsulfat getrocknet und im Vakuum eingeengt. Der Rückstand wird durch präparative Schichtchromatographie an Silicagel mit Chloroform/Methanol 95:5 gereinigt. Es entsteht die einheitliche Titelverbindung vom Rf-Wert 0,65 (Silicagel, Chloroform/Methanol 95:5). UV-Spektrum (Methanol): $\lambda_{max}$ ($\varepsilon$) 248 (14500 nm; IR-Spektrum (Methylenchlorid): charakteristische Absorptionsbanden bei 3,0; 5,57; 5,80; 6,45; 8,15 μ.

Beispiel 14: 7ß-[2-(5-tert.Butoxycarbonylamino-1,2,4-thiadiazol-3-yl)-2-hydroxyiminoacetamido]-3-acetoxymethyl-3-cephem-4-carbonsäure-diphenylmethylester

Eine Lösung von 340 mg (0,5 m Mol) 7ß-[2-(5-tert.-Butoxycarbonylamino-1,2,4-thiadiazol-3-yl)-acetamido]-3-acetoxymethyl-3-cephem-4-carbonsäure-diphenylmethylester in 3 ml Eisessig wird bei 10-15° mit 38 mg (0,55 m Mol) Natriumnitrit versetzt und während 30 Min. bei dieser Temperatur gerührt. Das Reaktionsgemisch wird mit 20 ml Aethylacetat

BAD ORIGINAL

verdünnt und nacheinander mit Wasser, wässriger Natriumbicarbonat- und wässriger Natriumchloridlösung gewaschen, über Magnesiumsulfat getrocknet und im Vakuum eingeengt. Der Rückstand wird durch präparative Schichtchromatographie an Silicagel mit Aethylacetat gereinigt. Man erhält die Titelverbindung vom Rf 0,30 (Silicagel, Aethylacetat). IR-Spektrum (Nujol): charakteristische Absorptionsbanden bei 3,05; 5,65; 5,85; 5,90; 6,45; 9,05 µ.

Beispiel 15: 7β-[2-(5-tert.Butoxycarbonylamino-1,2,4-thiadiazol-3-yl)-acetamido]-3-(1-methyltetrazol-5-yl-thiomethyl)-3-cephem-4-carbonsäure-diphenylmethylester

Aus 518 mg (2,0 m Mol) 2-(5-tert.Butoxycarbonylamino-1,2,4-thiadiazol-3-yl)essigsäure, 940 mg (1,90 m Mol) 7β-Amino-3-(1-methyltetrazol-5-yl-thiomethyl)-3-cephem-4-carbonsäure-diphenylmethylester, 210 mg 1-Hydroxybenzotriazol und 475 mg (2,30 m Mol) N,N'-Dicyclohexylcarbodiimid in 30 ml Tetrahydrofuran erhält man analog Beispiel 13 die einheitliche Titelverbindung vom Rf-Wert 0,60 (Silicagel, Chloroform/Methanol/Eisessig 85:12:3). IR-Spektrum (Methylenchlorid) charakteristische Absorptionsbanden bei 3,0; 5,65; 5,90; 6,45 µ.

Beislie1 16: 7β-[2-(5-tert.Butoxycarbonylamino-1,2,4-thiadiazol-3-yl)-2-hydroxyiminoacetamido]-3-(1-methyltetrazol-5-yl-thiomethyl)-3-cephem-4-carbonsäure-diphenylmethylester

Aus 258 mg (0,38 m Mol) 7β-[2-(5-tert.Butoxycarbonylamino-1,2,4-thiadiazol-3-yl)-acetamido]-3-(1-methyltetrazol-

BAD ORIGINAL

5-yl-thiomethyl)-3-cephem-4-carbonsäure-diphenylmethylester und 29 mg (0,42 m Mol) Natriumnitrit in 2,5 ml Eisessig erhält man analog Beispiel 14 die Titelverbindung vom Rf-Wert 0,30 (Silicagel, Toluol/Aethylacetat 1:1). IR-Spektrum (Nujol) charakteristische Absorptionsbanden bei 2,90; 5,60; 5,85; 5,95; 6,20; 6.45; 9,10 µ.

Beispiel 17: 7β-[2-(5-tert.Butoxycarbonylamino-1,2,4-thiadiazol-3-yl)-2-oxo-acetamido]-3-(1-methyltetrazol-5-yl-thiomethyl)-3-cephem-4-carbonsäure-diphenylmethylester

0,683 g (2,50 m Mol) 2-(5-tert.Butoxycarbonylamino-1,2,4-thiadiazol-3-yl)-2-oxoessigsäure und 12 ml Aethylacetat werden bei -5° zu einer gerührten Suspension eines Vilsmeier-Reagenses (hergestellt aus 0,38 g (3,0 m Mol) Oxalylchlorid, 0,20 ml N,N-Dimethylformamid in 1,5 ml trockenem Aethylacetat) gegeben. Das Gemisch wird während 35 Min. bei 0° gerührt. Nach Zugabe von 1,235 g (2,50 m Mol) 7β-Amino-3-(1-methyltetrazol-5-yl-thiomethyl)-3-cephem-4-carbonsäure-diphenylmethylester wird während 3 Stunden bei 0° gerührt, dann mit 50 ml Aethylacetat verdünnt und nacheinander mit Wasser, Phosphatpufferlösung pH 8,0 und wässriger Natriumchloridlösung gewaschen, über Magnesiumsulfat getrocknet und im Vakuum eingeengt. Der Rückstand wird durch präparative Schichtchromatographie an Silicagel mit Chloroform/Aethylacetat/Eisessig 100/100/0,5 gereinigt. Man erhält die einheitliche Titelverbindung vom Rf 0,60 (Silicagel, Chloroform/Aethylacetat/Eisessig 100:100:0,5). IR-Spektrum ($CH_2Cl_2$): charakteristische Absorptionsbanden bei 2,75; 5,60; 5,85; 6,10; 6,45;. 8,50 µ.

BAD ORIGINAL

Beispiel 18: 7β-[2-(5-Amino-1,2,4-thiadiazol-3-yl)-2-Z-hydroxyiminoacetamido]-3-(1-methyltetrazol-5-yl-thiomethyl)-3-cephem-4-carbonsäure Natriumsalz

Aus 355 mg (0,5 m Mol) 7β-[2-(5-tert.Butoxycarbonylamino-1,2,4-thiadiazol-3-yl)-2-hydroxyiminoacetamido]-3-(1-methyltetrazol-5-yl-thiomethyl)-3-cephem-4-carbonsäure-diphenylmethylester in 0,80 ml Methylenchlorid, 0,27 ml Anisol und 3,75 ml Trifluoressigsäure erhält man analog Beispiel 2 die einheitliche Titelverbindung vom Rf-Wert 0,15 ($UPC_{12}$-Platten, Wasser/Aectonitril 9:1) F.: ab 185° (Zers.). IR-Spektrum (Nujol): charakteristische Absorptionsbanden bei 3,00; 5,65; 5,95; 6,25; 6,55; 9,10 μ.

Beispiel 19: 7α-Methoxy-7β-[2-(5-tert.butoxycarbonylamino-1,2,4-thiadiazol-3-yl)-2-Z-methoxyiminoacetamido]-3-acetoxymethyl-3-cephem-4-carbonsäure-diphenylmethylester

Eine Lösung von 2,18 g (3,0 m Mol) 7β-[2-(5-tert.-Butoxycarbonylamino-1,2,4-thiadiazol-3-yl)-2-Z-methoxyiminoacetamido]-3-acetoxymethyl-3-cephem-4-carbonsäure-diphenylmethylester in 200 ml absolutem Tetrahydrofuran wird auf -75° (Trockeneis-Aceton-Bad) abgekühlt und eine vorgekühlte Lösung von Lithiummethoxid in Methanol (83 mg Lithium in 9 ml Methanol gelöst) innert 2 Minuten zugetropft, wobei die Temperatur auf -70° steigt und eine orangegefärbte Lösung entsteht. Das Reaktionsgemisch wird weitere 2 Minuten bei -75° gerührt, dann 0,25 ml tert.-Butylhypochlorit zugegeben und bei -75° weitergerührt. Nach 8 Minuten wird die Lösung mit weiteren 0,2 ml tert.-Butylhypochlorit versetzt. Nach 30 Minuten werden nacheinander 3,3 ml Eisessig und eine Lösung von 1,5 g Natriumthiosulfat in 1,9 ml Wasser in das Reaktionsgemisch getropft. Das Kühlbad wird entfernt und die Lösung auf Raumtemperatur erwärmt. Das Gemisch wird im Vakuum eingeengt und mit Aethylacetat verdünnt. Die Aethylacetatlösung wird nacheinander mit

BAD ORIGINAL

Wasser, wässriger Natriumbicarbonatlösung und Wasser gewaschen, über Magnesiumsulfat getrocknet und im Vakuum eingeengt. Das Rohprodukt wird durch Chromatographie an Silicagel mit Methylenchlorid und steigenden Anteilen Aethylacetat gereinigt. Man erhält die Titelverbindung vom Rf 0,30 (Silicagel, Toluol/Aethylacetat 3:2). IR-Spektrum (Methylenchlorid): charakteristische Absorptionsbanden bei 2,95; 5,60; 5,85; 5,95; 6,45; 9,50 µ.

Beispiel 20: 7α-Methoxy-7β-[2-(5-Amino-1,2,4-thiadiazol-3-yl)-2-Z-methoxyiminoacetamido]-3-acetoxymethyl-3-cephem-4-carbonsäure Natriumsalz

Aus 0,44 g (1,01 m Mol) 7a-Methoxy-7β-[2-(5-tert.-butoxycarbonylamino-1,2,4-thiadiazol-3-yl)-2-Z-methoxyimino-acetamido]-3-acetoxymethyl-4-carbonsäure-diphenylmethylester in 3 ml Methylenchlorid, 1 ml Anisol und 14,2 ml Trifluoressigsäure erhält man analog Beispiel 2 die Titelverbindung vom Rf-Wert 0,45 (UPC$_1$2-Platten, Wasser/Acetonitril 9:1). F. ab 190° (Zers.). IR-Spektrum (Nujol): charakteristische Absorptionsbanden bei 3,0; 5,60; 5,95; 6,20; 6,55; 9,55 µ.

Beispiel 21: 7β-[2-(5-Amino-1,2,4-thiadiazol-3-yl)-2-Z-methoxyiminoacetamido]-3-acetoxymethyl-3-cephem-4-carbonsäurepivaloylcxymethylester

Eine Mischung von 1,91 g (4,0 m Mol) 7β-[2-(5-Amino-1,2,4-thiadiazol-3-yl)-2-Z-methoxyiminoacetamido]-3-acetoxymethyl-3-cephem-4-carbonsäure Natriumsalz in 22 ml N,N-Dimethylformamid wird bei 0° mit 0,9 ml (3,85 m Mol) Jodmethyl pivalat versetzt und während 60 Min. bei dieser Temperatur gerührt, dann in 120 ml Phosphatpufferlösung pH 7.0 gegossen und mit Aethylacetat/Aceton 4:1 extrahiert. Die organischen Extrakte werden mit wässriger Natriumchloridlösung gewaschen, über Magnesiumsulfat getrocknet und im Vakuum eingeengt. Der

BAD ORIGINAL

Rückstand wird mit Diäthyläther verrieben, der Feststoff abgenutscht und mit Diäthyläther/Hexan 1:1 nachgewaschen. Man erhält die Titelverbindung vom Rf-Wert 0,80 (Silicagel, s-Butanol/Eisessig/Wasser 67:10:23). IR-Spektrum (Nujol): charakteristische Absorptionsbanden bei 3,0; 5,60; 5,70; 5,90; 6,15; 6.45 µ.

Beispiel 22: 7ß-[2-(5-Amino-1,2,4-thiadiazol-3-yl)-2-Z-methoxyiminoacetamido]-3-carbamoyloxymethyl-3-cephem-4-carbonsäure-pivaloyloxymethylester

Aus 2,39 g (5,0 m Mol) 7ß-[2-(5-Amino-1,2,4-thiadiazol-3-yl)-2-Z-methoxyiminoacetamido]-3-carbamoyloxymethyl-3-cephem-4-carbonsäure Natriumsalz und 1,13 ml (4,81 m Mol) Jodmethylpivalat in 25 ml N,N-Dimethylformamid erhält man analog Beispiel 21 die Titelverbindung vom Rf-Wert 0,70 (Silicagel, s-Butanol/Eisessig/Wasser 67:10:23). IR-Spektrum (Nujol): charakteristische Absorptionsbanden bei 3,05; 5,60; 5,72; 5,95; 6,25;. 6,45 µ.

Beispiel 23: 7ß-[2-(5-Amino-1,2,4-thiadiazol-3-yl)-2-Z-methoxyiminoacetamido]-3-(1-methyltetrazol-5-yl-thiomethyl)-3-cephem-4-carbonsäure-pivaloyloxymethylester

Aus 4,27 g (8,0 mMol) 7ß-[2-(5-Amino-1,2,4-thiadiazol-3-yl]-2-Z-methoxyiminoacetmido-3-(1-methyl-tetrazol-5-yl-thiomethyl)-3-cephem-4-carbonsäure Natriumsalz und 1,8 ml (7,70 m Mol) Jodmethylpivalat in 45 ml N,N-Dimethylformamid erhält man analog Beispiel 21 die Titelverbindung vom Rf-Wert 0,80 (Silicagel, Aceton/Eisessig/Wasser/Ammoniak 20:8:5:1). IR-Spektrum (Nujol): charakteristische Absorptionsbanden bei 2,95; 5,65; 5,80; 5,95; 6,45 µ.

BAD ORIGINAL

Beispiel 24: 7ß-[2-(5-Amino-1,2,4-thiadiazol-3-yl)-2-Z-methoxyiminoacetamido]-3-{1-[2-(N,N-dimethylamino)äthyl]-1H-tetrazol-5-yl-thiomethyl}-3-cephem-4-carbonsäure-pivaloyloxymethylester-hydrochlorid

Aus 3,5 g (5,91 m Mol) 7ß-[2-(5-Amino-1,2,4-thiadiazol-3-yl)-2-Z-methoxyiminoacetamido]-3-{1-[2-(N,N-dimethylamino)-äthyl]-1H-tetrazol-5-yl-thiomethyl}-3-cephem-4-carbonsäure Natriumsalz und 1,35 ml (5,78 m Mol) Jodmethylpivalat in 30 ml N,N-Dimethylformamid erhält man analog Beispiel 21 die Titelverbindung, die als Hydrochlorid isoliert wird. Rf-Wert: 0,60 (Aceton/Eisessig/Wasser/Ammoniak 20:8:5:1). IR-Spektrum (Nujol): charakteristische Absorptionsbanden bei 3,0; 5,60; 5,85; 6,05; 6.45 μ.

Beispiel 25: 7ß-[2-(5-Amino-1,2,4-thiadiazol-3-yl)-2-Z-methoxyiminoacetamido]-3-(1-pivaloyloxymethoxycarbonylmethyltetrazol-5-yl-thiomethyl-3-cephem-4-carbonsäure-pivaloyloxymethylester

Aus 4,8 g (8,28 m Mol) 7ß-[2-(5-Amino-1,2,4-thiadiazol-3-yl)-2-Z-methoxyiminoacetamido]-3-(1-carboxymethyl-tetrazol-5-yl-thiomethyl)-3-cephem-4-carbonsäure Natriumsalz und 3,6 ml (15,44 m Mol) Jodmethylpivalat in 60 ml N,N-Dimethylformamid erhält man analog Beispiel 21 ein Rohprodukt, das nach Chromatographie an Silicagel die Titelverbindung vom Rf-Wert 0,55 (Silicagel, Aceton/Eisessig/Wasser/Ammoniak 20:8:5:1) ergibt. IR-Spektrum (Nujol): charakteristische Absorptionsbanden bei 3,0; 5,65;. 6,20;. 6,50 μ.

Man erhält ausserdem geringe Mengen an 7ß-[2-(5-Amino-1,2,4-thiadiazol-3-yl)-2-Z-methoxyiminoacetamido]-3-(1-carboxymethyl-tetrazol-5-yl-thiomethyl)-3-cephem-4-carbonsäure-pivaloyloxymethylester vom Rf-Wert 0,40 (Silicagel, Aceton/Eisessig/Wasser/Ammoniak 20:8:5:1) sowie an 7ß-[2-(5-Amino-

BAD ORIGINAL

1,2,4-thiadiazol-3-yl)-2-Z-methoxyiminoacetamido]-3-(1-pivaloyloxymethoxycarbonylmethyl-tetrazol-5-yl-thiomethyl)-3-cephem-4-carbonsäure vom Rf-Wert 0,30 (Silicagel, Aceton/Eisessig/Wasser/Ammoniak 20:8:5:1).

Beispiel 26: 7ß-[2-(5-tert.Butoxycarbonylamino-1,2,4-thiadiazol-3-yl)-2-Z-methoxyiminoacetamido]-3-(1-sulfomethyl-tetrazol-5-yl-thiomethyl)-3-cephem-4-carbonsäure

295 mg (0,98 m Mol) 2-(5-tert.Butoxycarbonylamino-1,2,4-thiadiazol-3-yl)-2-methoxyiminoessigsäure wird bei -5° zu einer gerührten Suspension eines Vilsmeier-Reagenses (dargestellt aus 0,1 ml (1,2 m Mol) Oxalylchlorid und 0,09 ml N,N-Dimethylformamid in 4 ml trockenem Aethylacetat) gegeben und das Gemisch wird bei dieser Temperatur während 30 Min. gerührt (Lösung 1). Andererseits werden 408 mg (1,0 m Mol) 7ß-Amino-3-(1-sulfomethyl-tetrazol-5-yl-thiomethyl)-3-cephem-4-carbonsäure, 0,69 ml Triäthylamin und 3 ml N,O-Bis(trimethylsilyl)acetamid während 30 Min. bei Raumtemperatur gerührt und anschliessend auf -15° abgekühlt (Lösung 2). Lösung 1 wird zu der -15° kalten Lösung 2 gegeben und die Mischung wird bei dieser Temperatur während 1 Stunde gerührt. Die unlöslichen Bestandteile werden abfiltriert und das Filtrat mit Diäthyläther/Petroläther 1:1 versetzt. Der ausgeschiedene Feststoff wird abfiltriert und mit Petroläther nachgewaschen. Nach präparativer Schichtchromatographie An Silicagel $UPC_{12}$ mit Wasser/Acetonitril 4:1 erhält man die einheitliche Titelverbindung vom Rf-Wert 0,40 ($UPC_{12}$-Platten, Wasser/Acetonitril 4:1). IR-Spektrum (Nujol): charakteristische Absorptionsbanden bei 3,10; 5,65; 5,85; 6,05; 6,20; 6,45 µ.

BAD ORIGINAL

Beispiel 27: 7ß-[2-(5-Amino-1,2,4-thiadiazol-3-yl)-2-Z-methoxyiminoacetamido]-3-(1-sulfomethyl-tetrazol-5-yl-thiomethyl)-3-cephem-4-carbonsäure Natriumsalz

Eine Lösung von 400 mg (0,58 m Mol) 7ß-[2-(5-tert.-Butoxycarbonylamino-1,2,4-thiadiazol-3-yl)-2-Z-methoxyiminoacetamido]-3-(1-sulfomethyl-tetrazol-5-yl-thiomethyl)-3-cephem-4-carbonsäure im 0,95 ml Methylenchlorid wird bei Raumtemperatur mit 4 ml Trifluoressigsäure versetzt und während 30 Min. bei Raumtemperatur gerührt, dann in 300 ml Diäthyläther/Hexan 1:2 eingerührt. Der Niederschlag wird durch Filtration isoliert, in 8 ml Methanol gelöst und mit einer methanolischen 3N Natrium-2-äthylhexanoatlösung auf pH 7,0 gestellt. Nach Zugabe von 70 ml Aether wird der gebildete Niederschlag abgenutscht und chromatographiert ($UPC_{12}$-Platten, Wasser/Acetonitril 9:1). Man erhält die einheitliche Titelverbindung vom Rf-Wert 0,40 ($UPC_{12}$-Platten, Wasser/Acetonitril 9:1): F. ab 175° (Zers.). IR-Spektrum (Nujol): charakteristische Absorptionsbanden bei 3,0; 5,65; 6,20; 6,50;, 9,65 μ; UV-Spektrum (Methanol): $\lambda$max ($\varepsilon$) 233 (21950) nm.

Beispiel 28: 7ß-[2-(5-Amino-1,2,4-thiadiazol-3-yl)-2-Z-methoxyiminoacetamido]-3-(4-Oxo-3,4-dihydro-pyrimidin-2-yl-thiomethyl)-3-cephem-4-carbonsäure Natriumsalz

Eine Suspension von 478 mg (1,0 m Mol) 7ß-[2-(5-Amino-1,2,4-thiadiazol-3-yl)-2-Z-methoxyiminoacetamido]-3-acetoxymethyl-3-cephem-4-carbonsäure Natriumsalz und 154 mg (1,2 m Mol) 4-Oxo-3,4-dihydro-pyrimidin-2-yl-thiol in 5 ml Wasser wird mit festem Natriumbicarbonat auf pH 6,5 gestellt und bei diesem pH-Wert während 4 Std. bei 70° gerührt. Die Lösung wird mit festem Natriumbicarbonat auf pH 7,0 gebracht und lyophilisiert. Der Rückstand wird durch präparative Schichtchromatographie an $UPC_{12}$-Platten mit Wasser/Dioxan 98:2 gereinigt. Man erhält die einheitliche Titelverbindung vom Rf-Wert 0,25 ($UPC_{12}$-Plat-

BAD ORIGINAL

ten, Wasser/Dioxan 98:2). IR-Spektrum (Nujol): charakteristische Absorptionsbanden bei 3,0; 5,65; 6,15; 9,60 µ.

Beispiel 29: 7β-[2-(5-N-Methylamino-1,2,4-thiadiazol-3-yl-2-Z-methoxyiminoacetamido]-3-acetoxymethyl-3-cephem-4-carbonsäure-diphenylmethylester

1,84 g (10,0 m Mol) 2-(5-N-Methylamino-1,2,4-thiadiazol-3-yl)-2-methoxyiminoessigsäure und 5 ml Aethylacetat werden bei -5° zu einer gerührten Suspension eines Vilsmeier-Reagenses (dargestellt aus 1 ml (1,20 m Mol) Oxalylchlorid und 0,9 ml N,N-Dimethylformamid in 30 ml trockenem Aethylacetat) gegeben. Das Gemisch wird bei 0° während 1 Std. gerührt und anschliessend auf -10° abgekühlt. Nach Zugabe von 2,75 g (11,0 m Mol) 7β-Amino-3-acetoxymethyl-3-cephem-4-carbonsäure-diphenylmethylester wird während 3 Std. bei 0° gerührt, dann mit 100 ml Aethylacetat verdünnt und nacheinander mit wässriger Natriumbicarbonatlösung und wässriger Natriumchloridlösung gewaschen. Die organische Phase wird über Magnesiumsulfat getrocknet und im Vakuum eingeengt. Der Rückstand wird durch Chromatographie an Silicagel mit Chloroform gereinigt. Man erhält die Titelverbindung vom Rf-Wert 0,35 (Silicagel, Aethylacetat). IR-Spektrum (Methylenchlorid): charakteristische Absorptionsbanden bei 3,0; 5,65;. 5,80; 5,90; 6,40 µ.

Beispiel 30: 7β-[2-(5-N-Methylamino-1,2,4-thiadiazol-3-yl)-2-Z-methoxyiminoacetamido]-3-acetoxymethyl-3-cephem-4-carbonsäure Natriumsalz

Aus 1,0 g (1,57 m Mol) 7β-[2-(5-N-Methylamino-1,2,4-thiadiazol-3-yl)-2-methoxyiminoacetamido]-3-acetoxymethyl-3-cephem-4-carbonsäure-diphenylmethylester in 1,3 ml Methylenchlorid, 2,5 ml Anisol und 10 ml Trifluoressigsäure erhält man analog Beispiel 4 die Titelverbindung vom Rf-Wert 0,20

BAD ORIGINAL

($UPC_{12}$-Platten, Wasser/Acetonitril 6:1). F. ab 180° (Zers.). IR-Spektrum (Nujol): charakteristische Absorptionsbanden bei 3,0; 5,65; 5,95; 6,25; 6,50; 8,15 μ.

Beispiel 31: 7β-[2-(5-tert.Butoxycarbonylamino-1,2,4-thiadiazol-3-yl)-2-Z-methylcarbamoyloxyiminoacetamido]-3-acetoxymethyl-3-cephem-4-carbonsäure-diphenylmethylester

1,05 g (3,70 mMol) 2-(5-tert.Butoxycarbonylamino-1,2,4-thiadiazol-3-yl)-2-Z-methylcarbamoyloxyiminoessigsäure und 0,4 ml N-Methylmorpholin werden bei -5° zu einer gerührten Suspension eines Vilsmeier-Reagenses (dargestellt aus 0,031 ml Oxalylchlorid und 0,29 ml N,N-Dimethylformamid in 10 ml Aethylacetat) gegeben. Das Gemisch wird während 3o Min. bei 0° gerührt und anschliessend auf -10° abgekühlt. Nach Zugabe von 1,357 g (3,1 mMol) 7β-Amino-3-acetoxymethyl-3-cephem-4-carbonsäure-diphenylmethylester wird während 2,5 Stunden bei 0° gerührt, dann mit Aethylacetat verdünnt und nacheinander mit Wasser, Phosphatpuffer pH 8,0 und wässriger Natriumchloridlösung gewaschen, über Magnesiumsulfat getrocknet und im Vakuum eingeengt. Ein Teil des Rohproduktes wird durch präparative Schichtchromatographie mit Aethylacetat gereinigt. Man erhält die einheitliche Titelverbindung vom Rf-Wert 0,60 (Silicagel, Aethylacetat). IR-Spektrum ($CH_2Cl_2$): charakteristische Absorptionsbanden bei 2,95; 3,10; 5,65; 5,80; 5,95;, 8,20 μ.

Beispiel 32: 7β-[2-(5-Amino-1,2,4-thiadiazol-3-yl)-2-Z-methylcarbamoyloxyiminoacetamido]-3-acetoxymethyl-3-cephem-4-carbonsäure Natriumsalz

Aus 519 g (0,68 mMol) 7β-[2-(5-tert.Butoxycarbonylamino-1,2,4-thiadiazol-3-yl)-2-Z-methylcarbamoyloxyiminoacetamido]-3-acetoxymethyl-3-cephem-4-carbonsäurediphenylmethylester in 1,1 ml Methylenchlorid, 0,37 ml Anisol und

BAD ORIGINAL

5 ml Trifluoressigsäure erhält man analog Beispiel 2 die Titelverbindung vom Rf-Wert 0,40 ($UPC_{12}$-Platten, Wasser/Acetonitril 6:1). IR-Spektrum (Nujol): charakteristische Absorptionsbanden bei 3,0; 5,67; 6,22; 8,10 µ.

Beispiel 33: 7β-[2-(5-Amino-1,2,4-thiadiazol-3-yl)-2-Z-methylcarbamoyloxyiminoacetamido]-3-(1-methyltetrazol-5-yl-thiomethyl)-3-cephem-4-carbonsäure Natriumsalz

Aus 530 mg (1,02 mMol) 7β-[2-(5-Amino-1,2,4-thiadiazol-3-yl)-2-Z-methylcarbamoyloxyiminoacetamido]-3-acetoxymethyl-3-cephem-4-carbonsäure Natriumsalz und 150 mg (1,09 mMol) 1-Methyltetrazol-5-ylthiol in 5,1 ml wässriger Natriumbicarbonatlösung erhält man analog Beispiel 10b) die einheitliche Titelverbindung vom Rf-Wert 0,35 ($UPC_{12}$-Platten, Wasser/Acetonitril 6:1). IR-Spektrum (Nujol): charakteristische Absorptionsbanden bei 3,0; 5,65; 6,20 µ.

Beispiel 34: 7β-[2-(5-Amino-1,2,4-thiadiazol-3-yl)-2-Z-methylcarbamoyloxyiminoacetamido]-3-(1-carboxymethyl-tetrazol-5-yl-thiomethyl-3-cephem-4-carbonsäure Natriumsalz (1) und 7β-[2-(5-Amino-1,2,4-thiadiazol-3-yl)-2-Z-hydroxyimino-acetamido]-3-(1-carboxymethyl-tetrazol-5-yl-thiomethyl)-3-cephem-4-carbonsäure Natriumsalz (2)

Eine Suspension von 0,260 g (0,5 mMol) 7β-[2-(5-Amino-1,2,4-thiadiazol-3-yl)-2-Z-methylcarbamoyloxyiminoacetamido]-3-acetoxymethyl-3-cephem-4-carbonsäure Natriumsalz und 0,08 g 1-Carboxymethyl-tetrazol-5-yl-thiol in 2,5 ml wässriger Natriumbicarbonatlösung wird bei einem pH von 6,5 während 3 Stunden bei 70° gerührt, anschliessend mit weiteren 0,02 g 1-Carboxymethyl-tetrazol-5-ylthiol versetzt und nach Einstellung des pH-Wertes auf 6,5 mit festem Natriumbicarbonat noch eine Stunde bei 70° gerührt, dann mit festem Natriumbicarbonat

BAD ORIGINAL

auf pH 7,0 gebracht und mit Aethylacetat extrahiert. Die wässrige Phase wird im Vakuum eingeengt. Nach Chromatographie an Silicagel "Opti $UPC_{12}$" (Fa. Antec) mit Wasser erhält man die einheitliche Titelverbindung (1) vom Rf-Wert 0,60 ($UPC_{12}$-Platten, Wasser:Acetonitril 6:1). IR-Spektrum (Nujol): charakteristische Absorptionsbanden bei 3,0; 5,67; 6.17 µ sowie die einheitliche Titelverbindung (2) vom Rf-Wert 0,85 ($UPC_{12}$-Platten, Wasser:Acetonitril 6:1), IR-Spektrum (Nujol): charakteristische Absorptionsbanden bei 3,05; 5,67; 6.15; 8,30 µ.

Beispiel 35: 7ß-[2-(5-Amino-1,2,4-thiadiazol-3-yl)-2-Z-methylcarbamoyloxyiminoacetamido]-3-(1-sulfomethyl-tetrazol-5-ylthiomethyl)-3-cephem-4-carbonsäure Natriumsalz (1) und

7ß-[2-(5-Amino-1,2,4-thiadiazol-3-yl)-2-Z-hydroxyimino-acetamido]-3-(1-sulfomethyl-tetrazol-5-yl-thiomethyl)-3-cephem-4-carbonsäure Natriumsalz (2)

Eine Suspension von 0,306 g (0,5 mMol) 7ß-[2-(5-Ammonium-1,2,4-thiadiazol-3-yl)-2-Z-methylcarbamoyloxyiminoacetamido]-3-acetoxymethyl-3-cephem-4-carbonsäure-trifluoracetat, 0,164 g Natrium-1-sulfomethyl-tetrazol-5-yl-thiolat und 2,0 g Natriumjodid in 1,0 ml wässriger Natriumbicarbonatlösung wird bei einem pH von 6,5 während 2 Stunden bei 70° gerührt, anschliessend abgekühlt und auf 500 ml Aethanol gegossen. Der ausgefallene Niederschlag wird filtriert und nacheinander mit Aethanol und Hexan gewaschen. Nach Chromatographie an Silicagel Opti $UPC_{12}$ erhält man die einheitliche Titelverbindung (1) vom Rf-Wert 0,20 ($UPC_{12}$-Platten, Wasser:Acetonitril 98:2) sowie die einheitliche Titelverbindung (2) vom Rf-Wert 0,65 ($UPC_{12}$-Platten, Wasser:Acetonitril 98:2).

BAD ORIGINAL

Beispiel 36: 7ß-[2-(5-Amino-1,2,4-thiadiazol-3-yl)-2-Z-methylcarbamoyloxyiminoacetamido]-3-(1-methyltetrazol-5-yl-thiomethyl)-3-cephem-4-carbonsäure-pivaloyloxymethylester

Aus 2,30 g (4,0 mMol) 7ß-[2-(5-Amino-1,2,4-thiadiazol-3-yl)-2-Z-methylcarbamoyloxyiminoacetamido]-3-(1-methyltetrazol-5-yl-thiomethyl)-3-cephem-4-carbonsäure Natriumsalz, und 0,9 ml (3,85 mMol) Jodmethylpivalat in 20 ml N,N-Dimethylformamid erhält man analog Beispiel 21 die Titelverbindung vom Rf-Wert 0,85 (Silicagel, Aceton/Eisessig/Wasser/Ammoniak 20:8:5:1). IR-Spektrum (Nujol): charakteristische Absorptionsbanden bei 2,95; 5,63; 5,93;. 6.45 µ.

Beispiel 37: 7ß-[2-(5-N-Methylamino-1,2,4-thiadiazol-3-yl)-2-Z-methoxyiminoacetamido]-3-(1-carboxymethyltetrazol-5-yl-thiomethyl)-3-cephem-4-carbonsäure Natriumsalz

Aus 350 mg (0,71 mMol) 7ß-[2-(5-Methylamino-1,2,4-thiadiazol-3-yl)-2-Z-methoxyiminoacetamido]-3-acetoxymethyl-3-cephem-4-carbonsäure Natriumsalz und 136 mg 1-Carboxymethyl-tetrazol-5-ylthiol in 3 ml wässriger Natriumbicarbonatlösung erhält man analog Beispiel 10b) die Titelverbindung vom Rf-Wert 0,70 ($UPC_{12}$-Platten, Wasser/Acetonitril 9:1). IR-Spektrum (Nujol): charakteristische Absorptionsbanden bei 3,0; 5,65; 6,25; 6,45 µ.

Beispiel 38: 7ß-[2-(5-N-Methyl-tert.Butoxycarbonylamino-1,2,4-thiadiazol-3-yl)-2-methoxyiminoacetamido]-3-(1-methyltetrazol-5-yl-thiomethyl)-3-cephem-4-carbonsäure-diphenylmethylester

316 mg (1,0 mMol) 2-(5-N-Methyl-tert.Butoxycarbonylamino-1,2,4-thiadiazol-3-yl)-2-methoxyiminoessigsäure und 5 ml Aethylacetat werden bei -5° zu einer gerührten Suspen-

BAD ORIGINAL

sion eines Vilsmeier-Reagenses (dargestellt aus 151,5 mg Oxalylchlorid und 0,09 ml N,N-Dimethylformamid in 1 ml Aethylacetat) gegeben. Das Gemisch wird während 45 Min. bei 0° gerührt und anschliessend auf -10° abgekühlt. Nach Zugabe von 490 mg 7β-Amino-3-(1-methyltetrazol-5-yl-thiomethyl)-3-cephem-4-carbonsäure-diphenylmethylester wird während 3 Std. bei 0° gerührt, dann mit Aethylacetat verdünnt und nacheinander mit Wasser, Phosphatpuffer pH 8,0 und wässriger Natriumchloridlösung gewaschen, über Magnesiumsulfat getrocknet und im Vakuum eingeengt. Man erhält die Titelverbindung vom Rf-Wert 0,25 (Silicagel, Toluol/Aethylacetat 1:1). IR-Spektrum ($CH_2Cl_2$): charakteristische Absorptionsbanden bei 3,0; 5,65; 6,45 μ.

Beispiel 39: 7β-[2-(5-N-Methylamino-1,2,4-thiadiazol-3-yl)-2-Z-methoxyiminoacetamido]-3-(1-methyltetrazol-5-yl-thiomethyl-3-cephem-4-carbonsäure Natriumsalz

Aus 460 mg (0,61 mMol) 7β-[2-(5-N-Methyl-tert.Butoxycarbonylamino-1,2,4-thiadiazol-3-yl)-2-methoxyiminoacetamido]-3-(1-methyltetrazol-5-yl-thiomethyl)-3-cephem-4-carbonsäure-diphenylmethylester in 1 ml Methylenchlorid, 0,33 ml Anisol und 4,6 ml Trifluoressigsäure erhält man analog Beispiel 2 die Titelverbindung vom Rf-Wert 0,35 ($UPC_{12}$-Platten, Wasser/Acetonitril 6:1). IR-Spektrum (Nujol): charakteristische Absorptionsbanden bei 3,0; 5,65; 6.45 μ.

Beispiel 40: 7β-[2-(5-tert.Butoxycarbonylamino-1,2,4-thiadiazol-3-yl)-2-Z-2-tert.butoxycarbonylprop-2-yloxyamino)-acetamido]-3-acetoxymethyl-3-cephem-4-carbonsäure

Eine Suspension von 15 ml Methylenchlorid abs., 0,3 ml Dimethylformamid und 0,33 ml (7,84 mMol) Oxalsäuredichlorid wird bei -10° 30 Minuten unter Stickstoff gerührt. Dann

BAD ORIGINAL

wird 1,3 g (3,01 mMol) 7β-[2-(5-tert.Butoxycarbonylamino-1,2,4-thiadiazol-3-yl)-2-Z-(2-tert.butoxycarbonylprop-2-yloxyimino)essigsäure zur Suspension gegeben. Die entstehende klare Lösung wird 30 Minuten bei 0-5° unter Stickstoff gerührt. Zu dieser Lösung tropft man eine frisch hergestellte Lösung von 0,87 g (3,19 mMol) 7β-Aminocephalosporansäure in 18 ml Methylenchlorid abs. und 3 ml N,O-Bistrimethylsilylacetamid und rührt bei einer Temperatur von 0-5° unter Stickstoff 16 Stunden. Die Reaktionslösung wird nacheinander mit Wasser und gesättigter Natriumchloridlösung gewaschen, über Magnesiumsulfat getrocknet und am Vakuum zum trockenen Schaum eingedampft. Man erhält die Titelverbindung vom Rf-Wert 0,43 (Silicagel, Essigester/Essigsäure 9:1). IR-Spektrum (Nujol): charakteristische Absorptionsbanden bei 5,6; 5,82; 6,04 µ.

Beispiel 41: 7β-[2-(5-tert.Butoxycarbonylamino-1,2,4-thiadiazol-3-yl)-2-Z-(2-carboxyprop-2-yloxyimino)acetamido]-3-acetoxymethyl-3-cephem-4-carbonsäure Dinatriumsalz

Eine Lösung von 2,3 g (3,35 mMol) 7β-[2-(5-tert.Butoxycarbonylamino-1,2,4-thiadiazol-3-yl)-2-Z-(2-tert.butoxycarbonylprop-2-yloxyimino)acetamido]-3-acetoxymethyl-3-cephem-4-carbonsäure in 12,54 ml (16,38 mMol) Trifluoressigsäure und 2,10 ml (19,28 mMol) Anisol wird bei Zimmertemperatur 1 Stunde gerührt. Das Reaktionsgemisch wird bei 30° Badtemperatur am Vakuum zur Trockene eingedampft, der Rückstand mit Aether verrührt und das Produkt abfiltriert. Dann wird das Produkt in 28 ml Methanol gelöst und mit Natrium-äthylhexanoat versetzt. Man rührt die Lösung in 50 ml Essigsäureäthylester ein. Das ausgefallene Produkt wird abfiltriert und am Hochvakuum getrocknet. Man erhält die Titelverbindung mit dem Rf-Wert 0,78 ($UPC_{12}$-Platten, Wasser/Acetonitril 4:1). IR-Spektrum (Nujol): charakteristische Absorptionsbanden bei 3,04; 5,68; 6,24; 6,57 µ.

BAD ORIGINAL

Beispiel 42: 7β-[2-(5-tert.Butoxycarbonylamino-1,2,4-thiadiazol-3-yl)-2-Z-(2-tert.butoxycarbonylprop-2-yloxyimino)-acetamido]-3-(1-methyltetrazol-5-ylthiomethyl)-3-cephem-4-carbonsäure-diphenylmethylester

Eine Lösung von 15 ml Methylenchlorid, 0,094 ml (0,854 mMol) N-Methylmorpholin und 0,35 g (0,814 mMol) 7β-[2-(5-tert.Butoxycarbonylamino-1,2,4-thiadiazol-3-yl)-2-γ-(2-tert.butoxycarbonylprop-2-yloxyimino)essigsäure wird auf -10° gekühlt, mit 0,11 ml (0,854 mMol) Chlorameisensäurebutylester versetzt und 45 Minuten gerührt. Nach Zugabe von 0,422 g (0,854 mMol) 7β-Amino-3-(1-methyltetrazol-5-ylthiomethyl)-3-cephem-4-carbonsäure-diphenylmethylester wird die Lösung 5 Stunden bei Raumtemperatur gerührt. Das Reaktionsgemisch wird im Vakuum bei 35° Badtemperatur zur Trockne eingedampft. Der Rückstand wird in Essigsäureäthylester aufgenommen und nacheinander mit verdünnter Natriumbicarbonatlösung, Wasser, 1N Salzsäure und gesättigter Natriumchloridlösung gewaschen, über Magensiumsulfat getrocknet und im Vakuum eingedampft. Der Rückstand wird auf Dickschichtplatten gereinigt. Man erhält die Titelverbindung vom Rf-Wert 0,49 (Silicagel, Toluol/Essigsäureäthylester 1:1). IR-Spekrtum (Nujol): charakteristische Absorptionsbanden bei 5,58; 5,82; 6,17; 6,44 μ.

Beispiel 43: 7β-[2-(5-Amino-1,2,4-thiadiazol-3-yl)-2-Z-(2-carboxyprop-2-yloxyimino)acetamido]-3-(1-methyltertazol-5-ylthiomethyl)-3-cephem-4-carbonsäure-Dinatriumsalz

Eine Lösung von 100 mg (0,11 mMol) 7β-[2-(5-tert.-Butoxycarbonylamino-1,2,4-thiadiazol-3-yl)-2-Z-(2-tert.butoxycarbonylprop-2-yloxyimino)acetamido]-3-(1-methyltetrazol-5-ylthiomethyl)-3-cephem-4-carbonsäure-diphenylmethylester in 5 ml (65,33 mMol) Trifluoressigsäure und 1 ml (3,18 mMol) Anisol wird bei Raumtemperatur 3 Stunden gerührt. Das

BAD ORIGINAL

Reaktionsgemisch wird im Vakuum bei 30° Badtemperatur zur Trockne eingedampft, der Rückstand mit Aether verrührt und der Niederschlag abfiltriert. Das Produkt wird in 2 ml Methanol gelöst und mit Natrium-äthylhexanoat versetzt. Die Lösung wird mit Aktivkohle klarfiltriert. Die klare Lösung wird in 10 ml Essigsäureäthylester eingetragen und am Wasserstrahlvakuum eingeengt bis Kristallisation eintritt. Man erhält die Titelverbindung mit dem Rf-Wert 0,38 ($UPC_{12}$-Platten Wasser/Acetonitril 9:1). IR-Spektrum (Nujol): charakteristische Absorptionsbanden bei 2,95; 5,67; 5,97; 6,25 μ.

Beispiel 44: 7β-[2-(5-Amino-1,2,4-thiadiazol-3-yl)-2-Z-(2-carboxyprop-2-yloxyimino)acetamido]-3-[1-(2-N,N-dimethylaminoäthyl)tetrazol-5-ylthiomethyl]-3-cephem-4-carbonsäure

Eine Lösung von 0,28 g (0,5 mMol) 1-(2-N,N-Dimethylaminoäthyl)-5-mercapto-tetrazol und 1,64 g (10,94 mMol) Natriumjodid in 0,80 ml Wasser wird auf 80° erwärmt, mit 0,12 g (0,71 mMol) 7β-[2-(5-Amino-1,2,4-thiadiazol-3-yl)-2-Z-(2-carboxyprop-2-yloxyimino)acetamido]-3-acetoxymethyl-3-cephem-4-carbonsäure-dinatriumsalz und 0,05 ml konzentrierter Salzsäure versetzt und bei 70° während einer Stunde gerührt. Die auf Raumtemperatur abgekühlte Reaktionsmischung wird in 15 ml Eiswasser gerührt, klarfiltriert und mit 0,1 N Natronlauge versetzt bis pH 6 erreicht ist. Nach Abdampfen des Lösungsmittels bei 35° im Vakuum wird der Rückstand am Hochvakuum getrocknet und auf $UPC_{12}$-Platten chromatographisch gereinigt. Man erhält die Titelverbindung mit dem Rf-Wert 0,21 ($UPC_{12}$-Platten, Wasser/Acetonitril 9:1). IR-Spektrum (Nujol): charakteristische Absorptionsbanden bei 5,68; 5,94 und 9,24 u.

BAD ORIGINAL

Beispiel 45: 7β-[2-(5-Amino-1,2,4-thiadiazol-3-yl)-acetamido]-3-acetoxymethyl-3-cephem-4-carbonsäure (Natriumsalz)

Aus 1,0 g (1,47 mMol) 7β-[2-(5-tert.Butoxycarbonylamino-1,2,4-thiadiazol-3-yl)acetamido]-3-acetoxymethyl-3-cephem-4-carbonsäure-diphenylmethylester in 2,4 ml Methylenchlorid, 0,8 ml Anisol und 11,2 ml Trifluoressigsäure erhält man analog Beispiel 2 die Titelverbindung vom Rf-Wert 0,35 ($UPC_{12}$-Platten, Wasser/Acetonitril 6:1). UV-Spektrum (Methanol): $\lambda$ max ($\varepsilon$):246 (8100).

Beispiel 46: 7β-[2-(5-Amino-1,2,4-thiadiazol-3-yl)-acetamido]-3-(1-methyltetrazol-5-yl-thiomethyl)-3-cephem-4-carbonsäure (Natriumsalz)

Aus 0,70 g (0,95 mMol) 7β-[2-(5-tert.Butoxycarbonylamino-1,2,4-thiadiazol-3-yl)-acetamido]-3-(1-methyltetrazol-5-yl-thiomethyl)-3-cephem-4-carbonsäure-diphenylmethylester in 1,5 ml Methylenchlorid, 0,5 ml Anisol und 7,2 ml Trifluoressigsäure erhält man analog Beispiel 2 die Titelverbindung vom Rf-Wert 0,35 ($UPC_{12}$-Platten, Wasser/Acetonitril 4:1). UV-Spektrum (Methanol): $\lambda$ max ($\varepsilon$): 244 (13240).

Beispiel 47: In analoger Weise zu den vorstehenden Beispielen werden, ausgehend von entsprechenden Ausgangsmaterialien, die folgenden Verbindungen erhalten:

7β-[2-(5-Amino-1,2,4-thiadiazol-3-yl)-2-Z-methoxyiminoacetamido]-3-(2-methyl-1,3,4-thiadiazol-5-yl-thiomethyl)-3-cephem-4-carbonsäure,

7β-[2-(5-Amino-1,2,4-thiadiazol-3-yl)-2-Z-methoxyiminoacetamido]-3-[1-(2-sulfoäthyl)-1H-tetrazol-5-yl-thiomethyl]-3-cephem-4-carbonsäure.

BAD ORIGINAL

7β-[2-(5-Amino-1,2,4-thiadiazol-3-yl)-2-Z-hydroxyiminoacetamido]-3-acetoxymethyl-3-cephem-4-carbonsäure.

7β-[2-(5-Amino-1,2,4-thiadiazol-3-yl)-2-Z-methoxyiminoacetamido]-3-(3-hydroxypyridazin-6-yl-thiomethyl)-3-cephem-4-carbonsäure.

7β-[2-(5-Amino-1,2,4-thiadiazol-3-yl)-2-Z-(2-carboxymethoxyimino)acetamido]-3-acetoxymethyl-3-cephem-4-carbonsäure,

7β-[2-(5-Amino-1,2,4-thiadiazol-3-yl)-2-Z-carbamoyloxyiminoacetamido]-3-acetoxymethyl-3-cephem-4-carbonsäure ,

7β-[2-(5-Amino-1,2,4-thiadiazol-3-yl)-2-Z-(1-carboxycyclopropyloxyimino)acetamido]-3-(1-methyltetrazol-5-ylthiomethyl-3-cephem-4-carbonsäure,

7β-[2-(5-Amino-1,2,4-thiadiazol-3-yl)-2-Z-methylcarbamoyloxyiminoacetamido]-3-[1-(2-sulfoäthyl)-tetrazol-5-ylthiomethyl]3-cephem-4-carbonsäure,

7β-[2-(5-Amino-1,2,4-thiadiazol-3-yl)-2-Z-methylcarbamoyloxyiminoacetamido]-3-[1-(2-carboxyäthyl)-tetrazol-5-ylthiometh 3-cephem-4-carbonsäure,

7β-[2-(5-Amino-1,2,4-thiadiazol-3-yl)-2-Z-methylcarbamoyloxyiminoacetamido]-3-(1-carboxymethyltetrazol-5-ylthiomethyl)-3-cephem-4-carbonsäure-pivaloyloxymethylester,

7β-[2-(5-Amino-1,2,4-thiadiazol-3-yl)-2-Z-methylcarbamoyloxyiminoacetamodo]-3-(1-dimethylaminomethyltetrazol-5-ylthiomethyl-3-cephem-4-carbonsäure-pivaloyloxymethylester,

BAD ORIGINAL

7β-[2-(5-Amino-1,2,4-thiadiazol-3-yl)-2-Z-methylcarbamoyloxy-iminoacetamido]-3-carbamoyloxymethyl-3-cephem-4-carbonsäure,

7β-[2-(5-Amino-1,2,4-thiadiazol-3-yl)-2-Z-methylcarbamoyloxy-iminoacetamido]-3[1-(2-dimethylaminoäthyl)-tetrazol-5-ylthio-methyl]-3-cephem-4-carbonsäure,

7β-[2-(5-Amino-1,2,4-thiadiazol-3-yl)-2-Z-carbamoyloxyimino-acetamido]-3-(1-methyltetrazol-5-ylthiomethyl-3-cephem-4-carbonsäure,

7β-[2-(5-Amino-1,2,4-thiadiazol-3-yl)-2-Z-carbamoyloxyimino-acetamido]-3-carboxymethyltetrazol-5-ylthiomethyl)-3-cephem-4-carbonsäure,

7β-[2-(5-Amino-1,2,4-thiadiazol-3-yl)-2-Z-carbamoyloxyimino-acetamido]-3-(1-sulfomethyltetrazol-5-ylthiomethyl)-3-cephem-4-carbonsäure,

7β-[2-(5-Amino-1,2,4-thiadiazol-4-yl)-2-Z-carbamoyloxyimino-acetamido]-3-(1-dimethylaminomethyltetrazol-5-ylthiomethyl)-3-cephem-4-carbonsäure,

7β-[2-(5-Methylamino-1,2,4-thiadiazol-3-yl)-2-Z-methoxy-iminoacetamido]-3-(1-sulfomethyltetrazol-5-ylthiomethyl)-3-cephem-4-carbonsäure,

7β-[2-(5-Methylamino-1,2,4-thiadiazol-3-yl)-2-Z-methoxy-iminoacetamido]-3-[1-(2-dimethylaminoäthyl)tetrazol-3-ylthio-methyl]-3-cephem-4-carbonsäure,

7β-[2-(5-Methylamino-1,2,4-thiadiazol-3-yl)-2-Z-methoxy-iminoacetamido]-3-(1-methyltetrazol-5-ylthiomethyl)-3-cephem-4-carbonsäure-pivaloyloxymethylester,

BAD ORIGINAL

7β-[2-(5-Methylamino-1,2,4-thiadiazol-3-yl)-2-Z-methoxyimino-acetamido]-3-(1-carboxymethyltetrazol-5-ylthiomethyl)-3-caphem-4-carbonsäure-pivaloyloxymethylester,

7β-[2-(5-Amino-1,2,4-thiadiazol-3-yl)-2-hydroxyiminoacet-amido]-3-[1-(2-dimethylaminoäthyl)tetrazol-5-yl-thiomethyl]-3-cephem-4-carbonsäure,

7β-[2-(5-Amino-1,2,4-thiadiazol-3-yl)-2-hydroxyiminoacet-amido]-3-(1-methyltetrazol-5-ylthiomethyl)-3-cephem-4-carbonsäure-pivaloyloxymethylester,

7β-[2-(5-Amino-1,2,4-thiadiazol-3-yl)-acetamido]-3-(1-carboxy-methyltetrazol-5-ylthiomethyl)-3-cephem-4-carbonsäure,

7β-[2-(5-Amino-1,2,4-thiadiazol-3-yl)acetamido]-3-(1-sulfo-methyltetrazol-5-ylthiomethyl)-3-cephem-4-carbonsäure,

7β-[2-(5-Amino-1,2,4-thiadiazol-3-yl)acetamido]-3-[1-(2-di-methylaminoäthyltetrazol-5-ylthiomethyl]-3-cephem-4-carbonsäure,

die Natriumsalze und Dinatriumsalze von denjenigen Verbindungen, die eine bzw. zwei saure Gruppen enthalten und die Hydrochloride von denjenigen Verbindungen, die eine basische Gruppe enthalten.

Beispiel 48: Trockenampullen oder Vials, enthaltend 0,5 g Wirksubstanz, z.B. 7β-[2-(5-Amino-1,2,4-thiadiazol-3-yl)-2-Z-methoxyiminoacetamido]-3-(1-methyltetrazol-5-yl-thiomethyl)-3-cephem-4-carbonsäure (Natriumsalz), werden wie folgt hergestellt.

BAD ORIGINAL

Zusammensetzung (für 1 Ampulle oder Vial)

| | |
|---|---|
| Wirksubstanz | 0,5 g |
| Mannit | 0,05 g |

Eine sterile, wässrige Lösung der Wirksubstanz und des Mannits wird unter aseptischen Bedingungen in 5 ml.-Ampullen oder 5 ml.-Vials verschlossen und geprüft.

Beispiel 49: Trockenampullen oder Vials, enthaltend 0,25 g Wirksubstanz, z.B. 7ß-[2-(5-Amino-1,2,4-thiadiazol-3-yl)-2-Z-methoxyiminoacetamido]-3-(1-methyltetrazol-5-ylthiomethyl)-3-cephem-4-carbonsäure (Natriumsalz), werden wie folgt hergestellt:

Zusammensetzung (für 1-Ampulle oder Vial)

| | |
|---|---|
| Wirksubstanz | 0,25 g |
| Mannit | 0,03 g |

Eine sterile, wässrige Lösung der Wirksubstanz und des Mannits wird unter aseptischen Bedingungen in 5 ml.-Ampullen oder 5 ml.-Vials verschlossen und geprüft.

BAD ORIGINAL

Patentansprüche (für alle benannten Länder ausser Oesterreich)

1. 7β-Aminothiadiazolylacetamido-3-cephem-4-carbonsäureverbindungen der Formel

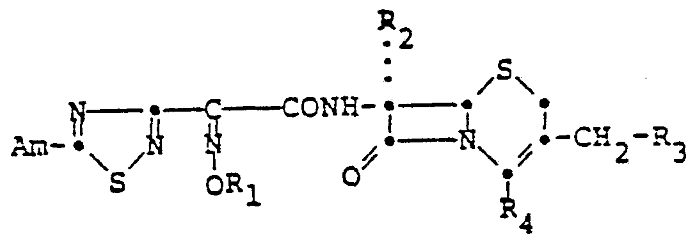

(I),

worin Am eine gegebenenfalls geschützte Aminogruppe darstell[t], $R_1$ für Wasserstoff, gegebenenfalls substituiertes Niederalky[l] oder Cycloalkyl, oder gegebenenfalls N-substituiertes Carbamoyl steht, $R_2$ Wasserstoff oder Niederalkoxy bedeutet, $R_3$ fü[r] Wasserstoff, Acyloxy oder Heterocyclylthio steht, und $R_4$ ein[e] gegebenenfalls geschützte Carboxylgruppe darstellt, und Salz[e] von solchen Verbindungen, die eine salzbildende Gruppierung aufweisen.

2. Verbindungen der Formel (I) nach Patentanspruch 1, worin Am Amino oder Methylamino, $R_1$ Wasserstoff, Niederalkyl, insbesondere Methyl, durch Carboxy substituiertes Niederalkyl oder Cycloalkyl, insbesondere 2-Carboxy-2-propyl oder 1-Carboxy-1-cyclopropyl, oder eine Gruppe -C(=O)-NHR, worin R Wasserstoff oder Niederalkyl, insbesondere Methyl, bedeute[t], $R_2$ Wasserstoff oder Methoxy, $R_3$ Niederalkanoyloxy, wie Acetoxy, Carbamoyloxy, N-Niederalkylcarbamoyloxy, oder gegebenenfalls substituiertes, mit einem Ringkohlenstoffatom an die Thiogruppe gebundenes Heterocyclylthio, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell hydriert und gegebenenfalls beispielsweise durch Niederalkyl, wie Methyl, N,N-Diniederalkylamino-

BAD ORIGINAL

niederalkyl, wie 2-N,N-Dimethylaminoäthyl, Carboxyniederalkyl, wie Carboxymethyl oder 2-Carboxyäthyl, Sulfoniederalkyl, wie Sulfomethyl oder 2-Sulfoäthyl, Amino, Carboxyniederalkanoylamino, Carbamoyl, Oxo oder Hydroxy substituiert sein können, und $R_4$ Carboxyl oder in physiologisch spaltbarer Form verestertes Carboxyl, z.B. Acyloxymethoxycarbonyl, darstellt und pharmazeutisch verwendbare Salze von solchen Verbindungen.

3. Verbindungen der Formel (I) nach Patentanspruch 1, worin Am Amino oder Methylamino, $R_1$ Wasserstoff, Niederalkyl, insbesondere Methyl, oder durch Carboxy substituiertes Niederalkyl oder Cycloalkyl, insbesondere 2-Carboxy-2-propyl oder 1-Carboxy-1-cyclopropyl, $R_2$ Wasserstoff oder Methoxy, $R_3$ Niederalkanoyloxy, wie Acetoxy, Carbamoyloxy, N-Niederalkylcarbamoyloxy, oder gegebenenfalls substituiertes, mit einem Ringkohlenstoffatom an die Thiogruppe gebundenes Heterocyclylthio, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell hydriert und gegebenenfalls beispielsweise durch Niederalkyl, wie Methyl, N,N-Diniederalkylaminoniederalkyl, wie 2-N,N-Dimethylaminoäthyl, Carboxyniederalkyl, wie Carboxymethyl oder 2-Carboxyäthyl, Sulfoniederalkyl, wie Sulfomethyl oder 2-Sulfoäthyl, Amino, Carboxyniederalkanoylamino, Carbamoyl, Oxo oder Hydroxy substituiert sein können, und $R_4$ Carboxyl oder in physiologisch spaltbarer Form verestertes Carboxyl, z.B. Acyloxymethoxycarbonyl, darstellt, sowie pharmazeutisch verwendbare Salze von solchen Verbindungen.

BAD ORIGINAL

4. Verbindungen der Formel (I) nach Patentanspruch 1 worin Am Amino oder Methylamino, $R_1$ Wasserstoff, Methyl, 2-Carboxy-2-propyl, 1-Carboxy-1-cyclopropyl, Carbamoyl oder N-Methylcarbamoyl, $R_2$ Wasserstoff oder Methoxy, $R_3$ Acetoxy, Carbamoyloxy, gegebenenfalls durch Niederalkyl, Carboxyniederalkyl, Sulfoniederalkyl oder Diniederalkylaminoniederalkyl substituiertes Tetrazolylthio, z.B. 1-Methyl-1H- tetrazol-5-ylthio, 1-Sulfomethyl-1H-tetrazol-5-ylthio, 1(2-Sulfoäthyl)-1H-tetrazol-5-ylthio, 1-Carboxymethyl-1H-tetrazol-5-ylthio, 1-(2-Dimethylaminoäthyl)-1H-tetrazol-5-ylthio, gegebenenfalls durch Niederalkyl substituiertes Thiadiazolylthio, wie 2-Methyl-1,3,4-thiadiazol-5-ylthio oder 3-Methyl-1,2,4-thiadiazol-5-ylthio, gegebenenfalls durch Hydroxy substituiertes Pyridazinylthio, z.B. 3-Hydroxy-pyridazin-6-ylthio, gegebenenfalls durch Hydroxy substituiertes Pyrimidinylthio, z.B. 4-Hydroxy-2-pyrimidinyl- bzw. das dazu tautomere 4-Oxo-3,4-dihydro-2-pyrimidinylthio, oder durch Niederalkyl und zwei Hydroxy substituiertes Triazinylthio, wie 2-Niederalkyl-5,6-dihydroxy-1,2,4-triazin-3-ylthio, bzw. dazu tautomeres 1,2,5,6-Tetrahydro-2-niederalkyl-5,6-dioxo-1,2,4-triazin-3-ylthio, z.B. 1,2,5,6-Tetrahydro-2-methyl-5,6-dioxo-1,2,4-triazinyl-3-ylthio, und $R_4$ Carboxyl oder in physiologisch spaltbarer Form verestertes Carboxyl, z.B. Pivaloyloxymethoxycarbonyl, oder Phthalidyloxycarbonyl, bedeutet, und pharmazeutisch verwendbare Salze von solchen Verbindungen.

5. Verbindungen der Formel (I) nach Patentanspruch 1, worin Am Amino oder Methylamino, $R_1$ Wasserstoff, Methyl, 2-Carboxy-2-propyl oder 1-Carboxy-1-cyclopropyl, $R_2$ Wasserstoff oder Methoxy, $R_3$ Acetoxy, Carbamoyloxy, gegebenenfalls durch Niederalkyl oder Carboxyniederalkyl substituiertes Triazolylthio, wie 4-Carboxymethyl-1,2,4-triazolylthio, gegebenenfalls durch Niederalkyl, Carboxyniederalkyl, Sulfoniederalkyl oder Diniederalkylaminoniederalkyl substituiertes

BAD ORIGINAL

Tetrazolylthio, wie 1-Methyl-1H-tetrazol-5-ylthio, 1-Sulfomethyl-1H-tetrazol-5-ylthio, 1(2-Sulfoäthyl)-1H-tetrazol-5-ylthio, 1-Carboxymethyl-1H-tetrazol-5-ylthio, 1-(2-Dimethylaminoäthyl)-1H-tetrazol-5-ylthio, gegebenenfalls durch Niederalkyl substituiertes Thiadiazolylthio, wie 2-Methyl-1,3,4-thiadiazol-5-ylthio oder 3-Methyl-1,2,4-thiadiazol-5-ylthio, gegebenenfalls durch Hydroxy substituiertes Pyridazinylthio, wie 3-Hydroxy-pyridazin-6-ylthio, gegebenenfalls durch Hydroxy substituiertes Pyrimidinylthio, wie 4-Hydroxy-2-pyrimidinyl- bzw. das dazu tautomere 4-Oxo-3,4-dihydro-2-pyrimidinylthio, oder durch Niederalkyl und zwei Hydroxy substituiertes Triazinylthio, wie 2-Niederalkyl-5,6-dihydroxy-1,2,4-triazin-3-ylthio, und $R_4$ Carboxyl oder in physiologisch spaltbarer Form verestertes Carboxyl, z.B. Pivaloyloxymethoxycarbonyl, oder Phthalidyloxycarbonyl, bedeutet, sowie pharmazeutisch verwendbare Salze von solchen Verbindungen.

6. 7β-[2-(5-Amino-1,2,4-thiadiazol-3-yl)-2-Z-methoxyiminoacetamido]-3-(1,2,5,6-tetrahydro-2-methyl-5,6-dioxo-1,2,4-triazin-3-yl-thiomethyl)-3-cephem-4-carbonsäure, und pharmazeutisch annehmbare Salze davon, nach Patentanspruch 1.

7. 7β-[2-(5-Amino-1,2,4-thiadiazol-3-yl)-2-Z-hydroxyiminoacetamido]-3-(1-methyltetrazol-5-yl-thiomethyl)-3-cephem-4-carbonsäure, und pharmazeutisch annehmbare Salze davon, nach Patentanspruch 1.

8. 7β-[2-(5-Amino-1,2,4-thiadiazol-3-yl)-2-Z-methoxyiminoacetamido]-3-acetoxymethyl-3-cephem-4-carbonsäure-pivaloyloxymethylester, und pharmazeutisch annehmbare Salze davon, nach Patentanspruch 1.

BAD ORIGINAL

9. 7ß-[2-(5-Amino-1,2,4-thiadiazol-3-yl)-2-Z-methoxyiminoacetamido]-3-carbamoyloxymethyl-3-cephem-4-carbonsäure-pivaloyloxymethylester, und pharmazeutisch annehmbare Salze davon, nach Patentanspruch 1.

10. 7ß-[2-(5-Amino-1,2,4-thiadiazol-3-yl)-2-Z-methoxyiminoacetamido]-3-(1-methyltetrazol-5-yl-thiomethyl)-3-cephem-4-carbonsäure-pivaloyloxymethylester, und pharmazeutisch annehmbare Salze davon, nach Patentanspruch 1.

11. 7ß-[2-(5-Amino-1,2,4-thiadiazol-3-yl)-2-Z-methoxyiminoacetamido]-3-{1-[2-(N,N-dimethylamino)äthyl]-1H-tetrazol-5-yl-thiomethyl}-3-cephem-4-carbonsäure-pivaloyloxymethylester, und pharmazeutisch annehmbare Salze davon, nach Patentanspruch 1.

12. 7ß-[2-(5-Amino-1,2,4-thiadiazol-3-yl)-2-Z-methoxyiminoacetamido]-3-(1-pivaloyloxymethoxycarbonylmethyl-tetrazol-5-yl-thiomethyl-3-cephem-4-carbonsäure-pivaloyloxymethylester, und pharmazeutisch annehmbare Salze davon, nach Patentanspruch 1.

13. 7ß-[2-(5-Amino-1,2,4-thiadiazol-3-yl)-2-Z-methoxyiminoacetamido]-3-(4-oxo-3,4-dihydro-pyrimidin-2-yl-thiomethyl)-3-cephem-4-carbonsäure, und pharmazeutisch annehmbare Salze davon, nach Patentanspruch 1.

14. 7ß-[2-(5-N-Methylamino-1,2,4-thiadiazol-3-yl)-2-Z-methoxyiminoacetamido]-3-acetoxymethyl-3-cephem-4-carbonsäure und pharmazeutisch annehmbare Salze davon, nach Patentanspruch 1.

15. 7ß-[2-(5-Amino-1,2,4-thiadiazol-3-yl)-2-Z-methylcarbamoyloxyiminoacetamido]-3-acetoxymethyl-3-cephem-4-carbonsäure und pharmazeutisch annehmbare Salze davon, nach Patentanspruch 1.

BAD ORIGINAL

16. 7ß-[2-(5-Amino-1,2,4-thiadiazol-3-yl)-2-Z-methylcarbamoyloxyiminoacetamido]-3-(1-methyltetrazol-5-yl-thiomethyl)-3-cephem-4-carbonsäure und pharmazeutisch annehmbare Salze davon, nach Patentanspruch 1.

17. 7ß-[2-(5-Amino-1,2,4-thiadiazol-3-yl)-2-Z-methylcarbamoyloxyiminoacetamido]-3-(1-carboxymethyl-tetrazol-5-yl-thiomethyl)-3-cephem-4-carbonsäure und pharmazeutisch annehmbare Salze davon, nach Patentanspruch 1.

18. 7ß-[2-(5-Amino.1,2,4-thiadiazol-3-yl)-2-Z-hydroxyimino-acetamido]-3-(1-carboxymethyl-tetrazol-5-yl-thiomethyl)-3-cephem-4-carbonsäure und pharmazeutisch annehmbare Salze davon, nach Patentanspruch 1 .

19. 7ß-[2-(5-Amino-1,2,4-thiadiazol-3-yl)-2-Z-methylcarbamoyloxyiminoacetamido]-3-(1-sulfomethyl-tetrazol-5-yl-thiomethyl)-3-cephem-4-carbonsäure und pharmazeutisch annehmbare Salze davon, nach Patentanspruch 1.

20. 7ß-[2-(5-Amino-1,2,4-thiadiazol-3-yl)-2-Z-hydroxyimino-acetamido]-3-(1-sulfomethyl-tetrazol-5-yl-thiomethyl)-3-cephem-4-carbonsäure und pharmazeutisch annehmbare Salze davon, nach Patentanspruch 1.

21. 7ß-[2-(5-Amino-1,2,4-thiadiazol-3-yl)-2-Z-methylcarbamoyloxyiminoacetamido]-3-(1-methyltetrazol-5-yl-thiomethyl)-3-cephem-4-carbonsäure-pivaloyloxymethylester und pharmazeutisch annehmbare Salze davon, nach Patentanspruch 1.

22. 7ß-[2-(5-N-Methylamino-1,2,4-thiadiazol-3-yl)-2-Z-methoxyiminoacetamido]-3-(1-carboxymethyltetrazol-5-yl-thiomethyl)-3-cephem-4-carbonsäure und pharmazeutisch annehmbare Salze davon, nach Patentanspruch 1.

BAD ORIGINAL

23. 7β-[2-(5-N-Methylamino-1,2,4-thiadiazol-3-yl)-2-Z-methoxyiminoacetamido]-3-(1-methyltetrazol-5-yl-thiomethyl)-3-cephem-4-carbonsäure und pharmazeutisch annehmbare Salze davon, nach Patentanspruch 1.

24. 7β-[2-(5-Amino-1,2,4-thiadiazol-3-yl)-2-Z-(2-carboxyprop-2-yloxyimino)acetamido]-3-(1-methyltetrazol-5-ylthiomethyl)-3-cephem-4-carbonsäure und pharmazeutisch annehmbare Salze davon, nach Patentanspruch 1.

25. 7β-[2-(5-Amino-1,2,4-thiadiazol-3-yl)-2-Z-(2-carboxyprop-2-yloxyimino)acetamido]-3-[1-(2-N,N-dimethylaminoäthyl)tetrazol-5-ylthiomethyl]-3-cephem-4-carbonsäure und pharmazeutisch annehmbare Salze davon, nach Patentanspruch 1.

26. Pharmazeutische Präparate enthaltend eine der Verbindungen der Patentansprüche 1, 3, 5 oder 6 - 14.

27. Pharmazeutische Präparate enthaltend eine der Verbindungen der Patentansprüche 2, 4 oder 15 - 25.

28. Verfahren zur Herstellung von 7β-Aminothiadiazolylacetamido-3-cephem-4-carbonsäureverbindungen der Formel

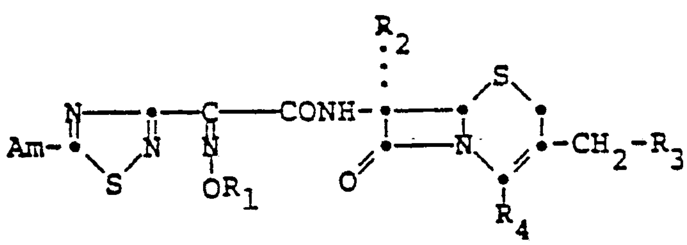

(I),

BAD ORIGINAL

worin Am eine gegebenenfalls geschützte Aminogruppe darstellt, $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, Acyloxy oder Heterocyclylthio steht, und $R_4$ eine gegebenenfalls geschützte Carboxylgruppe darstellt, und Salze von solchen Verbindungen, die eine salzbildende Gruppierung aufweisen, dadurch gekennzeichnet, dass man

a) in einer Verbindung der Formel

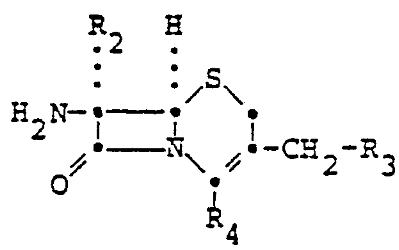

(II),

worin $R_2$, $R_3$ und $R_4$ die oben angegebenen Bedeutungen haben, und worin die 7ß-Aminogruppe gegebenenfalls durch eine die Acylierung erlaubende Gruppe substituiert ist, die 7ß-Aminogruppe durch Behandeln mit einem den Acylrest einer Carbonsäure der Formel

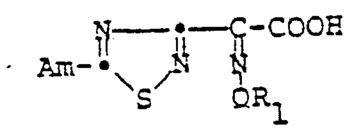

(III)

einführenden Acylierungsmittel, worin Am und $R_1$ die oben angegebenen Bedeutungen haben, acyliert oder

b) eine Verbindung der Formel

BAD ORIGINAL

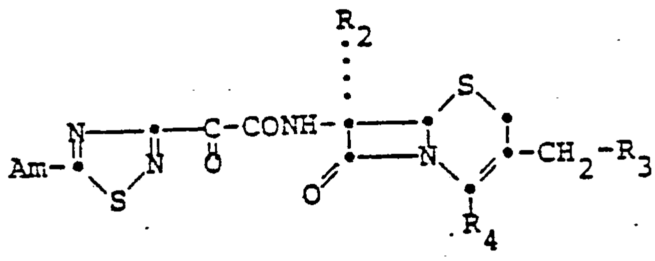

(IV)

worin Am, $R_2$, $R_3$ und $R_4$ die oben angegebenen Bedeutungen haben, mit einem Hydroxylamin der Formel $H_2N-O-R_1$ (V), worin $R_1$ die oben angegebene Bedeutung hat, umsetzt, oder

c) eine 2-Cephemverbindung der Formel

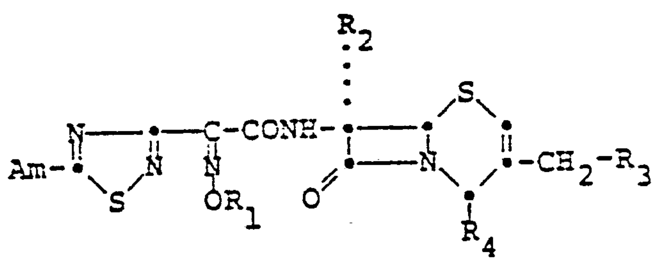

(VI),

worin Am, $R_1$, $R_2$, $R_3$ und $R_4$ die oben angegebenen Bedeutungen haben, isomerisiert, oder

d) zur Herstellung einer Verbindung der Formel (I), worin Am eine primäre oder sekundäre Aminogruppe bedeutet, eine Verbindung der Formel

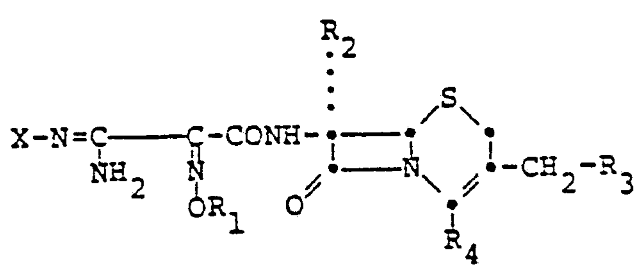

(VII),

BAD ORIGINAL

worin X Halogen oder Hydroxy bedeutet und $R_1$, $R_2$, $R_3$ und $R_4$ die oben angegebenen Bedeutungen haben, mit einem Salz der Rhodanwasserstoffsäure, bzw. mit einem Isorhodanwasserstoffsäure-ester, oder eine Verbindung der Formel (VII), worin X Wasserstoff ist, mit Rhodan behandelt, oder

e) zur Herstellung einer Verbindung der Formel (I), worin $R_1$ Wasserstoff bedeutet und worin Am, $R_2$, $R_3$ und $R_4$ die oben angegebenen Bedeutungen haben, eine Verbindung der Formel

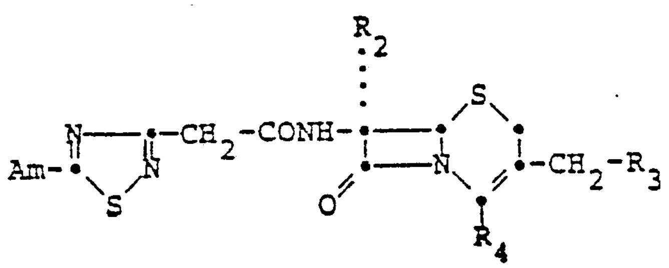

(VIII),

worin Am, $R_2$, $R_3$ und $R_4$ die oben angegebenen Bedeutungen haben, mit einem Nitrosierungsmittel behandelt, oder

f) zur Herstellung einer Verbindung der Formel I, worin $R_1$ gegebenenfalls substituiertes Niederalkyl oder Cycloalkyl, oder gegebenenfalls N-substituiertes Carbamoyl bedeutet und worin Am, $R_2$, $R_3$ und $R_4$ die oben angegebene Bedeutung haben, in einer Verbindung der Formel

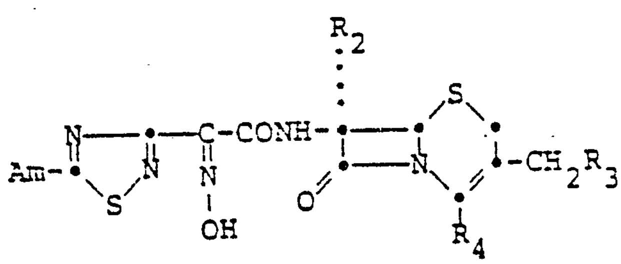

(If)

worin Am, $R_2$, $R_3$ und $R_4$ die oben gegebenen Bedeutungen haben, die Hydroxyiminogruppe mit einem den Rest $R_1$ einführenden Mittel behandelt, oder

BAD ORIGINAL

g) zur Herstellung einer Verbindung der Formel (I), worin $R_2$ Niederalkoxy bedeutet, und worin Am, $R_1$, $R_3$ und $R_4$ die oben angegebenen Bedeutungen haben, in eine Verbindung der Formel

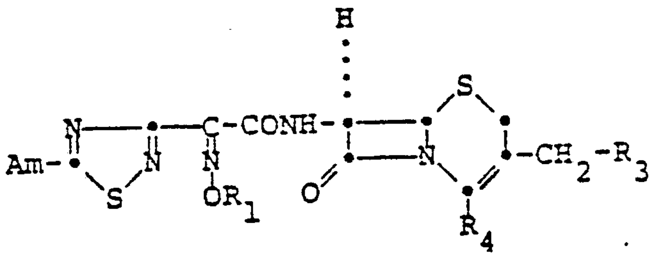

(Ig),

worin Am, $R_1$, $R_3$ und $R_4$ die oben gegebenen Bedeutungen haben, wobei funktionelle Gruppen in geschützter Form vorliegen, in α-Stellung eine Niederalkoxygruppe einführt, oder

h) zur Herstellung einer Verbindung der Formel (I), worin $R_3$ für Heterocyclythio steht und worin Am, $R_1$, $R_2$ und $R_4$ die oben gegebenen Bedeutungen haben, eine Verbindung der Formel

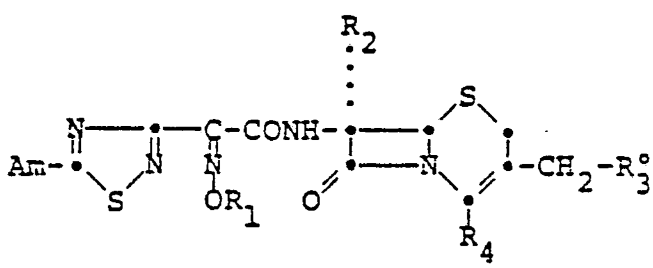

(Ih),

worin Am, $R_1$, $R_2$ und $R_4$ die oben gegebenen Bedeutungen haben, und $R_3^o$ einen durch einen Heterocyclylthiorest $R_3$ ersetzbaren Rest bedeutet, mit einem Mercaptan $H-R_3$, behandelt, oder

i) zur Herstellung einer Verbindung der Formel (I), worin $R_3$ für Acyloxy steht und worin Am, $R_1$, $R_2$ und $R_4$ die oben gegebenen Bedeutungen haben, eine Verbindung der Formel

BAD ORIGINAL

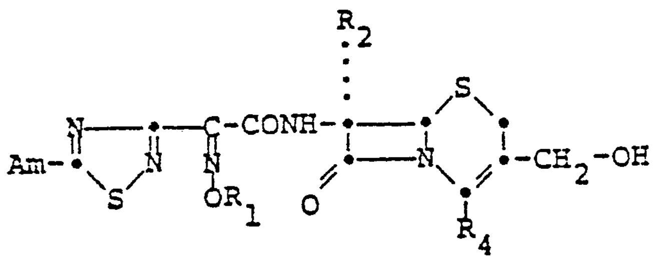

(Ii),

worin Am, $R_1$, $R_2$ und $R_4$ die oben gegebenen Bedeutungen haben, mit einem Acylierungsmittel behandelt, oder

j) zur Herstellung einer Verbindung der Formel (I), worin $R_4$ freies Carboxyl darstellt und worin Am, $R_1$, $R_2$ und $R_3$ die oben gegebenen Bedeutungen haben, in einer Verbindung der Formel

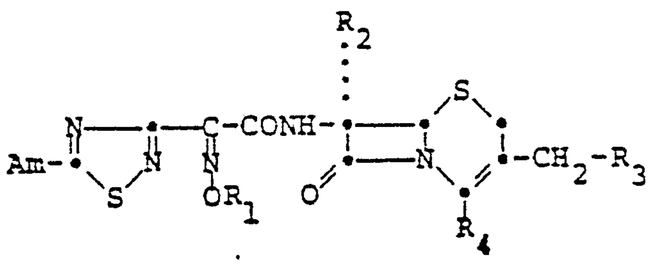

(Ij),

worin Am, $R_1$, $R_2$ und $R_3$ die oben gegebenen Bedeutungen haben, und $R_4$ eine geschützte Carboxylgruppe darstellt, die Carboxylschutzgruppe entfernt und durch Wasserstoff ersetzt, oder

k) zur Herstellung einer Verbindung der Formel (I), worin $R_4$ eine veresterte Carboxylgruppe darstellt und worin Am, $R_1$, $R_2$ und $R_3$ die oben gegebenen Bedeutungen haben, in einer Verbindung der Formel

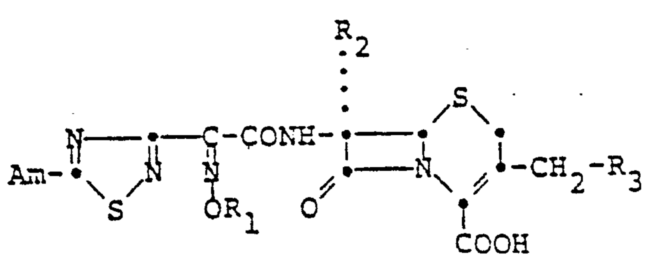

(Ik),

worin Am, $R_1$, $R_2$ und $R_3$ die oben gegebenen Bedeutungen haben, die 4-Carboxylgruppe verestert, oder

BAD ORIGINAL

l) zur Herstellung von Verbindungen der Formel (I), worin Am eine primäre, sekundäre oder tertiäre Aminogruppe ist, eine Verbindung der Formel

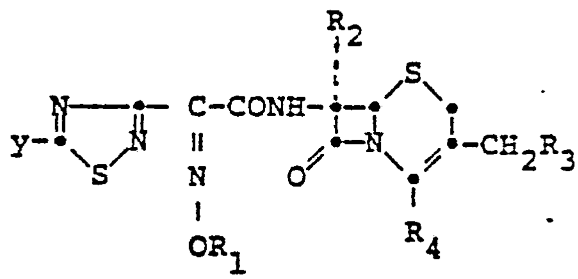
(XVIII)

worin y Halogen ist und $R_1$, $R_2$, $R_3$ und $R_4$ die oben angegebenen Bedeutungen haben, mit Ammoniak oder einem primären oder sekundären Amin-Am-H, oder einem Metallamid davon, umsetzt, und, wenn notwendig, in einer erhaltenen Verbindung nicht erwünschte Schutzgruppen abspaltet, und/oder, wenn erwünscht, eine erhaltene Verbindung mit salzbildender Gruppe in ein Salz, oder ein erhaltenes Salz in eine freie Verbindung oder in ein anderes Salz überführt.

29. Verfahren zur Herstellung von 7β-Aminothiadiazolylacetamido-3-cephem-4-carbonsäureverbindungen der Formel

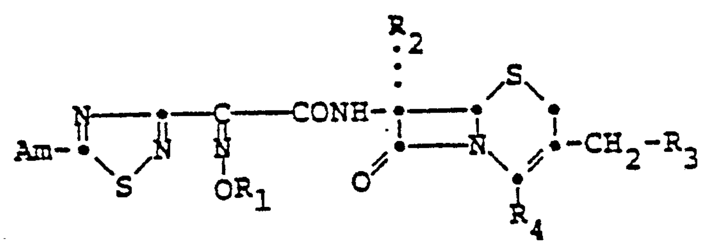
(I),

worin Am eine gegebenenfalls geschützte Aminogruppe darstellt, $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, Acyloxy oder Heterocyclylthio steht, und $R_4$ eine gegebenenfalls geschützte Carboxylgruppe darstellt, und Salze von solchen Verbindungen, die eine salzbildende Gruppierung aufweisen, dadurch gekennzeichnet, dass man

BAD ORIGINAL

a) in einer Verbindung der Formel

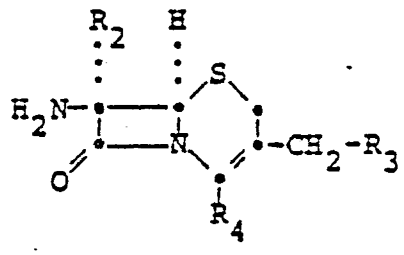 (II),

worin $R_2$, $R_3$ und $R_4$ die oben angegebenen Bedeutungen haben, und worin die 7ß-Aminogruppe gegebenenfalls durch eine die Acylierung erlaubende Gruppe substituiert ist, die 7ß-Aminogruppe durch Behandeln mit einem den Acylrest einer Carbonsäure der Formel

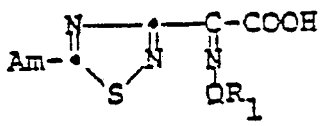 (III)

einführenden Acylierungsmittel, worin Am und $R_1$ die oben angegebenen Bedeutungen haben, acyliert oder

b) eine Verbindung der Formel

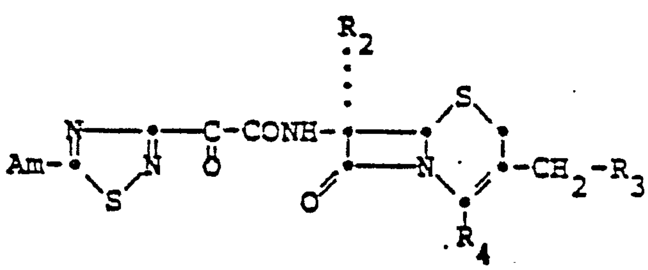 (IV)

worin Am, $R_2$, $R_3$ und $R_4$ die oben angegebenen Bedeutungen haben, mit einem Hydroxylamin der Formel $H_2N-O-R_1$ (V), worin $R_1$ die oben angegebene Bedeutung hat, umsetzt, oder

c) eine 2-Cephemverbindung der Formel

BAD ORIGINAL

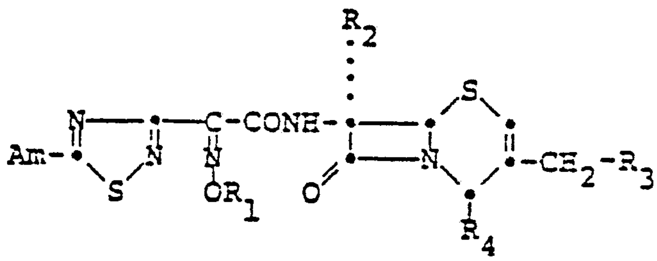

(VI),

worin Am, $R_1$, $R_2$, $R_3$ und $R_4$ die oben angegebenen Bedeutungen haben, isomerisiert, oder

d) Zur Herstellung einer Verbindung der Formel (I), worin Am eine primäre oder sekundäre Aminogruppe bedeutet, eine Verbindung der Formel

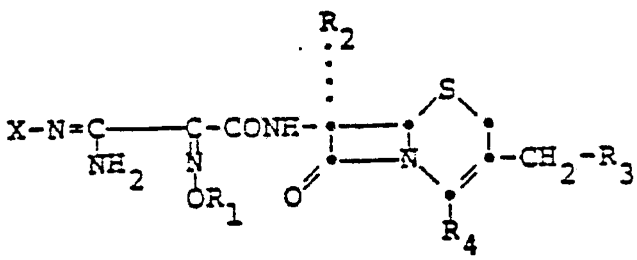

(VII),

worin X Halogen oder Hydroxy bedeutet und $R_1$, $R_2$, $R_3$ und $R_4$ die oben angegebenen Bedeutungen haben, mit einem Salz der Rhodanwasserstoffsäure, bzw. mit einem Isorhodanwasserstoffsäure-ester, oder eine Verbindung der Formel (VII), worin X Wasserstoff ist, mit Rhodan behandelt, oder

e) zur Herstellung einer Verbindung der Formel (I), worin $R_1$ Wasserstoff bedeutet und worin Am, $R_2$, $R_3$ und $R_4$ die oben angegebenen Bedeutungen haben, eine Verbindung der Formel

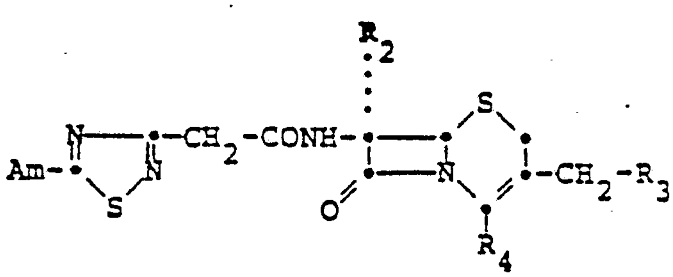

(VIII),

BAD ORIGINAL

worin Am, $R_2$, $R_3$ und $R_4$ die oben angegebenen Bedeutungen haben, mit einem Nitrosierungsmittel behandelt, oder

f) zur Herstellung einer Verbindung der Formel (I), worin $R_1$ gegebenenfalls substituiertes Niederalkyl bedeutet, und worin Am, $R_2$, $R_3$ und $R_4$ die oben angegebene Bedeutung haben, in einer Verbindung der Formel

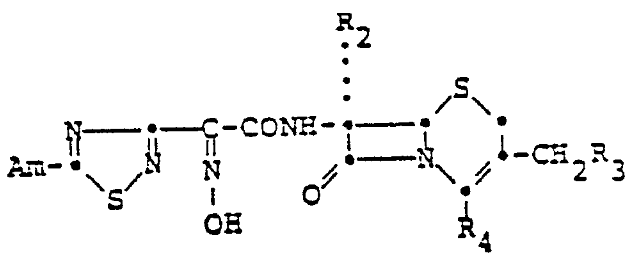

(If)

worin Am, $R_2$, $R_3$ und $R_4$ die oben angegebenen Bedeutungen haben, die Hydroxyiminogruppe mit einem den gegebenenfalls substituierten Niederalkylrest $R_1$ einführenden Alkylierungsmittel behandelt, oder

g) zur Herstellung einer Verbindung der Formel (I), worin $R_2$ Niederalkoxy bedeutet, und worin Am, $R_1$, $R_3$ und $R_4$ die oben gegebenen Bedeutungen haben, in eine Verbindung der Formel

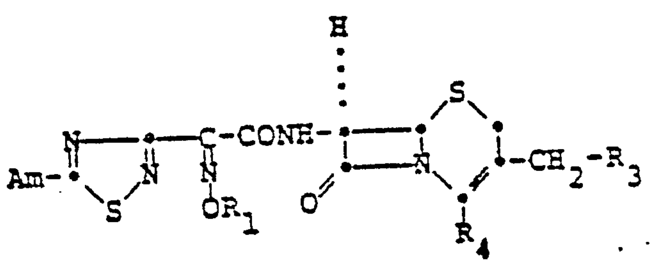

(Ig),

worin Am, $R_1$, $R_3$ und $R_4$ die oben gegebenen Bedeutungen haben, wobei funktionelle Gruppen in geschützter Form vorliegen, in 7α-Stellung eine Niederalkoxygruppe einführt, oder

BAD ORIGINAL

h) zur Herstellung einer Verbindung der Formel (I), worin $R_3$ für Heterocycylthio steht und worin Am, $R_1$, $R_2$ und $R_4$ die oben gegebenen Bedeutungen haben, eine Verbindung der Formel

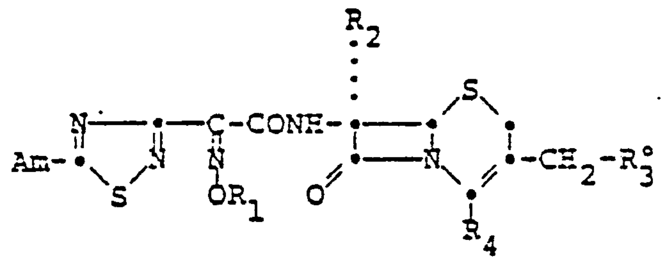

(Ih),

worin Am, $R_1$, $R_2$ und $R_4$ die oben gegebenen Bedeutungen haben, und $R_3^\circ$ einen durch einen Heterocyclylthiorest $R_3$ ersetzbaren Rest bedeutet, mit einem Mercaptan $H-R_3$, behandelt, oder

i) zur Herstellung einer Verbindung der Formel (I), worin $R_3$ für Acyloxy steht und worin Am, $R_1$, $R_2$ und $R_4$ die oben gegebenen Bedeutungen haben, eine Verbindung der Formel

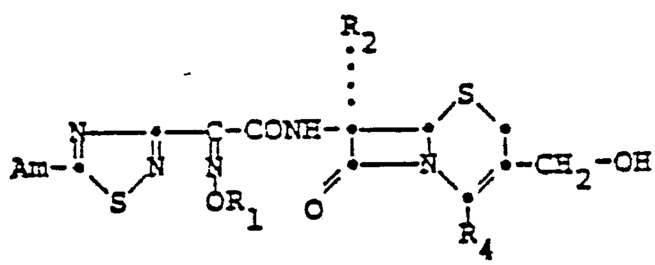

(Ii),

worin Am, $R_1$, $R_2$ und $R_4$ die oben gegebenen Bedeutungen haben, mit einem Acylierungsmittel behandelt, oder

j) zur Herstellung einer Verbindung der Formel (I), worin $R_4$ freies Carboxyl darstellt und worin Am, $R_1$, $R_2$ und $R_3$ die oben gegebenen Bedeutungen haben, in einer Verbindung Formel

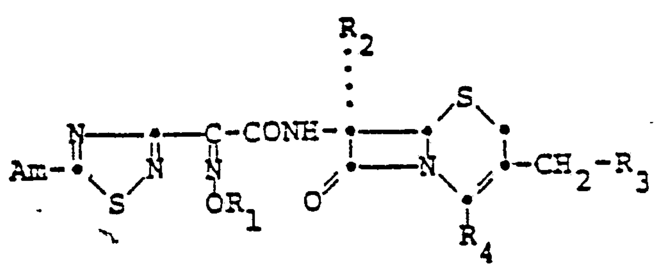

(Ij),

BAD ORIGINAL

worin Am, $R_1$, $R_2$ und $R_3$ die oben gegebenen Bedutungen haben und $R_4$ eine geschützte Carboxylgruppe darstellt, die Carboxylschutzgruppe entfernt und durch Wasserstoff ersetzt, oder

k) zur Herstellung einer Verbindung der Formel (I), worin $R_4$ eine veresterte Carboxylgruppe darstellt und worin Am, $R_1$, $R_2$ und $R_3$ die oben gegebenen Bedeutungen haben, in einer Verbindung der Formel

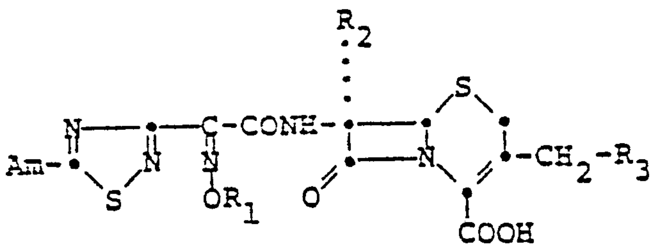

(Ik),

worin Am, $R_1$, $R_2$ und $R_3$ die oben gegebenen Bedeutungen haben, die 4-Carboxylgruppe verestert, oder

l) zur Herstellung von Verbindungen der Formel (I), worin Am eine primäre, sekundäre oder tertiäre Aminogruppe ist, eine Verbindung der Formel

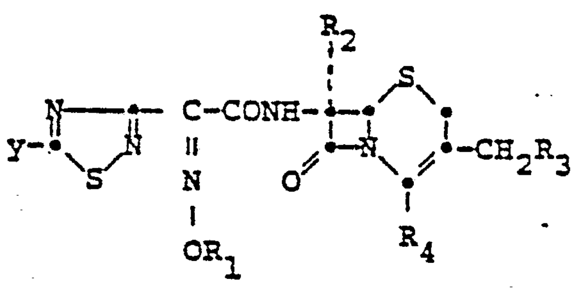

(XVIII)

worin y Halogen ist und $R_1$, $R_2$, $R_3$ und $R_4$ die oben angegebenen Bedeutungen haben, mit Ammoniak oder einem primären oder sekundären Amin Am-H, oder einem Metallamid davon, umsetzt, und, wenn notwendig, in einer erhaltenen Verbindung nicht erwünschte Schutzgruppen abspaltet, und/oder, wenn erwünscht, eine erhaltene Verbindung mit salzbildender Gruppe in ein Salz, oder ein erhaltenes Salz in eine freie Verbindung oder in ein anderes Salz überführt.

BAD ORIGINAL

30. Verfahren zur Herstellung von Verbindungen der Formel (I) nach Patentanspruch 47, worin Am eine gegebenenfalls geschützte Aminogruppe darstellt, $R_1$ für Wasserstoff oder Niederalkyl steht, $R_2$ Wasserstoff oder Niederalkyloxy bedeutet, $R_3$ für Wasserstoff, Acyloxy oder Heterocyclylthio steht, und $R_4$ eine gegebenenfalls geschützte Carboxylgruppe darstellt, und Salzen von solchen Verbindungen, die eine salzbildende Gruppe aufweisen, dadurch gekennzeichnet, dass man

a) in einer Verbindung der Formel

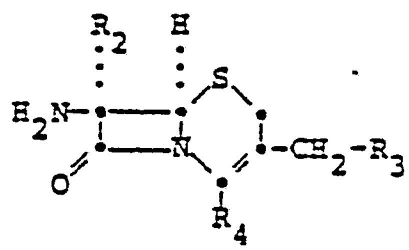

(II);

worin $R_2$, $R_3$ und $R_4$ die oben angegebenen Bedeutungen haben, und worin die 7β-Aminogruppe gegebenenfalls durch eine die Acylierung erlaubende Gruppe substituiert ist, die 7β-Aminogruppe durch Behandeln mit einem den Acylrest einer Carbonsäure der Formel

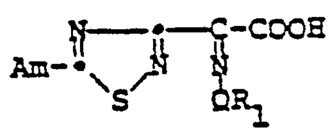

(III)

einführenden Acylierungsmittel, worin Am und $R_1$ die oben angegebenen Bedeutungen haben, acyliert oder

b) eine Verbindung der Formel

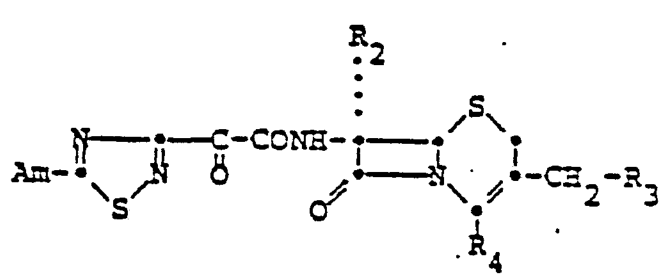

(IV)

BAD ORIGINAL

worin Am, $R_2$, $R_3$ und $R_4$ die oben angegebenen Bedeutungen haben, mit einem Hydroxylamin der Formel $H_2N-O-R_1$ (V), worin $R_1$ die oben angegebene Bedeutung hat, umsetzt, oder

c) eine 2-Cephemverbindung der Formel

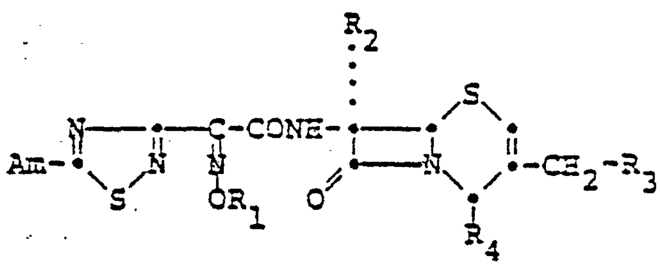

(VI),

worin Am, $R_1$, $R_2$, $R_3$ und $R_4$ die oben angegebenen Bedeutungen haben, isomerisiert, oder

d) zur Herstellung einer Verbindung der Formel (I), worin Am eine freie Aminogruppe bedeutet, eine Verbindung der Formel

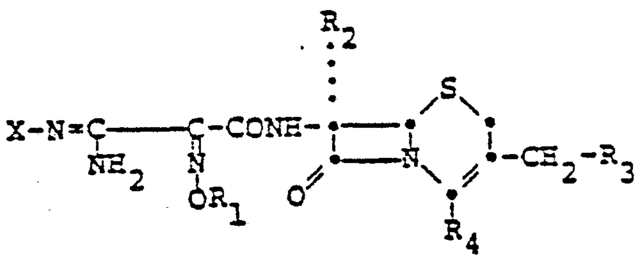

(VII),

worin X Halogen bedeutet und $R_1$, $R_2$, $R_3$ und $R_4$ die oben angegebenen Bedeutungen haben, mit einem Salz der Rhodanwasserstoffsäure, oder eine Verbindung der Formel (VII), worin X Wasserstoff ist, mit Rhodan behandelt, oder

e) zur Herstellung einer Verbindung der Formel (I), worin $R_1$ Wasserstoff bedeutet und worin Am, $R_2$, $R_3$ und $R_4$ die oben angegebenen Bedeutungen haben, eine Verbindung der Formel

BAD ORIGINAL

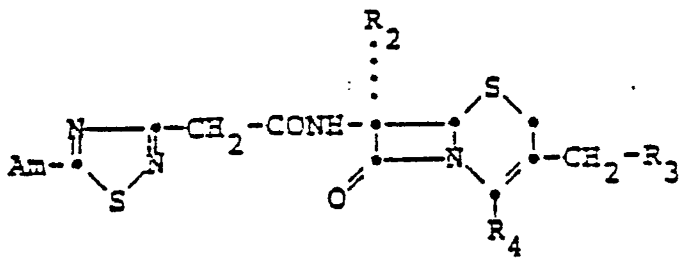

(VIII),

worin Am, $R_2$, $R_3$ und $R_4$ die oben angegebenen Bedeutungen haben, mit einem Nitrosierungsmittel behandelt, oder

f) zur Herstellung einer Verbindung der Formel (I), worin $R_1$ Niederalkyl bedeutet und worin Am, $R_2$, $R_3$ und $R_4$ die oben angegebenen Bedeutungen haben, in einer Verbindung der Formel

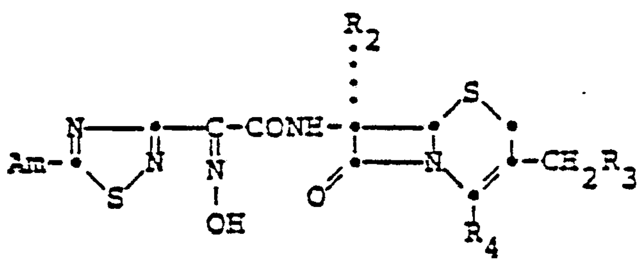

(If)

worin Am, $R_2$, $R_3$ und $R_4$ die oben gegebenen Bedeutungen haben, die Hydroxyiminogruppe mit einem den Niederalkylrest $R_1$ einführenden Alkylierungsmittel behandelt, oder

g) zur Herstellung einer Verbindung der Formel (I), worin $R_2$ Niederalkoxy bedeutet, und worin Am, $R_1$, $R_3$ und $R_4$ die oben gegebenen Bedeutungen haben, in eine Verbindung der Formel

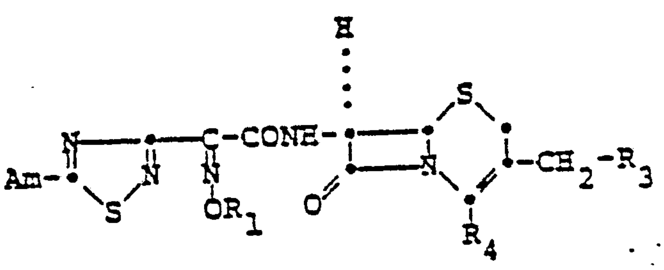

(Ig),

BAD ORIGINAL

worin Am, $R_1$, $R_3$ und $R_4$ die oben gegebenen Bedeutungen haben, wobei funktionelle Gruppen in geschützter Form vorliegen, in 7α-Stellung eine Niederalkoxygruppe einführt, oder

h) zur Herstellung einer Verbindung der Formel (I), worin $R_3$ für Heterocycylthio steht und worin Am, $R_1$, $R_2$ und $R_4$ die oben gegebenen Bedeutungen haben, eine Verbindung der Formel

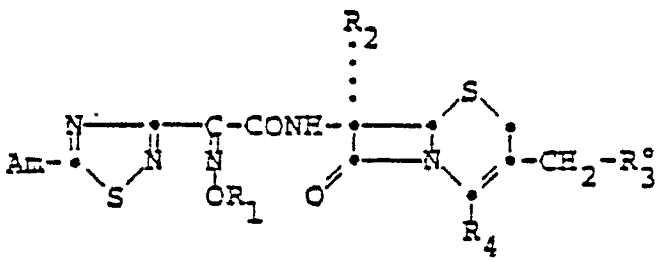

(Ih),

worin Am, $R_1$, $R_2$ und $R_4$ die oben gegebenen Bedeutungen haben, und $R_3^\circ$ einen durch eine Heterocyclylthiogruppe $R_3$ ersetzbaren Rest bedeutet, mit einem Mercaptan $H-R_3$, behandelt, oder

i) zur Herstellung einer Verbindung der Formel (I), worin $R_3$ für Acyloxy steht und worin Am, $R_1$, $R_2$ und $R_4$ die oben gegebenen Bedeutungen haben, eine Verbindung der Formel

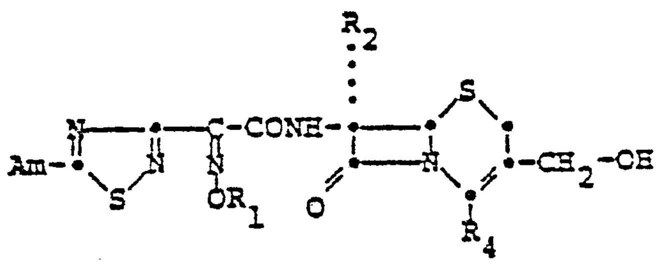

(Ii),

worin Am, $R_1$, $R_2$ und $R_4$ die oben gegebenen Bedeutungen haben, mit einem Acylierungsmittel behandelt, oder

j) zur Herstellung einer Verbindung der Formel (I), worin $R_4$ freies Carboxyl darstellt und worin Am, $R_1$, $R_2$ und $R_3$ die oben gegebenen Bedeutungen haben, in einer Verbindung der Formel

BAD ORIGINAL

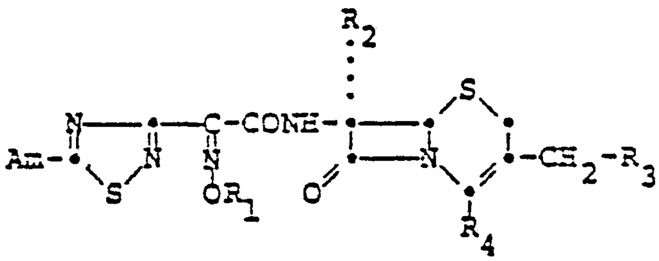

(Ij),

worin Am, $R_1$, $R_2$ und $R_3$ die oben gegebenen Bedeutungen haben und $R_4$ eine geschützte Carboxylgruppe darstellt, die Carboxylschutzgruppe entfernt und durch Wasserstoff ersetzt, oder

k) zur Herstellung einer Verbindung der Formel (I), worin $R_4$ eine veresterte Carboxylgruppe darstellt und worin Am, $R_1$, $R_2$ und $R_3$ die oben gegebenen Bedeutungen haben, in einer Verbindung der Formel

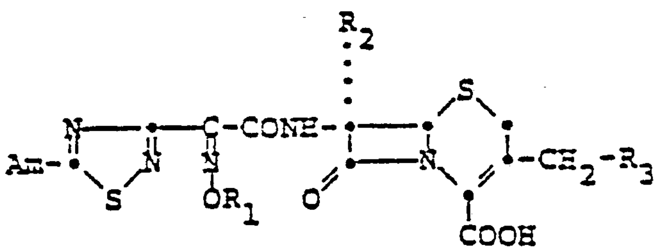

(Ik),

worin Am, $R_1$, $R_2$ und $R_3$ die oben gegebenen Bedeutungen haben, die 4-Carboxylgruppe verestert, und, wenn notwendig, in einer erhaltenen Verbindung nicht erwünschte Schutzgruppen abspaltet und/oder, wenn erwünscht, eine erhaltene Verbindung mit salzbildender Gruppe in ein Salz, oder ein erhaltenes Salz in eine freie Verbindung oder in ein anderes Salz überführt.

31. Verbindungen der Formel

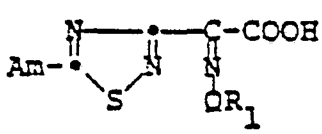

(III)

BAD ORIGINAL

worin Am eine gegebenenfalls geschützte Aminogruppe darstellt und $R_1$ für Wasserstoff, gegebenenfalls substituiertes Niederalkyl oder Cycloalkyl, oder gegebenenfalls N-substituiertes Carbamoyl steht, Estern davon und ihre zur Acylierung geeigneten Derivate.

32. Verfahren zur Herstellung von Verbindungen der Formel

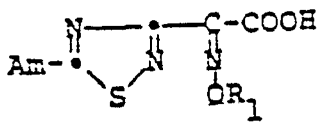

(III)

worin Am eine gegebenenfalls geschützte Aminogruppe darstellt und $R_1$ für Wasserstoff, gegebenenfalls substituiertes Niederalkyl oder Cycloalkyl, oder gegebenenfalls N-substituiertes Carbamoyl steht, Estern davon und ihrer zur Acylierung geeigneten Derivate, dadurch gekennzeichnet, dass man

a) eine Verbindung der Formel

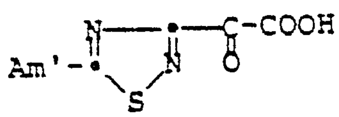

(XII),

worin Am' eine geschützte oder tertiäre Aminogruppe bedeutet, oder einen Ester davon mit einem Hydroxylamin der Formel $H_2N-O-R_1$ (V), worin $R_1$ die oben genannte Bedeutung hat, behandelt, oder

b) eine Verbindung der Formel

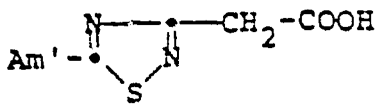

(XI),

BAD ORIGINAL

worin Am' eine geschützte oder tertiäre Aminogruppe bedeutet, oder einen Ester davon, nitrosiert oder

c) eine Verbindung der Formel

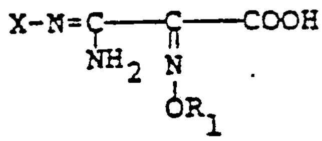

(XIII),

worin X Halogen oder Hydroxy bedeutet und $R_1$ die oben angegebene Bedeutung hat, oder einen Ester davon, mit einem Salz der Rhodanwasserstoffsäure, bzw. einem Isorhodanwasserstoffsäure-ester, oder eine Verbindung der Formel XIII, worin X Wasserstoff ist, mit Rhodan behandelt, oder

d) zur Herstellung einer Verbindung der Formel III, oder eines Esters davon, worin Am eine primäre, sekundäre oder tertiäre Aminogruppe bedeutet, eine Verbindung der Formel

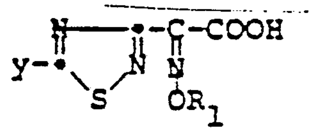

(XIX),

XIX

oder einen Ester davon, worin Y Halogen ist und $R_1$ die oben angegebene Bedeutung hat, mit Ammoniak oder einem primären oder sekundären Amin Am-H, oder einem Metallamid davon, umsetzt, oder

e) zur Herstellung einer Verbindung der Formel (III), worin $R_1$ für gegebenenfalls substituiertes Niederalkyl oder Cycloalkyl, oder für gegebenenfalls N-substituiertes Carbamoyl steht, eines Esters oder eines zur Acylierung geeigneten Derivates davon, eine Verbindung der Formel (III), worin $R_1$ Wasserstoff ist, oder einen Ester davon, mit einem den Rest $R_1$ einführenden Mittel behandelt, und gewünschtenfalls eine geschütz

BAD ORIGINAL

Aminogruppe in eine freie Aminogruppe oder in ein andersartig geschützte Aminogruppe überführt, und/oder eine Carbonsäure der Formel III in ein reaktionsfähiges Derivat davon überführt.

33. Verfahren zur Herstellung von Verbindungen der Formel

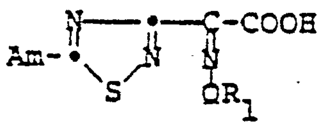

(III)

worin Am eine gegebenenfalls geschützte Aminogruppe darstellt und $R_1$ für Wasserstoff, gegebenenfalls substituiertes Niederalkyl oder Cycloalkyl, oder gegebenenfalls N-substituiertes Carbamoyl steht, Estern davon und ihrer zur Acylierung geeigneten Derivate, dadurch gekennzeichnet, dass man

a) eine Verbindung der Formel

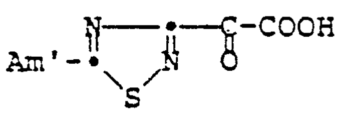

(XII),

worin Am' eine geschützte oder tertiäre Aminogruppe bedeutet, oder einen Ester davon mit einem Hydroxylamin der Formel $H_2N-O-R_1$ (V), worin $R_1$ die oben genannte Bedeutung hat, behandelt, oder

b) eine Verbindung der Formel

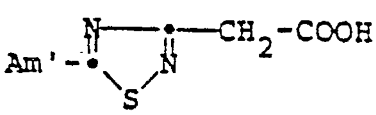

(XI),

worin Am' eine geschützte oder tertiäre Aminogruppe bedeutet, oder einen Ester davon, nitrosiert oder

BAD ORIGINAL

c) eine Verbindung der Formel

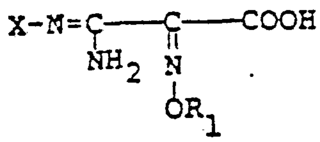

(XIII),

worin X Halogen oder Hydroxy bedeutet und $R_1$ die oben angegebene Bedeutung hat, oder einen Ester davon, mit einem Salz der Rhodanwasserstoffsäure, bzw. einem Isorhodanwasserstoffsäure-ester, oder eine Verbindung der Formel XIII, worin X Wasserstoff ist, mit Rhodan behandelt, oder

d) zur Herstellung einer Verbindung der Formel III,

oder eines Esters davon, worin Am eine primäre, sekundäre oder tertiäre Aminogruppe bedeutet, eine Verbindung der Formel

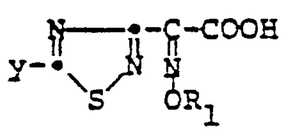

(XIX),

oder einen Ester davon, worin Y Halogen ist und $R_1$ die oben angegebene Bedeutung hat, mit Ammoniak oder einem primären oder sekundären Amin Am-H, oder einem Metallamid davon, umsetzt und gewünschtenfalls eine erhalten Verbindung der Formel III, worin $R_1$ Wasserstoff bedeutet, in eine Verbindung der Formel III überführt, worin $R_1$ gegebenenfalls substituiertes Niederalkyl bedeutet, und/oder eine geschützte Aminogruppe in eine freie Aminogruppe oder in eine andersartig geschützte Aminogruppe überführt, und/oder eine Carbonsäure der Formel III in ein reaktionsfähiges Derivat davon überführt.

34. Verfahren nach Patentanspruch 33 zur Herstellung von Verbindungen der Formel III, worin Am eine gegebenenfalls geschützte Aminogruppe darstellt und $R_1$ für Wasserstoff

BAD ORIGINAL

oder Niederalkyl steht, und ihre zur Acylierung geeigneten Derivate, dadurch gekennzeichnet, dass man

a) eine Verbindung der Formel

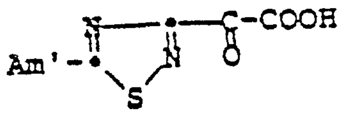

(XII),

worin Am' eine geschützte Aminogruppe bedeutet, mit einem Hydroxylamin der Formel $H_2N-O-R_1$ (V), worin $R_1$ die oben genannte Bedeutung hat, behandelt, oder

b) eine Verbindung der Formel

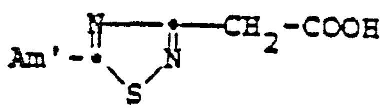

(XI),

worin Am' eine geschützte Aminogruppe bedeutet, nitrosiert, oder

c) eine Verbindung der Formel

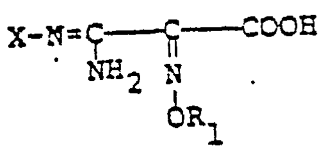

(XIII),

worin X Halogen bedeutet und $R_1$ die oben angegebene Bedeutung hat, mit einem Salz der Rhodanwasserstoffsäure, oder eine Verbindung der Formel XIII, worin X Wasserstoff ist, mit Rhodan behandelt, und gewünschtenfalls eine erhaltene Verbindung der Formel III, worin $R_1$ Wasserstoff bedeutet, in eine Verbindung der Formel III überführt, worin $R_1$ Niederalkyl bedeutet, und/oder eine geschützte Aminogruppe in eine freie Aminogruppe

BAD ORIGINAL

oder in eine andersartig geschützte Aminogruppe überführt, und/oder eine Carbonsäure der Formel III in ein reaktionsfähiges Derivat davon überführt.

35. Verbindungen der Formel

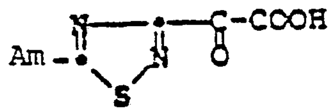

(XII),

worin Am eine gegebenenfalls geschützte Aminogruppe darstellt, Ester davon und ihre zur Acylierung geeigneten Derivate.

36. Verbindungen der Formel

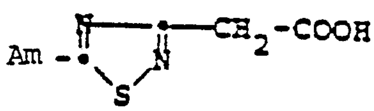

(XI),

worin Am eine gegebenenfalls geschützte Aminogruppe darstellt, Ester davon und ihre zur Acylierung geeigneten Derivate.

37. Verbindungen der Formel (XI) nach Patentanspruch 36, worin Am eine gegebenenfalls geschützte primäre Aminogruppe darstellt und ihre zur Acylierung geeigneten Derivate.

38. 7β-Aminothiadiazolylacetamido-3-cephem-4-carbonsäureverbindungen der Formel

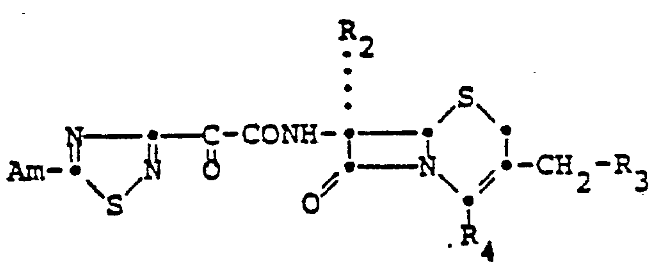

(IV)

BAD ORIGINAL

worin Am eine gegebenenfalls geschützte Aminogruppe darstellt, $R_2$ Wasserstoff oder Niederalkoxy bedeutet, $R_3$ für Wasserstoff, Acyloxy oder Heterocyclylthio steht, und $R_4$ eine gegebenenfalls geschützte Carboxylgruppe darstellt, und Salze von solchen Verbindungen, die eine salzbildende Gruppierung aufweisen.

39. 7β-Aminothiadiazolylacetamido-3-cephem-4-carbonsäureverbindungen der Formel

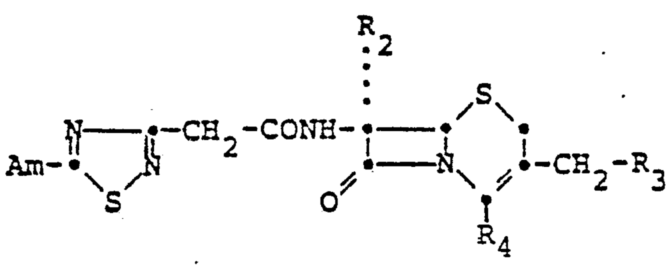 (VIII),

worin Am eine gegebenenfalls geschützte Aminogruppe darstellt, $R_2$ Wasserstoff oder Niederalkoxy bedeutet, $R_3$ für Wasserstoff, Acyloxy oder Heterocyclylthio steht, und $R_4$ eine gegebenenfalls geschützte Carboxylgruppe darstellt, und Salze von solchen Verbindungen, die eine salzbildende Gruppierung aufweisen.

BAD ORIGINAL

<u>Patentansprüche</u> (für Oesterreich)

1. Verfahren zur Herstellung von 7β-Aminothiadiazolyl-acetamido-3-cephem-4-carbonsäureverbindungen der Formel

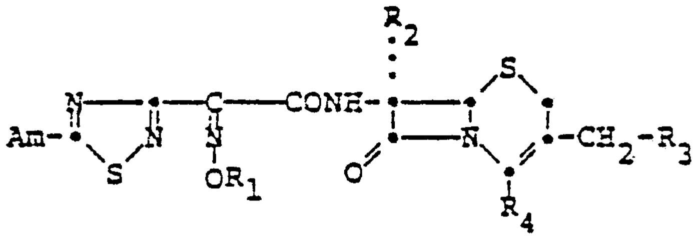

(I),

worin Am eine gegebenenfalls geschützte Aminogruppe darstellt, $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, Acyloxy oder Heterocyclylthio steht, und $R_4$ eine gegebenenfalls geschützte Carboxylgruppe darstellt, und Salze von solchen Verbindungen, die eine salzbildende Gruppierung aufweisen, dadurch gekennzeichnet, dass man

a) in einer Verbindung der Formel

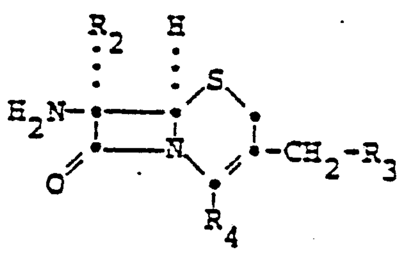

(II),

worin $R_2$, $R_3$ und $R_4$ die oben angegebenen Bedeutungen haben, und worin die 7β-Aminogruppe gegebenenfalls durch eine die Acylierung erlaubende Gruppe substituiert ist, die 7β-Aminogruppe durch Behandeln mit einem den Acylrest einer Carbonsäure der Formel

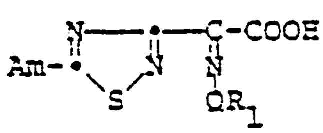

(III)

BAD ORIGINAL

einführenden Acylierungsmittel, worin Am und $R_1$ die oben angegebenen Bedeutungen haben, acyliert oder

b) eine Verbindung der Formel

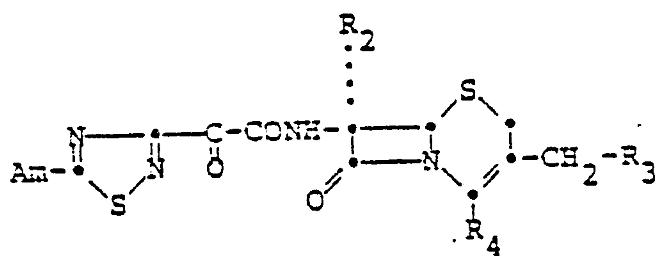

(IV)

worin Am, $R_2$, $R_3$ und $R_4$ die oben angegebenen Bedeutungen haben, mit einem Hydroxylamin der Formel $H_2N-O-R_1$ (V), worin $R_1$ die oben angegebene Bedeutung hat, umsetzt, oder

c) eine 2-Cephemverbindung der Formel

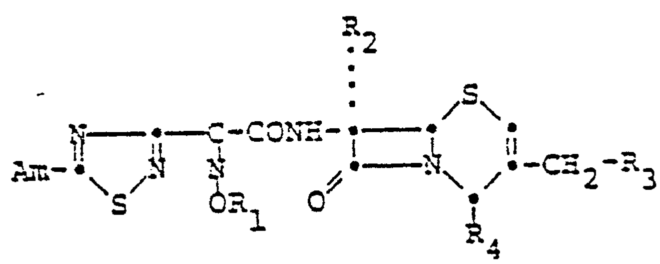

(VI),

worin Am, $R_1$, $R_2$, $R_3$ und $R_4$ die oben angegebenen Bedeutungen haben, isomerisiert, oder

d) zur Herstellung einer Verbindung der Formel (I), worin Am eine primäre oder sekundäre Aminogruppe bedeutet, eine Verbindung der Formel

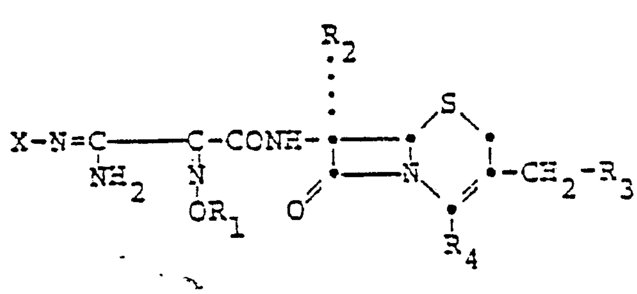

(VII),

BAD ORIGINAL

worin X Halogen oder Hydroxy bedeutet und $R_1$, $R_2$, $R_3$ und $R_4$ die oben angegebenen Bedeutungen haben, mit einem Salz der Rhodanwasserstoffsäure, bzw. mit einem Isorhodanwasserstoffsäure-ester, oder eine Verbindung der Formel (VII), worin X Wasserstoff ist, mit Rhodan behandelt, oder

e) zur Herstellung einer Verbindung der Formel (I), worin $R_1$ Wasserstoff bedeutet und worin Am, $R_2$, $R_3$ und $R_4$ die oben angegebenen Bedeutungen haben, eine Verbindung der Formel

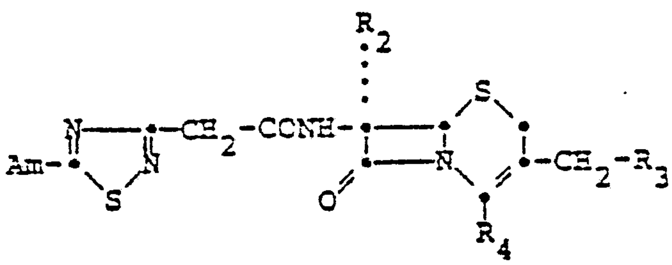

(VIII),

worin Am, $R_2$, $R_3$ und $R_4$ die oben angegebenen Bedeutungen habe mit einem Nitrosierungsmittel behandelt, oder

f) zur Herstellung einer Verbindung der Formel I, worin $R_1$ gegebenenfalls substituiertes Niederalkyl oder Cycloalkyl, oder gegebenenfalls N-substituiertes Carbamoyl bedeutet und worin Am, $R_2$, $R_3$ und $R_4$ die oben angegebene Bedeutung haben, in einer Verbindung der Formel

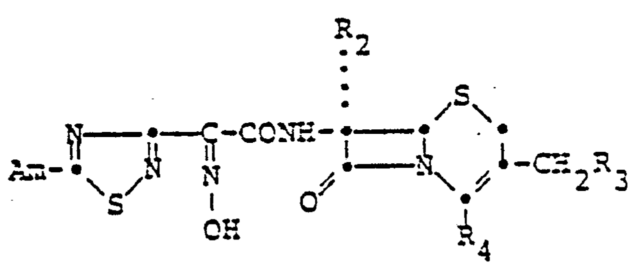

(If)

worin Am, $R_2$, $R_3$ und $R_4$ die oben gegebenen Bedeutungen haben, die Hydroxyiminogruppe mit einem den Rest $R_1$ einführenden Mittel behandelt, oder

BAD ORIGINAL

g) zur Herstellung einer Verbindung der Formel (I), worin $R_2$ Niederalkoxy bedeutet, und worin Am, $R_1$, $R_3$ und $R_4$ die oben angegebenen Bedeutungen haben, in eine Verbindung der Formel

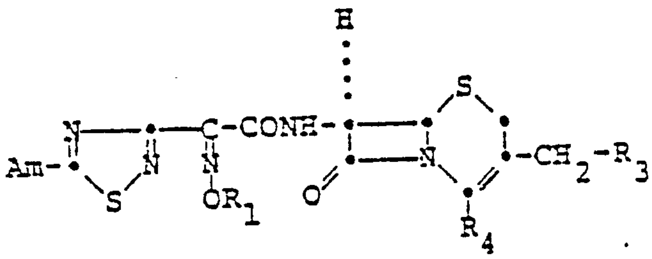

(Ig),

worin Am, $R_1$, $R_3$ und $R_4$ die oben gegebenen Bedeutungen haben, wobei funktionelle Gruppen in geschützter Form vorliegen, in α-Stellung eine Niederalkoxygruppe einführt, oder

h) zur Herstellung einer Verbindung der Formel (I), worin $R_3$ für Heterocyclythio steht und worin Am, $R_1$, $R_2$ und $R_4$ die oben gegebenen Bedeutungen haben, eine Verbindung der Formel

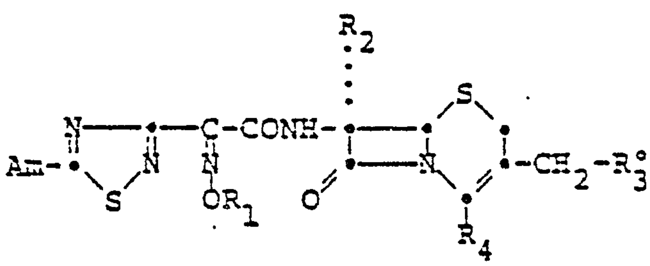

(Ih),

worin Am, $R_1$, $R_2$ und $R_4$ die oben gegebenen Bedeutungen haben, und $R_3^\circ$ einen durch einen Heterocyclylthiorest $R_3$ ersetzbaren Rest bedeutet, mit einem Mercaptan $H-R_3$, behandelt, oder

i) zur Herstellung einer Verbindung der Formel (I), worin $R_3$ für Acyloxy steht und worin Am, $R_1$, $R_2$ und $R_4$ die oben gegebenen Bedeutungen haben, eine Verbindung der Formel

BAD ORIGINAL

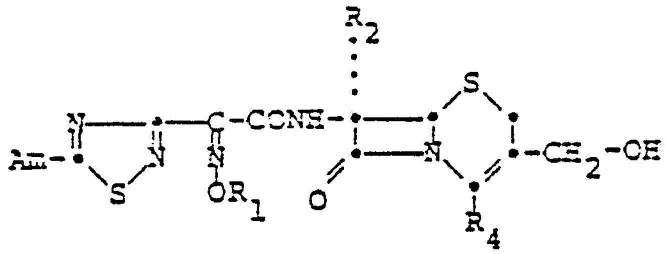

(Ii),

worin Am, $R_1$, $R_2$ und $R_4$ die oben gegebenen Bedeutungen haben, mit einem Acylierungsmittel behandelt, oder

j) zur Herstellung einer Verbindung der Formel (I), worin $R_4$ freies Carboxyl darstellt und worin Am, $R_1$, $R_2$ und $R_3$ die oben gegebenen Bedeutungen haben, in einer Verbindung der Formel

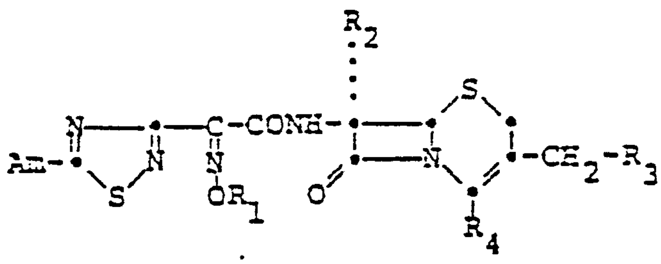

(Ij),

worin Am, $R_1$, $R_2$ und $R_3$ die oben gegebenen Bedeutungen haben, und $R_4$ eine geschützte Carboxylgruppe darstellt, die Carboxylschutzgruppe entfernt und durch Wasserstoff ersetzt, oder

k) zur Herstellung einer Verbindung der Formel (I), worin $R_4$ eine veresterte Carboxylgruppe darstellt und worin Am, $R_1$, $R_2$ und $R_3$ die oben gegebenen Bedeutungen haben, in einer Verbindung der Formel

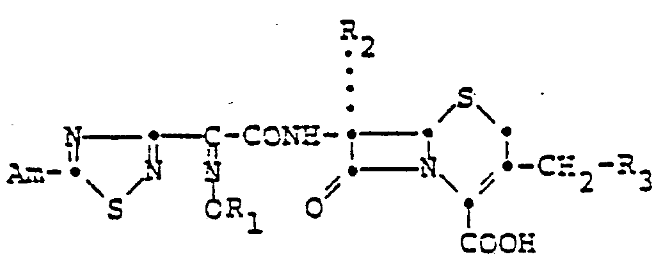

(Ik),

worin Am, $R_1$, $R_2$ und $R_3$ die oben gegebenen Bedeutungen haben, die 4-Carboxylgruppe verestert, oder

BAD ORIGINAL

1) zur Herstellung von Verbindungen der Formel (I), worin Am eine primäre, sekundäre oder tertiäre Aminogruppe ist, eine Verbindung der Formel

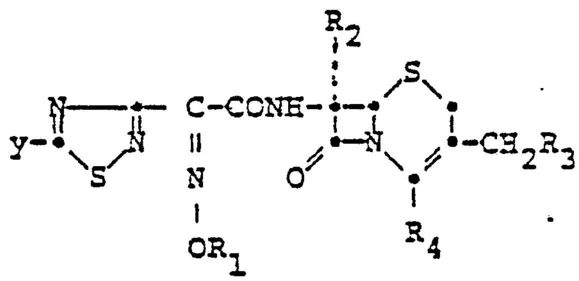

(XVIII)

worin y Halogen ist und $R_1$, $R_2$, $R_3$ und $R_4$ die oben angegebenen Bedeutungen haben, mit Ammoniak oder einem primären oder sekundären Amin ~~Am-~~H, oder einem Metallamid davon, umsetzt, und, wenn notwendig, in einer erhaltenen Verbindung nicht erwünschte Schutzgruppen abspaltet, und/oder, wenn erwünscht, eine erhaltene Verbindung mit salzbildender Gruppe in ein Salz, oder ein erhaltenes Salz in eine freie Verbindung oder in ein anderes Salz überführt.

2. Verfahren zur Herstellung von 7β-Aminothiadiazolyl-acetamido-3-cephem-4-carbonsäureverbindungen der Formel I nach Patentanspruch 1, worin Am eine gegebenenfalls geschützte Aminogruppe darstellt, $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, Acyloxy oder Heterocyclylthio steht, und $R_4$ eine gegebenenfalls geschützte Carboxylgruppe darstellt, und Salze von solchen Verbindungen, die eine salzbildende Gruppierung aufweisen, dadurch gekennzeichnet, dass man

BAD ORIGINAL

a) in einer Verbindung der Formel

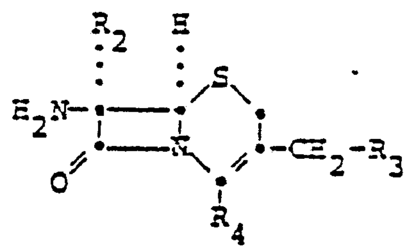

(II);

worin $R_2$, $R_3$ und $R_4$ die oben angegebenen Bedeutungen haben, und worin die 7ß-Aminogruppe gegebenenfalls durch eine die Acylierung erlaubende Gruppe substituiert ist, die 7ß-Aminogruppe durch Behandeln mit einem den Acylrest einer Carbonsäure der Formel

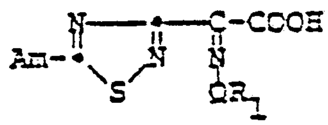

(III)

einführenden Acylierungsmittel, worin Am und $R_1$ die oben angegebenen Bedeutungen haben, acyliert oder

b) eine Verbindung der Formel

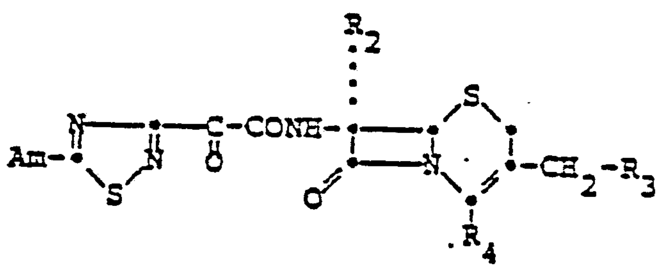

(IV)

worin Am, $R_2$, $R_3$ und $R_4$ die oben angegebenen Bedeutungen haben, mit einem Hydroxylamin der Formel $H_2N-O-R_1$ (V), worin $R_1$ die oben angegebene Bedeutung hat, umsetzt, oder

c) eine 2-Cephemverbindung der Formel

BAD ORIGINAL

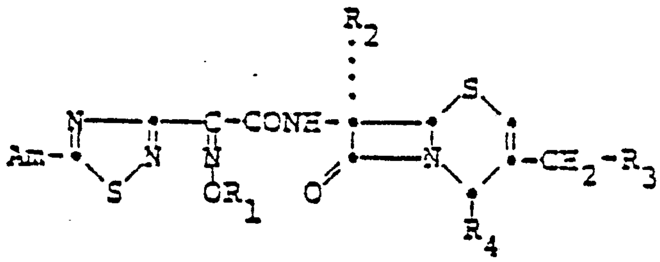

(VI),

worin Am, $R_1$, $R_2$, $R_3$ und $R_4$ die oben angegebenen Bedeutungen haben, isomerisiert, oder

d) Zur Herstellung einer Verbindung der Formel (I), worin Am eine primäre oder sekundäre Aminogruppe bedeutet, eine Verbindung der Formel

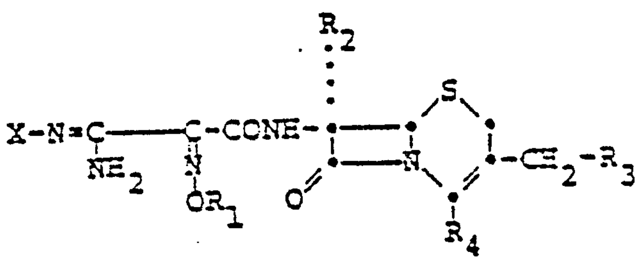

(VII),

worin X Halogen oder Hydroxy bedeutet und $R_1$, $R_2$, $R_3$ und $R_4$ die oben angegebenen Bedeutungen haben, mit einem Salz der Rhodanwasserstoffsäure, bzw. mit einem Isorhodanwasserstoffsäure-ester, oder eine Verbindung der Formel (VII), worin X Wasserstoff ist, mit Rhodan behandelt, oder

e) zur Herstellung einer Verbindung der Formel (I), worin $R_1$ Wasserstoff bedeutet und worin Am, $R_2$, $R_3$ und $R_4$ die oben angegebenen Bedeutungen haben, eine Verbindung der Formel

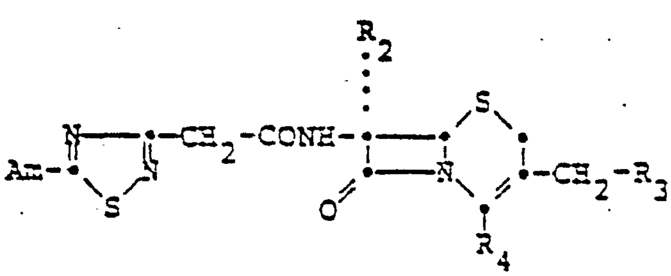

(VIII),

BAD ORIGINAL

worin Am, $R_2$, $R_3$ und $R_4$ die oben angegebenen Bedeutungen haben, mit einem Nitrosierungsmittel behandelt, oder

f) zur Herstellung einer Verbindung der Formel (I), worin $R_1$ gegebenenfalls substituiertes Niederalkyl bedeutet, und worin Am, $R_2$, $R_3$ und $R_4$ die oben angegebene Bedeutung haben, in einer Verbindung der Formel

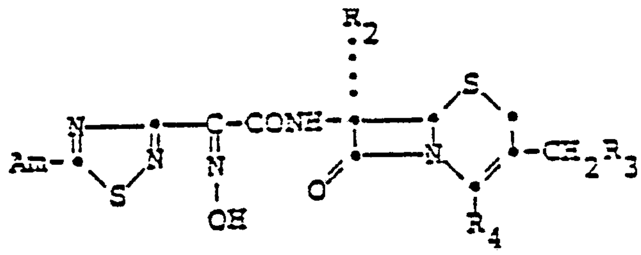

(If)

worin Am, $R_2$, $R_3$ und $R_4$ die oben angegebenen Bedeutungen haben, die Hydroxyiminogruppe mit einem den gegebenenfalls substituierten Niederalkylrest $R_1$ einführenden Alkylierungsmittel behandelt, oder

g) zur Herstellung einer Verbindung der Formel (I), worin $R_2$ Niederalkoxy bedeutet, und worin Am, $R_1$, $R_3$ und $R_4$ die oben gegebenen Bedeutungen haben, in eine Verbindung der Formel

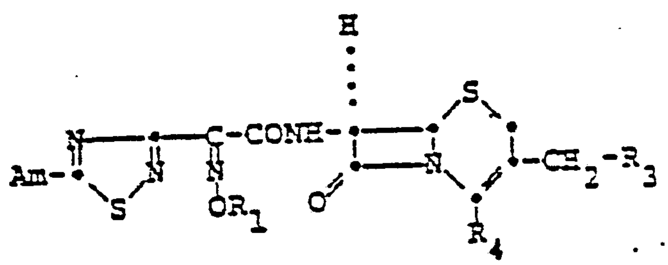

(Ig),

worin Am, $R_1$, $R_3$ und $R_4$ die oben gegebenen Bedeutungen haben, wobei funktionelle Gruppen in geschützter Form vorliegen, in 7α-Stellung eine Niederalkoxygruppe einführt, oder

BAD ORIGINAL

h) zur Herstellung einer Verbindung der Formel (I), worin $R_3$ für Heterocyclthio steht und worin Am, $R_1$, $R_2$ und $R_4$ die oben gegebenen Bedeutungen haben, eine Verbindung der Formel

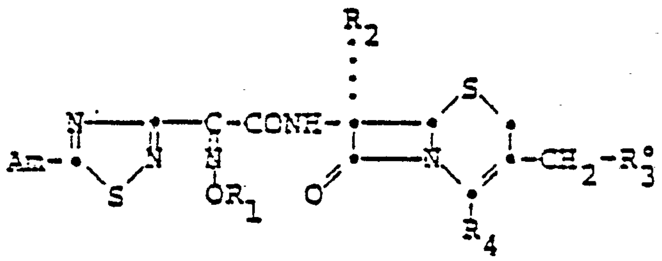

(Ih),

worin Am, $R_1$, $R_2$ und $R_4$ die oben gegebenen Bedeutungen haben, und $R_3^\circ$ einen durch einen Heterocyclylthiorest $R_3$ ersetzbaren Rest bedeutet, mit einem Mercaptan $H-R_3$, behandelt, oder

i) zur Herstellung einer Verbindung der Formel (I), worin $R_3$ für Acyloxy steht und worin Am, $R_1$, $R_2$ und $R_4$ die oben gegebenen Bedeutungen haben, eine Verbindung der Formel

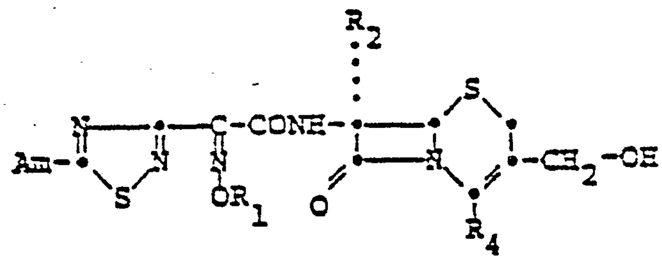

(Ii),

worin Am, $R_1$, $R_2$ und $R_4$ die oben gegebenen Bedeutungen haben, mit einem Acylierungsmittel behandelt, oder

j) zur Herstellung einer Verbindung der Formel (I), worin $R_4$ freies Carboxyl darstellt und worin Am, $R_1$, $R_2$ und $R_3$ die oben gegebenen Bedeutungen haben, in einer Verbindung der Formel

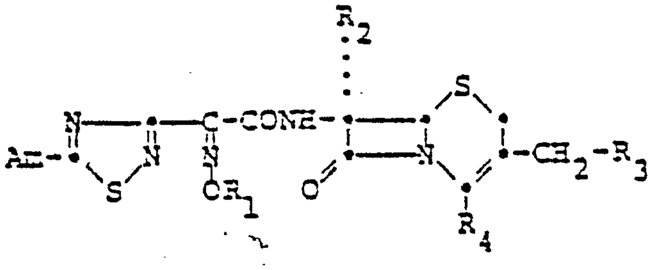

(Ij),

BAD ORIGINAL

worin Am, $R_1$, $R_2$ und $R_3$ die oben gegebenen Bedutungen haben und $R_4$ eine geschützte Carboxylgruppe darstellt, die Carboxylschutzgruppe entfernt und durch Wasserstoff ersetzt, oder

k) zur Herstellung einer Verbindung der Formel (I), worin $R_4$ eine veresterte Carboxylgruppe darstellt und worin Am, $R_1$, $R_2$ und $R_3$ die oben gegebenen Bedeutungen haben, in einer Verbindung der Formel

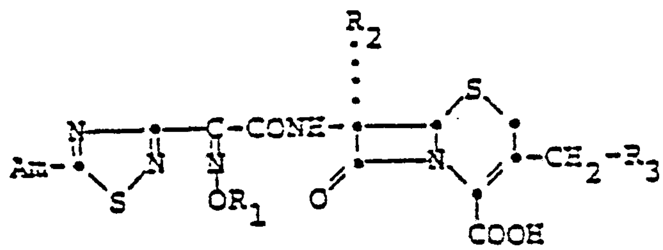

(Ik),

worin Am, $R_1$, $R_2$ und $R_3$ die oben gegebenen Bedeutungen haben, die 4-Carboxylgruppe verestert, oder

l) zur Herstellung von Verbindungen der Formel (I), worin Am eine primäre, sekundäre oder tertiäre Aminogruppe ist, eine Verbindung der Formel

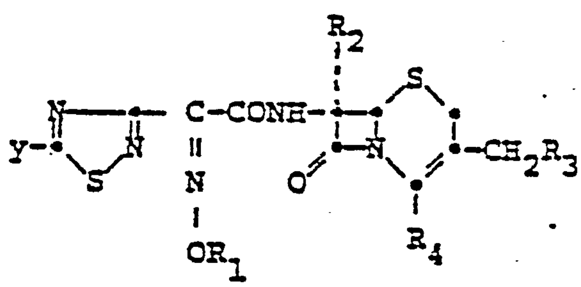

(XVIII)

worin y Halogen ist und $R_1$, $R_2$, $R_3$ und $R_4$ die oben angegebenen Bedeutungen haben, mit Ammoniak oder einem primären oder sekundären Amin Am-H, oder einem Metallamid davon, umsetzt, und, wenn notwendig, in einer erhaltenen Verbindung nicht erwünschte Schutzgruppen abspaltet, und/oder, wenn erwünscht, eine erhaltene Verbindung mit salzbildender Gruppe in ein Salz, oder ein erhaltenes Salz in eine freie Verbindung oder in ein anderes Salz überführt.

BAD ORIGINAL

3. Verfahren zur Herstellung von Verbindungen der Formel (I) nach Patentanspruch 1, worin Am eine gegebenenfalls geschützte Aminogruppe darstellt, $R_1$ für Wasserstoff oder Niederalkyl steht, $R_2$ Wasserstoff oder Niederalkyloxy bedeutet, $R_3$ für Wasserstoff, Acyloxy oder Heterocyclylthio steht, und $R_4$ eine gegebenenfalls geschützte Carboxylgruppe darstellt, und Salzen von solchen Verbindungen, die eine salzbildende Gruppe aufweisen, dadurch gekennzeichnet, dass man

a) in einer Verbindung der Formel

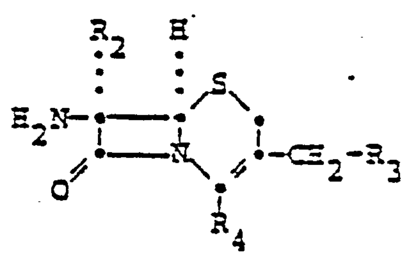

(II);

worin $R_2$, $R_3$ und $R_4$ die oben angegebenen Bedeutungen haben, und worin die 7β-Aminogruppe gegebenenfalls durch eine die Acylierung erlaubende Gruppe substituiert ist, die 7β-Aminogruppe durch Behandeln mit einem den Acylrest einer Carbonsäure der Formel

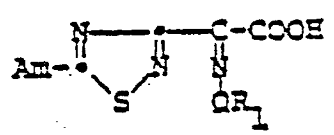

(III)

einführenden Acylierungsmittel, worin Am und $R_1$ die oben angegebenen Bedeutungen haben, acyliert oder

b) eine Verbindung der Formel

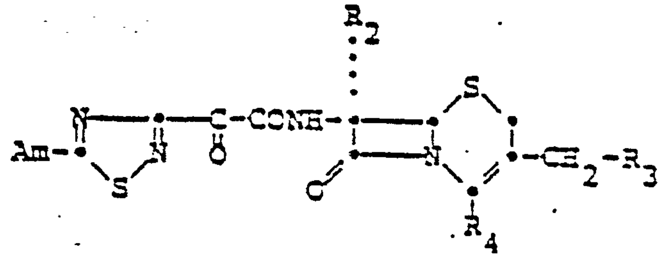

(IV)

worin Am, $R_2$, $R_3$ und $R_4$ die oben angegebenen Bedeutungen haben, mit einem Hydroxylamin der Formel $H_2N-O-R_1$ (V), worin $R_1$ die oben angegebene Bedeutung hat, umsetzt, oder

c) eine 2-Cephemverbindung der Formel

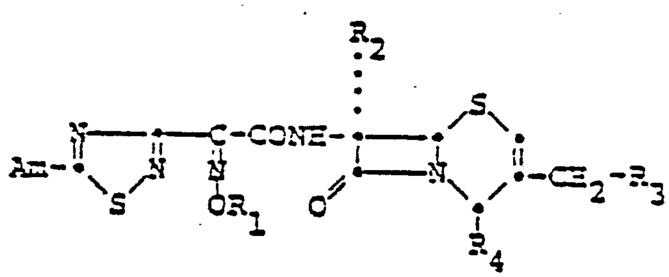

(VI),

worin Am, $R_1$, $R_2$, $R_3$ und $R_4$ die oben angegebenen Bedeutungen haben, isomerisiert, oder

d) zur Herstellung einer Verbindung der Formel (I), worin Am eine freie Aminogruppe bedeutet, eine Verbindung der Formel

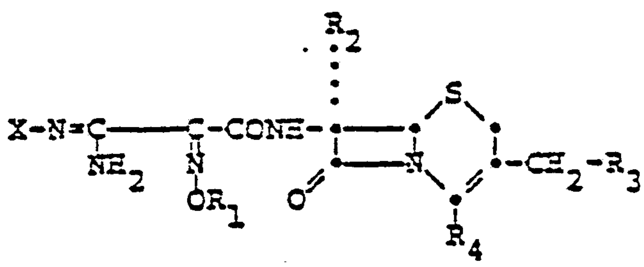

(VII),

worin X Halogen bedeutet und $R_1$, $R_2$, $R_3$ und $R_4$ die oben angegebenen Bedeutungen haben, mit einem Salz der Rhodanwasserstoffsäure, oder eine Verbindung der Formel (VII), worin X Wasserstoff ist, mit Rhodan behandelt, oder

e) zur Herstellung einer Verbindung der Formel (I), worin $R_1$ Wasserstoff bedeutet und worin Am, $R_2$, $R_3$ und $R_4$ die oben angegebenen Bedeutungen haben, eine Verbindung der Formel

BAD ORIGINAL

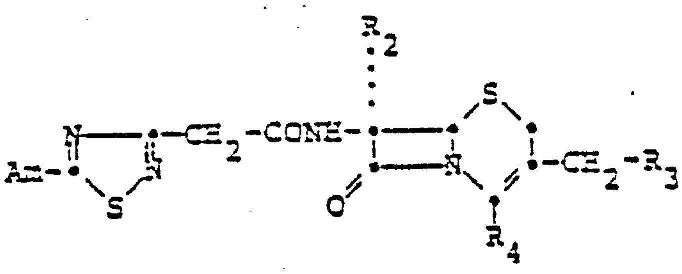

(VIII),

worin Am, $R_2$, $R_3$ und $R_4$ die oben angegebenen Bedeutungen haben, mit einem Nitrosierungsmittel behandelt, oder

f) zur Herstellung einer Verbindung der Formel (I), worin $R_1$ Niederalkyl bedeutet und worin Am, $R_2$, $R_3$ und $R_4$ die oben angegebenen Bedeutungen haben, in einer Verbindung der Formel

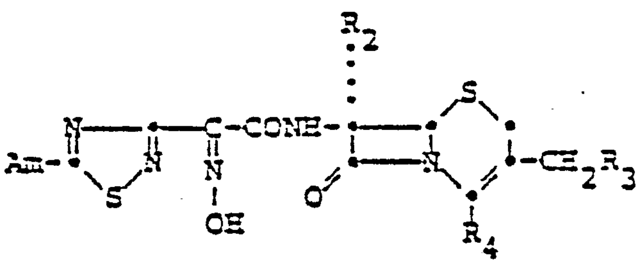

(If)

worin Am, $R_2$, $R_3$ und $R_4$ die oben gegebenen Bedeutungen haben, die Hydroxyiminogruppe mit einem den Niederalkylrest $R_1$ einführenden Alkylierungsmittel behandelt, oder

g) zur Herstellung einer Verbindung der Formel (I), worin $R_2$ Niederalkoxy bedeutet, und worin Am, $R_1$, $R_3$ und $R_4$ die oben gegebenen Bedeutungen haben, in eine Verbindung der Formel

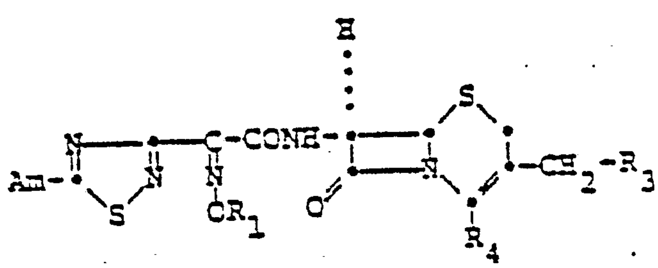

(Ig),

BAD ORIGINAL

worin Am, $R_1$, $R_3$ und $R_4$ die oben gegebenen Bedeutungen haben, wobei funktionelle Gruppen in geschützter Form vorliegen, in 7α-Stellung eine Niederalkoxygruppe einführt, oder

h) zur Herstellung einer Verbindung der Formel (I), worin $R_3$ für Heterocyclylthio steht und worin Am, $R_1$, $R_2$ und $R_4$ die oben gegebenen Bedeutungen haben, eine Verbindung der Formel

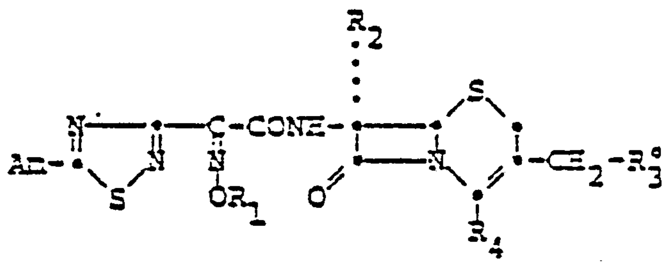

(Ih),

worin Am, $R_1$, $R_2$ und $R_4$ die oben gegebenen Bedeutungen haben, und $R_3^o$ einen durch eine Heterocyclylthiogruppe $R_3$ ersetzbaren Rest bedeutet, mit einem Mercaptan $H-R_3$, behandelt, oder

i) zur Herstellung einer Verbindung der Formel (I), worin $R_3$ für Acyloxy steht und worin Am, $R_1$, $R_2$ und $R_4$ die oben gegebenen Bedeutungen haben, eine Verbindung der Formel

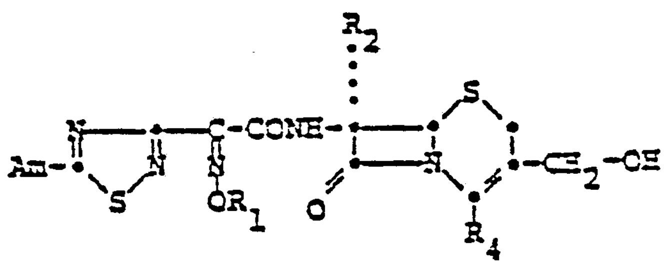

(Ii),

worin Am, $R_1$, $R_2$ und $R_4$ die oben gegebenen Bedeutungen haben, mit einem Acylierungsmittel behandelt, oder

j) zur Herstellung einer Verbindung der Formel (I), worin $R_4$ freies Carboxyl darstellt und worin Am, $R_1$, $R_2$ und $R_3$ die oben gegebenen Bedeutungen haben, in einer Verbindung der Formel

BAD ORIGINAL

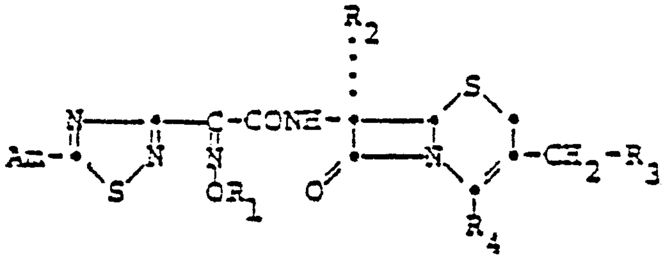 (Ij),

worin Am, $R_1$, $R_2$ und $R_3$ die oben gegebenen Bedeutungen haben und $R_4$ eine geschützte Carboxylgruppe darstellt, die Carboxylschutzgruppe entfernt und durch Wasserstoff ersetzt, oder

k) zur Herstellung einer Verbindung der Formel (I), worin $R_4$ eine veresterte Carboxylgruppe darstellt und worin Am, $R_1$, $R_2$ und $R_3$ die oben gegebenen Bedeutungen haben, in einer Verbindung der Formel

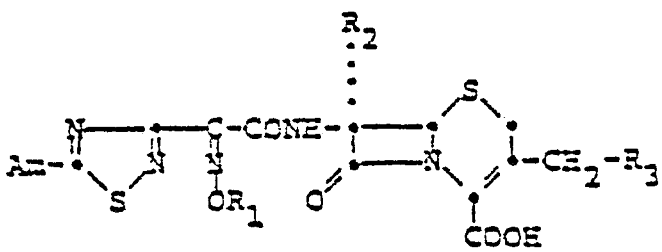 (Ik),

worin Am, $R_1$, $R_2$ und $R_3$ die oben gegebenen Bedeutungen haben, die 4-Carboxylgruppe verestert, und, wenn notwendig, in einer erhaltenen Verbindung nicht erwünschte Schutzgruppen abspaltet, und/oder, wenn erwünscht, eine erhaltene Verbindung mit salzbildender Gruppe in ein Salz, oder ein erhaltenes Salz in eine freie Verbindung oder in ein anderes Salz überführt.

4. Verfahren nach Patentanspruch 1 zur Herstellung von Verbindungen der Formel (I), worin Am Amino oder Methylamino, $R_1$ Wasserstoff, Niederalkyl, insbesondere Methyl, durch Carboxy substituiertes Niederalkyl oder Cycloalkyl, insbesondere 2-Carboxy-2-propyl oder 1-Carboxy-1-cyclopropyl, oder eine Gruppe -C(=O)-NHR, worin R Wasserstoff oder Nie-

BAD ORIGINAL

deralkyl, insbesondere Methyl, bedeutet, $R_2$ Wasserstoff oder Methoxy, $R_3$ Niederalkanoyloxy, wie Acetoxy, Carbamoyloxy, N-Niederalkylcarbamoyloxy, oder gegebenenfalls substituiertes, mit einem Ringkohlenstoffatom an die Thiogruppe gebundenes Heterocyclylthio, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell hydriert und gegebenenfalls beispielsweise durch Niederalkyl, wie Methyl, N,N-Diniederalkylaminoniederalkyl, wie 2-N,N-Dimethylaminoäthyl, Carboxyniederalkyl, wie Carboxymethyl oder 2-Carboxyäthyl, Sulfoniederalkyl, wie Sulfomethyl oder 2-Sulfoäthyl, Amino, Carboxyniederalkanoylamino, Carbamoyl, Oxo oder Hydroxy substituiert sein können, und $R_4$ Carboxyl oder in physiologisch spaltbarer Form verestertes Carboxyl, z.B. Acyloxymethoxycarbonyl, darstellt und pharmazeutisch verwendbaren Salzen von solchen Verbindungen.

5. Verfahren nach Patentanspruch 2 zur Herstellung von Verbindungen der Formel (I), worin Am Amino oder Methylamino, $R_1$ Wasserstoff, Niederalkyl, insbesondere Methyl, oder durch Carboxy substituiertes Niederalkyl oder Cycloalkyl, insbesondere 2-Carboxy-2-propyl oder 1-Carboxy-1-cyclopropyl, $R_2$ Wasserstoff oder Methoxy, $R_3$ Niederalkanoyloxy, wie Acetoxy, Carbamoyloxy, N-Niederalkylcarbamoyloxy, oder gegebenenfalls substituiertes, mit einem Ringkohlenstoffatom an die Thiogruppe gebundenes Heterocyclylthio, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell hydriert und gegebenenfalls beispielsweise durch Niederalkyl, wie Methyl, N,N-Diniederalkylaminoniederalkyl, wie 2-N,N-Dimethylaminoäthyl, Carboxyniederalkyl, wie Carboxymethyl oder 2-Carboxyäthyl,

BAD ORIGINAL

Sulfoniederalkyl, wie Sulfomethyl oder 2-Sulfoäthyl, Amino, Carboxyniederalkanoylamino, Carbamoyl, Oxo oder Hydroxy substituiert sein können, und $R_4$ Carboxyl oder in physiologisch spaltbarer Form verestertes Carboxyl, z.B. Acyloxymethoxycarbonyl, darstellt, sowie pharmazeutisch verwendbaren Salzen von solchen Verbindungen.

6. Verfahren nach Patentanspruch 1 zur Herstellung von Verbindungen der Formel (I), worin Am Amino oder Methylamino, $R_1$ Wasserstoff, Methyl, 2-Carboxy-2-propyl, 1-Carboxy-1-cyclopropyl, Carbamoyl oder N-Methylcarbamoyl, $R_2$ Wasserstoff oder Methoxy, $R_3$ Acetoxy, Carbamoyloxy, gegebenenfalls durch Niederalkyl, Carboxyniederalkyl, Sulfoniederalkyl oder Diniederalkylaminoniederalkyl substituiertes Tetrazolylthio, z.B. 1-Methyl-1H-tetrazol-5-yl-thio, 1-Sulfomethyl-1H-tetrazol-5-ylthio, 1-(2-Sulfoäthyl)-1H-tetrazol-5-ylthio, 1-Carboxymethyl-1H-tetrazol-5-ylthio, 1-(2-Dimethylaminoäthyl)-1H-tetrazol-5-ylthio, gegebenenfalls durch Niederalkyl substituiertes Thiadiazolylthio, wie 2-Methyl-1,3,4-thiadiazol-5-yl-thio oder 3-Methyl-1,2,4-thiadiazol-5-ylthio, gegebenenfalls durch Hydroxy substituiertes Pyridazinylthio, z.B. 3-Hydroxy-pyridazin-6-ylthio, gegebenenfalls durch Hydroxy substituiertes Pyrimidinylthio, z.B. 4-Hydroxy-2-pyrimidinyl- bzw. das dazu tautomere 4-Oxo-3,4-dihydro-2-pyrimidinylthio, oder durch Niederalkyl und zwei Hydroxy substituiertes Triazinylthio, wie 2-Niederalkyl-5,6-dihydroxy-1,2,4-triazin-3-ylthio, bzw. dazu tautomeres 1,2,5,6-Tetrahydro-2-niederalkyl-5,6-dioxo-1,2,4-triazin-3-ylthio, z.B. 1,2,5,6-Tetrahydro-2-methyl-5,6-dioxo-1,2,4-triazinyl-3-ylthio, und $R_4$ Carboxyl oder in physiologisch spaltbarer Form verestertes Carboxyl, z.B. Pivaloyloxymethoxycarb onyl, oder Phthalidyloxycarbonyl, bedeutet, und pharmazeutisch verwendbaren Salzen von solchen Verbindungen.

BAD ORIGINAL

7. Verfahren nach Patentanspruch 2 zur Herstellung von Verbindungen der Formel (I), worin Am Amino oder Methylamino, $R_1$ Wasserstoff, Methyl, 2-Carboxy-2-propyl oder 1-Carboxy-1-cyclopropyl, $R_2$ Wasserstoff oder Methoxy, $R_3$ Acetoxy, Carbamoyloxy, gegebenenfalls durch Niederalkyl oder Carboxyniederalkyl substituiertes Triazolylthio, wie 4-Carboxymethyl-1,2,4-triazolylthio, gegebenenfalls durch Niederalkyl, Carboxyniederalkyl, Sulfoniederalkyl oder Diniederalkylaminoniederalkyl substituiertes Tetrazolylthio, wie 1-Methyl-1H-tetrazol-5-ylthio, 1-Sulfomethyl-1H-tetrazol-5-ylthio, 1-(2-Sulfoäthyl)-1H-tetrazol-5-ylthio, 1-Carboxymethyl-1H-tetrazol-5-ylthio, 1-(2-Dimethylaminoäthyl)-1H-tetrazol-5-ylthio, gegebenenfalls durch Niederalkyl substituiertes Thiadiazolylthio, wie 2-Methyl-1,3,4-thiadiazol-5-ylthio oder 3-Methyl-1,2,4-thiadiazol-5-ylthio, gegebenenfalls durch Hydroxy substituiertes Pyridazinylthio, wie 3-Hydroxy-pyridazin-6-ylthio, gegebenenfalls durch Hydroxy substituiertes Pyrimidinylthio, wie 4-Hydroxy-2-pyrimidinyl- bzw. das dazu tautomere 4-Oxo-3,4-dihydro-2-pyrimidinylthio, oder durch Niederalkyl und zwei Hydroxy substituiertes Triazinylthio, wie 2-Niederalkyl-5,6-dihydroxy-1,2,4-triazin-3-ylthio, und $R_4$ Carboxyl oder in physiologisch spaltbarer Form verestertes Carboxyl, z.B. Pivaloyloxymethoxycarbonyl, oder Phthalidyloxycarbonyl, bedeutet, sowie pharmazeutisch verwendbaren Salzen von solchen Verbindungen.

---

8. Verfahren zur Herstellung von 7β-[2-(5-Amino-1,2,4-thiadiazol-3-yl)-2-Z-methoxyiminoacetamido]-3-(1,2,5,6-tetrahydro-2-methyl-5,6-dioxo-1,2,4-triazin-3-yl-thiomethyl)-3-cephem-4-carbonsäure, und pharmazeutisch annehmbaren Salzen davon, nach Patentanspruch 2.

BAD ORIGINAL

9. Verfahren zur Herstellung von 7ß-[2-(5-Amino-1,2,4-thiadiazol-3-yl)-2-Z-hydroxyiminoacetamido]-3-(1-methyltetrazol-5-yl-thiomethyl)-3-cephem-4-carbonsäure, und pharmazeutisch annehmbaren Salzen davon, nach Patentanspruch 2.

10. Verfahren zur Herstellung von 7ß-[2-(5-Amino-1,2,4-thiadiazol-3-yl)-2-Z-methoxyiminoacetamido]-3-acetoxymethyl-3-cephem-4-carbonsäure-pivaloyloxymethylester, und pharmazeutisch annehmbaren Salzen davon, nach Patentanspruch 1.

11. Verfahren zur Herstellung von 7ß-[2-(5-Amino-1,2,4-thiadiazol-3-yl)-2-Z-methoxyiminoacetamido]-3-carbamoyloxymethyl-3-cephem-4-carbonsäure-pivaloyloxymethylester, und pharmazeutisch annehmbaren Salzen davon, nach Patentanspruch 2.

12. Verfahren zur Herstellung von 7ß-[2-(5-Amino-1,2,4-thiadiazol-3-yl)-2-Z-methoxyiminoacetamido]-3-(1-methyltetrazol-5-yl-thiomethyl)-3-cephem-4-carbonsäure-pivaloyloxymethylester, und pharmazeutisch annehmbaren Salzen davon, nach Patentanspruch 2.

13. Verfahren zur Herstellung von 7ß-[2-(5-Amino-1,2,4-thiadiazol-3-yl)-2-Z-methoxyiminoacetamido]-3{1-[2-(N,N-dimethylamino)äthyl]-1H-tetrazol-5-yl-thiomethyl} 3-cephem-4-carbonsäure-pivaloyloxymethylester, und pharmazeutisch annehmbaren Salzen davon, nach Patentanspruch 2.

14. Verfahren zur Herstellung von 7ß-[2-(5-Amino-1,2,4-thiadiazol-3-yl)-2-Z-methoxyiminoacetamido]-3-(1-pivaloyloxymethoxycarbonylmethyl-tetrazol-5-yl-thiomethyl-3-cephem-4-carbonsäure-pivaloyloxymethylester, und pharmazeutisch annehmbaren Salzen davon, nach Patentanspruch 2.

BAD ORIGINAL

15. Verfahren zur Herstellung von 7ß-[2-(5-Amino-1,2,4-thiadiazol-3-yl)-2-Z-methoxyiminoacetamido]-3-(4-oxo-3,4-dihydro-pyrimidin-2-yl-thiomethyl)-3-cephem-4-carbonsäure, und pharmazeutisch annehmbaren Salzen davon, nach Patentanspruch 2.

16. Verfahren zur Herstellung von 7ß-[2-(5-N-Methylamino-1,2,4-thiadiazol-3-yl)-2-Z-methoxyiminoacetamido]-3-acetoxymethyl-3-cephem-4-carbonsäure, und pharmazeutisch annehmbaren Salzen davon, nach Patentanspruch 2.

17. Verfahren zur Herstellung von 7ß-[2-(5-Amino-1,2,4-thiadiazol-3-yl)-2-Z-methylcarbamoyloxyiminoacetamido]-3-acetoxymethyl-3-cephem-4-carbonsäure und pharmazeutisch annehmbaren Salzen davon, nach Patentanspruch 1.

18. Verfahren zur Herstellung von 7ß-[2-(5-Amino-1,2,4-thiadiazol-3-yl)-2-Z-methylcarbamoyloxyiminoacetamido]-3-(1-methyltetrazol-5-yl-thiomethyl)-3-cephem-4-carbonsäure und pharmazeutisch annehmbaren Salzen davon, nach Patentanspruch 1.

19. Verfahren zur Herstellung von 7ß-[2-(5-Amino-1,2,4-thiadiazol-3-yl)-2-Z-methylcarbamoyloxyiminoacetamido]-3-(1-carboxymethyl-tetrazol-5-yl-thiomethyl)-3-cephem-4-carbonsäure und pharmazeutisch annehmbaren Salzen davon, nach Patentanspruch 1.

20. Verfahren zur Herstellung von 7ß-[2-(5-Amino-1,2,4-thiadiazol-3-yl)-2-Z-hydroxyimino-acetamido]-3-(1-carboxymethyl-tetrazol-5-yl-thiomethyl)-3-cephem-4-carbonsäure und pharmazeutisch annehmbaren Salzen davon, nach Patentanspruch 1.

BAD ORIGINAL

21. Verfahren zur Herstellung von 7ß-[2-(5-Amino-1,2,4-thiadiazol-3-yl)-2-Z-methylcarbamoyloxyiminoacetamido]-3-(1-sulfomethyl-tetrazol-5-yl-thiomethyl)-3-cephem-4-carbonsäure und pharmazeutisch annehmbaren Salzen davon, nach Patentanspruch 1.

22. Verfahren zur Herstellung von 7ß-[2-(5-Amino-1,2,4-thiadiazol-3-yl)-2-Z-hydroxyimino-acetamido[-3-(1-sulfomethyl-tetrazol-5-yl-thiomethyl)-3-cephem-4-carbonsäure und pharmazeutisch annehmbaren Salzen davon, nach Patentanspruch 1.

23. Verfahren zur Herstellung von 7ß-[2-(5-Amino-1,2,4-thiadiazol-3-yl)-2-Z-methylcarbamoyloxyiminoacetamidoy-3-(1-methyltetrazol-5-yl-thiomethyl)-3-cephem-4-carbonsäure-pivaloyloxymethylester und pharmazeutisch annehmbaren Salzen davon, nach Patentanspruch 1.

24. Verfahren zur Herstellung von 7ß-[2-(5-N-Methylamino-1,2,4-thiadiazol-3-yl)-2-Z-methoxyiminoacetamido]-3-(1-carboxymethyltetrazol-5-yl-thiomethyl)-3-cephem-4-carbonsäure und pharmazeutisch annehmbaren Salzen davon, nach Patentanspruch 1.

25. Verfahren zur Herstellung von 7ß-[2-(5-N-Methylamino-1,2,4-thiadiazol-3-yl)-2-Z-methoxyiminoacetamido]-3-(1-methyltetrazol-5-yl-thiomethyl)-3-cephem-4-carbonsäure und pharmazeutisch annehmbaren Salzen davon, nach Patentanspruch 1.

26. Verfahren zur Herstellung von 7ß-[2-(5-tert.Butoxycarbonylamino-1,2,4-thiadiazol-3-yl)-2-Z-(2-carboxyprop-2-yloxyimino)acetamido]-3-acetoxymethyl-3-cephem-4-carbonsäure und pharmazeutisch annehmbaren Salzen davon, nach Patentanspruch 1.

BAD ORIGINAL

27. Verfahren zur Herstellung von 7ß-[2-(5-Amino-1,2,4-thiadiazol-3-yl)-2-Z-(2-carboxyprop-2-yloxyimino)acetamido]-3-(1-methyltetrazol-5-ylthiomethyl)-3-cephem-4-carbonsäure und pharmazeutisch annehmbaren Salzen davon, nach Patentanspruch 1.

28. Verfahren zur Herstellung von 7ß-[2-(5-Amino-1,2,4-thiadiazol-3-yl)-2-Z-(2-carboxyprop-2-yloxyimino)acetamido]-3-[1-(2-N,N-dimethylaminoäthyl)tetrazol-5-ylthiomethyl]-3-cephem-4-carbonsäure und pharmazeutisch annehmbaren Salzen davon, nach Patentanspruch 1.

29. Verfahren zur Herstellung von Verbindungen der Formel

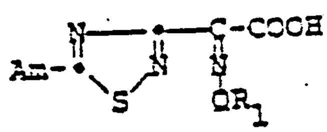

(III)

worin Am eine gegebenenfalls geschützte Aminogruppe darstellt und $R_1$ für Wasserstoff, gegebenenfalls substituiertes Niederalkyl oder Cycloalkyl, oder gegebenenfalls N-substituiertes Carbamoyl steht, Estern davon und ihrer zur Acylierung geeigneten Derivate, dadurch gekennzeichnet, dass man

a) eine Verbindung der Formel

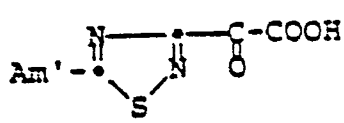

(XII),

worin Am' eine geschützte oder tertiäre Aminogruppe bedeutet, oder einen Ester davon mit einem Hydroxylamin der Formel $H_2N-O-R_1$ (V), worin $R_1$ die oben genannte Bedeutung hat, behandelt, oder

BAD ORIGINAL

b) eine Verbindung der Formel

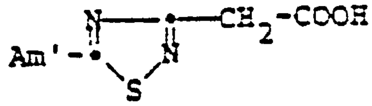

(XI),

worin Am' eine geschützte oder tertiäre Aminogruppe bedeutet, oder einen Ester davon, nitrosiert oder

c) eine Verbindung der Formel

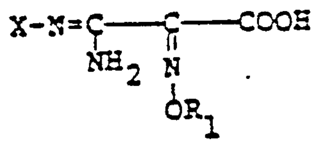

(XIII),

worin X Halogen oder Hydroxy bedeutet und $R_1$ die oben angegebene Bedeutung hat, oder einen Ester davon, mit einem Salz der Rhodanwasserstoffsäure, bzw. einem Isorhodanwasserstoffsäure-ester, oder eine Verbindung der Formel XIII, worin X Wasserstoff ist, mit Rhodan behandelt, oder

d) zur Herstellung einer Verbindung der Formel III, oder eines Esters davon, worin Am eine primäre, sekundäre oder tertiäre Aminogruppe bedeutet, eine Verbindung der Formel

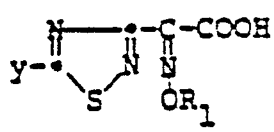

(XIX),

XIX

oder einen Ester davon, worin Y Halogen ist und $R_1$ die oben angegebene Bedeutung hat, mit Ammoniak oder einem primären oder sekundären Amin Am-H, oder einem Metallamid davon, umsetzt, oder

BAD ORIGINAL

e) Zur Herstellung einer Verbindung der Formel (III), worin $R_1$ für gegebenenfalls substituiertes Niederalkyl oder Cycloalkyl, oder für gegebenenfalls N-substituiertes Carbamoyl steht, oder eines Esters oder eines zur Acylierung geeigneten Derivates davon, dadurch gekennzeichnet, dass man eine Verbindung der Formel (III), worin $R_1$ Wasserstoff ist, oder einen Ester davon, mit einem den Rest $R_1$ einführenden Mittel behandelt, und gewünschtenfalls eine geschützte Aminogruppe in eine freie Aminogruppe oder in ein andersartig geschützte Aminogruppe überführt, und/oder eine Carbonsäure der Formel III in ein reaktionsfähiges Derivat davon überführt.

30. Verfahren zur Herstellung von Verbindungen der Formel

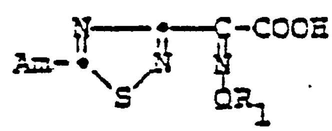

(III)

worin Am eine gegebenenfalls geschützte Aminogruppe darstellt und $R_1$ für Wasserstoff, gegebenenfalls substituiertes Niederalkyl oder Cycloalkyl, oder gegebenenfalls N-substituiertes Carbamoyl steht, Estern davon und ihrer zur Acylierung geeigneten Derivate, dadurch gekennzeichnet, dass man

a) eine Verbindung der Formel

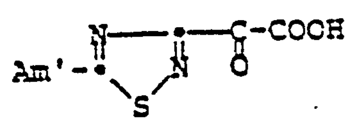

(XII),

worin Am' eine geschützte oder tertiäre Aminogruppe bedeutet, oder einen Ester davon mit einem Hydroxylamin der Formel $H_2N-O-R_1$ (V), worin $R_1$ die oben genannte Bedeutung hat, behandelt, oder

BAD ORIGINAL

b) eine Verbindung der Formel

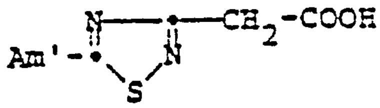

(XI),

worin Am' eine geschützte oder tertiäre Aminogruppe bedeutet, oder einen Ester davon, nitrosiert oder

c) eine Verbindung der Formel

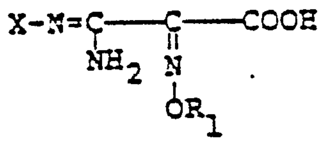

(XIII),

worin X Halogen oder Hydroxy bedeutet und $R_1$ die oben angegebene Bedeutung hat, oder einen Ester davon, mit einem Salz der Rhodanwasserstoffsäure, bzw. einem Isorhodanwasserstoffsäure-ester, oder eine Verbindung der Formel XIII, worin X Wasserstoff ist, mit Rhodan behandelt, oder

d) zur Herstellung einer Verbindung der Formel III, oder eines Esters davon, worin Am eine primäre, sekundäre oder tertiäre Aminogruppe bedeutet, eine Verbindung der Formel

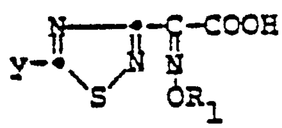

(XIX),

XIX

oder einen Ester davon, worin Y Halogen ist und $R_1$ die oben angegebene Bedeutung hat, mit Ammoniak oder einem primären oder sekundären Amin Am-H, oder einem Metallamid davon, umsetzt und gewünschtenfalls eine erhalten Verbindung der Formel III, worin $R_1$ Wasserstoff bedeutet, in eine Verbindung der Formel III überführt, worin $R_1$ gegebenenfalls substituiertes Niederalkyl bedeutet, und/oder eine geschützte Aminogruppe in eine freie Aminogruppe oder in eine andersartig geschützte Aminogruppe überführt, und/oder eine Carbonsäure der Formel III in ein reaktionsfähiges Derivat davon überführt.

BAD ORIGINAL